(19) **Europäisches Patentamt / European Patent Office / Office européen des brevets**

(11) **EP 3 882 268 A1**

(12) **EUROPEAN PATENT APPLICATION**
published in accordance with Art. 153(4) EPC

(43) Date of publication:
**22.09.2021 Bulletin 2021/38**

(21) Application number: **19885765.8**

(22) Date of filing: **11.11.2019**

(51) Int Cl.:
**C07K 16/28** (2006.01)          **C07K 16/46** (2006.01)
**A61K 39/395** (2006.01)        **C12N 15/13** (2006.01)
**C07K 19/00** (2006.01)          **A61P 35/00** (2006.01)

(86) International application number:
**PCT/CN2019/117114**

(87) International publication number:
**WO 2020/098599 (22.05.2020 Gazette 2020/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.11.2018 CN 201811337068**

(71) Applicants:
• **Jiangsu Hengrui Medicine Co., Ltd.**
  **Lianyungang, Jiangsu 222047 (CN)**
• **Shanghai Hengrui Pharmaceutical Co., Ltd.**
  **Shanghai 200245 (CN)**

(72) Inventors:
• **CAO, Zhuoxiao**
  **Shanghai 200245 (CN)**
• **FU, Yayuan**
  **Shanghai 200245 (CN)**
• **XU, Zhibin**
  **Shanghai 200245 (CN)**
• **ZHANG, Limin**
  **Shanghai 200245 (CN)**
• **HU, Qiyue**
  **Shanghai 200245 (CN)**
• **TAO, Weikang**
  **Shanghai 200245 (CN)**

(74) Representative: **Potter Clarkson**
  **The Belgrave Centre**
  **Talbot Street**
  **Nottingham NG1 5GG (GB)**

(54) **ANTI-CD73 ANTIBODY, ANTIGEN-BINDING FRAGMENT THEREOF AND APPLICATION THEREOF**

(57)    Provided are an anti-CD73 antibody, an antigen-binding fragment thereof, and an application thereof. Specifically, provided are a murine, chimeric or humanized anti-CD73 antibody comprising a specific CDR region and an antigen-binding fragment thereof. Also provided are a pharmaceutical composition comprising the anti-CD73 antibody or the antigen-binding fragment thereof and the use thereof as a diagnostic agent and a therapeutic agent for CD73-related diseases.

EP 3 882 268 A1

**Description**

**FIELD OF THE INVENTION**

The present disclosure belongs to the field of biotechnology. More specifically, the present disclosure relates to anti-CD73 antibodies and applications thereof.

**BACKGROUND OF THE INVENTION**

The descriptions herein only provide background information about the present disclosure, and do not necessarily constitute prior art.

CD73, also known as extracellular-5'-nucleotidase (Ecto-5'-Nucleotidase, EC3.1.3.5, ecto-5'-NT), is a glycoprotein anchored to the membrane through glycosyl phosphatidyl inositol (GPI). CD73 is widely expressed on the surface of a variety of tissues and cells in the human body, such as endothelial cells, lymphocytes, Treg and others. Recently, studies have found that CD73 is highly expressed in a variety of tumors. A large number of studies have confirmed that CD73 is closely related to the occurrence and development of tumors.

The gene encoding human CD73 is located on chromosome 6q14-q21. The evolution of CD73 is relatively conservative, and the DNA sequence encoding human CD73 has 86% homology with the murine DNA sequence. The CD73 gene consists of 57 kbp and the coding region is 1,725 bp in length. The G+C ratio in the promoter region sequences of CD73 genome is relatively higher, and CD73 genome comprises many binding sites for transcription factors, including one cAMP response element (eRE), five Sp-1 binding sites, two Ap-2 binding sites, one hypoxia response element (HRE), and one TCF/LEF binding site. There are three GATA boxes, two MyoD sites, IgH and NF-$\kappa$b binding sites, and 3 C-EBP sites in the upstream region of the promoter. The human CD73 precursor protein consists of 574 amino acid residues. After the amino acid residues 1-26 at the N-terminus and the amino acid residues 550-574 at the C-terminus of the CD73 molecule are excised, the amino acid residues 27-549 constitute a mature CD73, forming a subunit with the molecular weight of about 70 KD. Two subunits constitute a homodimer with biological activity. The mature CD73 includes five N-linked glycosylation sites; the C-terminal amino acid sequence is GPI anchor sequence, in which serine on position 523 serves as an anchor site, which is anchored to the outer membrane of the cell through GPI. CD73 can be shed from the surface of the plasma membrane due to the hydrolysis of GPI by endogenous phospholipase C, thereby reducing the production of adenosine.

CD73 is widely expressed on the surface of tissue cells such as human endothelial cells and lymphocytes. CD73 has extracellular nucleotidase activity and hydrolyzes AMP to produce adenosine, which has immunosuppressive effect. The binding of adenosine to the receptor can promote angiogenesis, prevent tissue ischemia-reperfusion injury, and inhibit inflammation and immune response. Recently, it has been discovered that adenosine can inhibit the pro-inflammatory response of human vascular endothelial cells. CD73 also has non-hydrolase activity and is involved in cell adhesion and signal transduction.

CD73 plays an important role in the occurrence and development of tumors. CD73 is involved in tumor angiogenesis. Related studies have shown that CD73 can promote the neovascularization *in vivo* in C57BL/6 mice bearing B16F10 melanoma cells. Pharmacological inhibition of CD73 can reduce the number of new blood vessels and hinder the maturation of new blood vessels, thereby reducing the angiogenesis of melanoma. Another study has found that treating tumor-bearing mice with anti-CD73 monoclonal antibody can reduce angiogenesis of tumor. CD73 can also promote the migration of microvascular endothelial cells to form a tubular structure, based on its enzymatic and non-enzymatic functions. CD73 induces the growth of epithelial cells by up-regulating cell proliferation protein D1. In addition, the adenosine produced by CD73 can promote the production and release of VEGF, and support the formation of new blood vessels, through tissue hypoxia caused by A2A receptors.

Next, studies *in vivo* and *in vitro* have shown that CD73 can promote the growth of tumor cells. The research results of ZHI et al. (Cancer Sci, 2010, 101(12): 2561-2569) showed that inhibition of the CD73 expression by siRNA can block the cell cycle and apoptosis, thereby inhibiting the proliferation and differentiation of breast cancer cells. Use of 5'-$\alpha$, $\beta$-methylene-adenosine diphosphate (APCP), a specific inhibitor of CD73 enzyme activity, can also inhibit the proliferation of tumor cells in a dose-dependent manner. Over-expression of CD73 in breast cancer cells transfected with CD73 increased cell viability and promoted the cell cycle. Some researchers have found that APCP can reduce the proliferation of glioma cells by 30%, while adenosine can increase the cell proliferation by 35%. Stagg J et al. (PNAS, 2010, 107(4): 1547-1552) has found that the activity of CD73 is significantly increased in bladder cancer cells with a higher degree of malignancy, confirming that the purine signaling pathway promotes the formation of bladder tumors. In addition, CD73 can also promote the growth of colon cancer cells, melanoma cells and lymphoma cells. Recent studies have found that leukemic T lymphocytes with up-regulated CD73 expression can specifically inhibit TRAIL-induced apoptosis and induce resistance to multiple drugs in cells, thereby promoting the survival of tumor cells.

In addition, tumor-derived CD73 expressed on the surface of tumor cells is an important reason for tumor immune escape. RYZHOV et al. (J Immunol, 2011, 187(11):6120-6129) also found that CD73 in tumors is related to the penetration and aggregation of MDSCs, whereas MDSCs are immature bone marrow cells that inhibit T cell activation. The adenosine in tumor microenvironment can also help Treg to suppress immune activity. Therefore, CD73 can indirectly promote the progression of tumor by suppressing the immune response.

In addition to the above-mentioned role in tumors, CD73 has also been found to be closely related to tumor metastasis. The primary condition required by tumor metastasis refers to the acquisition of aggressive phenotype so as to increase the migration ability of tumor cells. The association between CD73 and metastasis-related markers has been discovered. CD73 level is significantly higher in patients with lymphatic metastatic prostate cancer compared to non-metastatic prostate cancer, indicating that CD73 may play an important role in the process of tumor cell metastasis. XIONG et al. (Cell Tissue Res, 2014, 355(2):365-374) has demonstrated that CD73 can promote the metastasis and invasion by inducing the mesenchymal transformation of tumor cells. STAGG and Wang et al. (Cancer Res, 2011, 71(8): 2892-2900; J Cancer Res Clin Oncol, 2008, 134(3): 365-372) found that *in vivo* CD73-deficient mice showed resistance to the occurrence of lung metastatic tumor after intravenous injection of B16F10 melanoma, and the CD73-deficient host can enhance the homing of tumor T cell-specific antigens into the tumor. Clinical studies in tumor patients have also revealed that the overexpression of CD73 in tumors is associated with the metastasis of gastric cancer, prostate cancer and malignant melanoma. The above results prove that both host CD73 and tumor CD73 can significantly promote tumor metastasis, whereas the resistance to tumor metastasis in CD73-deficient host is related to the enhanced endogenous anti-tumor immunity.

Currently, CD73-related antibodies have been reported in patent applications such as US9938356, US9605080, WO2016075099, WO2017152085, WO2017064043 and WO2018013611.

## SUMMARY OF THE INVENTION

The inventors of the present application have developed new anti-CD73 antibodies or antigen-binding fragment thereof after a large number of experiments, in which:
In some embodiments, the present disclosure discloses an anti-CD73 antibody or an antigen-binding fragment thereof specifically binding to human CD73, wherein the antibody comprises a light chain variable region and a heavy chain variable region, wherein:

the light chain variable region comprises light chain LCDR1, LCDR2 and LCDR3 as shown in SEQ ID NOs: 27, 28 and 29 respectively, or comprises LCDR variant(s) having no more than 3 amino acid mutation(s) relative to the sequence as shown in SEQ ID NOs: 27, 28 and 29 respectively; and
the heavy chain variable region comprises:

i) HCDR1, HCDR2 and HCDR3 as shown in SEQ ID NOs: 10, 11 and 12 respectively or HCDR variant(s) having no more than 3 amino acid mutation(s) relative to the sequence as shown in SEQ ID NOs: 10, 11 and 12 respectively; or
ii) HCDR1, HCDR2 and HCDR3 as shown in SEQ ID NOs: 16, 17 and 18 respectively or HCDR variant(s) having no more than 3 amino acid mutation(s) relative to the sequence as shown in SEQ ID NOs: 16, 17 and 18 respectively; or
iii) HCDR1, HCDR2 and HCDR3 as shown in SEQ ID NOs: 22, 67 and 24 respectively or HCDR variant(s) having no more than 3 amino acid mutation(s) relative to the sequence as shown in SEQ ID NOs: 22, 67 and 24 respectively.

In some embodiments, "no more than 3 amino acid mutation(s)" described above means 3, 2, 1, or 0 amino acid mutation(s).

In some embodiments, the anti-CD73 antibody or the antigen-binding fragment thereof of the present disclosure binds to human CD73 with a dissociation equilibrium constant equal to or less than $10^{-7}$ M; and in some embodiments, binds to human CD73 with a dissociation equilibrium constant equal to or less than $10^{-8}$M, $10^{-9}$M, $10^{-10}$M or $10^{-11}$M.

In some embodiments, the anti-CD73 antibody or the antigen-binding fragment thereof of the present disclosure comprises:

iv) HCDR1, HCDR2, HCDR3, LCDR1, LCDR2 and LCDR3 as shown in SEQ ID NOs: 10, 11, 12, 13, 14 and 15, respectively; or
v) HCDR1, HCDR2, HCDR3, LCDR1, LCDR2 and LCDR3 as shown in SEQ ID NOs: 16, 17, 18, 19, 20 and 21, respectively; or
vi) HCDR2 as shown in SEQ ID NOs: 23, 68, 69, 70, 71, 72, 73, 74, 75 or 76 and HCDR1, HCDR3, LCDR1, LCDR2 and LCDR3 as shown in SEQ ID NOs: 22, 24, 25, 14 and 26, respectively.

In some embodiments, the anti-CD73 antibody or the antigen-binding fragment thereof of the present disclosure comprises:

> vii) HCDR1, HCDR2, HCDR3, LCDR1, LCDR2 and LCDR3 as shown in SEQ ID NOs: 22, 23, 24, 25, 14 and 26, respectively; or
> viii) HCDR1, HCDR2, HCDR3, LCDR1, LCDR2 and LCDR3 as shown in SEQ ID NOs: 22, 71, 24, 25, 14 and 26, respectively; or
> ix) HCDR1, HCDR2, HCDR3, LCDR1, LCDR2 and LCDR3 as shown in SEQ ID NOs: 22, 76, 24, 25, 14 and 26, respectively.

In some embodiments, the anti-CD73 antibody or the antigen-binding fragment thereof of the present disclosure is a recombinant antibody, preferably a murine antibody, a chimeric antibody, or a humanized antibody.

In some embodiments, the anti-CD73 antibody or the antigen-binding fragment thereof of the present disclosure is a murine or chimeric antibody selected from any anti-CD73 antibody or antigen-binding fragment thereof as defined in item A-C:

> (A) an anti-CD73 antibody or an antigen-binding fragment thereof comprising a heavy chain variable region as shown in SEQ ID NO: 4 and a light chain variable region as shown in SEQ ID NO: 5;
> (B) an anti-CD73 antibody or an antigen-binding fragment thereof comprising a heavy chain variable region as shown in SEQ ID NO: 6 and a light chain variable region as shown in SEQ ID NO: 7; and
> (C) an anti-CD73 antibody or an antigen-binding fragment thereof comprising a heavy chain variable region as shown in SEQ ID NO: 8 and a light chain variable region as shown in SEQ ID NO: 9.

In some embodiments of the anti-CD73 antibody or the antigen-binding fragment thereof of the present disclosure, the antibody is a humanized antibody, and the light chain framework regions (FRs) and the heavy chain framework regions (FRs) of the antibody are respectively derived from human germline light chain and heavy chain or mutant sequence(s) thereof; preferably, the humanized antibody comprises any one selected from the group consisting of D-F:

> (D) HCDR1, HCDR2, HCDR3, LCDR1, LCDR2 and LCDR3 as shown in SEQ ID NOs: 10, 11, 12, 13, 14 and 15, respectively, and heavy chain framework region(s) and light chain framework region(s); and
> wherein the heavy chain framework region(s) comprise(s) one or more back mutation(s) selected from the group consisting of 6Q, 10G, 30K, 37V, 44G, 49G and 94G; and wherein the light chain framework region(s) comprise(s) one or more back mutation(s) selected from the group consisting of 36L, 42G, 44I, 60K, 69S, 71Y and 85D;
> (E) HCDR1, HCDR2, HCDR3, LCDR1, LCDR2 and LCDR3 as shown in SEQ ID NOs: 16, 17, 18, 19, 20 and 21, respectively, and heavy chain framework region(s) and light chain framework region(s); and
> wherein the heavy chain framework region(s) comprise(s) one or more back mutation(s) selected from the group consisting of 38K, 44G, 48I, 66K, 67A, 69L and 71A; and wherein the light chain framework region(s) comprise(s) one or more back mutation(s) selected from the group consisting of 36L, 42G, 44I, 66R, 71Y and 85D; and
> (F) HCDR2 as shown in SEQ ID NO: 23, 68, 69, 70, 71, 72, 73, 74, 75 or 76, and HCDR1, HCDR3, LCDR1, LCDR2 and LCDR3 as shown in SEQ ID NOs: 22, 24, 25, 14 and 26 respectively, and heavy chain framework region(s) and light chain framework region(s); and

wherein the heavy chain framework region(s) comprise(s) one or more back mutation(s) selected from the group consisting of 38M, 44S, 48I, 67A, 69L, 71V, 73K, 82A R and 94T; and wherein the light chain framework region(s) comprise(s) one or more back mutation(s) selected from the group consisting of 36L, 42G, 44L, 69S, 70D and 71Y; wherein, the positions of back mutation are numbered according to the Kabat numbering criteria. In some embodiments of the anti-CD73 antibody or the antigen-binding fragment thereof of the present disclosure, the antibody comprises a heavy chain variable region as shown in SEQ ID NO: 30, 41 or 49 or variant(s) thereof.

In some embodiments of the anti-CD73 antibody or the antigen-binding fragment thereof of the present disclosure, the heavy chain variable region variant has 1-10 amino acid back mutation(s) on the FR region(s) of the heavy chain variable region as shown in SEQ ID NO: 30, 41 or 49.

In some embodiments, the heavy chain variable region variant of the present disclosure is any one selected from the group consisting of G-I:

> (G) a heavy chain variable region variant, having one or more back mutation(s) selected from the group consisting of E6Q, V10G, S30K, I37V, L44G, A49G and R94G on the FR region of the heavy chain variable region as shown in SEQ ID NO: 30;
> (H) a heavy chain variable region variant, having one or more back mutation(s) selected from the group consisting of R38K, R44G, M48I, R66K, V67A, I69L and R71A on the FR region of the heavy chain variable region as shown in SEQ ID NO: 41; and

(I) a heavy chain variable region variant, having one or more back mutation(s) selected from the group consisting of R38M, G44S, M48I, V67A, M69L, R71V, T73K, S82AR and R94T on the FR region(s) of the heavy chain variable region as shown in SEQ ID NO: 49.

In some embodiments, the anti-CD73 antibody or the antigen-binding fragment thereof of the present disclosure comprises a heavy chain variable region of any one selected from the group consisting of J-L:

(J) a heavy chain variable region as shown in SEQ ID NO: 30, 37, 38, 39 or 40;
(K) a heavy chain variable region as shown in SEQ ID NO: 41, 45, 46, 47 or 48; and
(L) a heavy chain variable region as shown in SEQ ID NO: 49, 54, 55, 56, 57, 58, 59, 60, 61, 62, 63, 64, 65 or 66.

In some embodiments of the anti-CD73 antibody or the antigen-binding fragment thereof of the present disclosure, the antibody comprises a light chain variable region as shown in SEQ ID NO: 31, 41 or 50 or variant(s) thereof.
In some embodiments of the anti-CD73 antibody or the antigen-binding fragment thereof of the present disclosure, the light chain variable region variant has 1-10 amino acid back mutation(s) on the FR region(s) of the light chain variable region as shown in SEQ ID NO: 31, 41 or 50.
In some embodiments, the light chain variable region variant described in the present disclosure is any one selected from the group consisting of M-O:

(M) a light chain variable region variant, having one or more back mutation(s) selected from the group consisting of 36L, 42G, 441, 60K, 69S, F71Y and T85D on the FR region(s) of the light chain variable region as shown in SEQ ID NO: 31;
(N) a light chain variable region variant, having one or more back mutation(s) selected from the group consisting of Y36L, K42G, P44I, G66R, F71Y and T85D on the FR region(s) of the light chain variable region as shown in SEQ ID NO: 42; and
(O) a light chain variable region variant, having one or more back mutation(s) selected from the group consisting of Y36L, K42G, P44L, T69S, E70D and F71Y on the FR region(s) of the light chain variable region as shown in SEQ ID NO: 50.

In some embodiments, the anti-CD73 antibody or the antigen-binding fragment thereof of the present disclosure comprises a light chain variable region of any one selected from the group consisting of P-R:

(P) a light chain variable region as shown in SEQ ID NO: 31, 32, 33, 34, 35 or 36;
(Q) a light chain variable region as shown in SEQ ID NO: 42, 43 or 44; and
(R) a light chain variable region as shown in SEQ ID NO: 50, 51, 52 or 53.

In some embodiments, the anti-CD73 antibody or the antigen-binding fragment thereof of the present disclosure comprises a heavy chain and a light chain variable region selected from the group consisting of S-U:

(S) a heavy chain variable region as shown in amino acid sequence SEQ ID NO: 30, 37, 38, 39 or 40 or having at least 95% sequence identity to SEQ ID NO: 41, 45, 46, 47 or 48, and a light chain variable region as shown in amino acid sequence SEQ ID NO: 31, 32, 33, 34, 35 or 36 or having at least 95% sequence identity to SEQ ID NO: 42, 43 or 44;
(T) a heavy chain variable region as shown in amino acid sequence SEQ ID NO: 41, 45, 46, 47 or 48 or having at least 95% sequence identity to SEQ ID NO: 41, 45, 46, 47 or 48, and a light chain variable region as shown in amino acid sequence SEQ ID NO: 42, 43 or 44 or having at least 95% sequence identity to SEQ ID NO: 42, 43 or 44; and
(U) a heavy chain variable region as shown in amino acid sequence SEQ ID NO: 49, 54, 55, 56, 57, 58, 59, 60, 61, 62, 63, 64, 65 or 66 or having at least 95% sequence identity to SEQ ID NO: 49, 54, 55, 56, 57, 58, 59, 60, 61, 62, 63, 64, 65 or 66, and a light chain variable region as shown in amino acid sequence SEQ ID NO: 50, 51, 52, or 53 or having at least 95% sequence identity to SEQ ID NO: 50, 51, 52, or 53.

In some embodiments, the anti-CD73 antibody or the antigen-binding fragment thereof of the present disclosure comprises any one selected from the group consisting of V-X:

(V) a heavy chain variable region as shown in SEQ ID NO: 39 and a light chain variable region as shown in SEQ ID NO: 36;
(W) a heavy chain variable region as shown in SEQ ID NO: 41 and a light chain variable region as shown in SEQ ID NO: 43; and

(X) a heavy chain variable region as shown in SEQ ID NO: 61 and a light chain variable region as shown in SEQ ID NO: 52.

In some embodiments, the anti-CD73 antibody or the antigen-binding fragment thereof of the present disclosure comprises constant region(s).

In some embodiments, the antibody of the present disclosure is a chimeric antibody or a humanized antibody, the heavy chain constant region of which is derived from a human IgG1, IgG2, IgG3 or IgG4 or mutant sequence(s) of IgG1, IgG2, IgG3 or IgG4, and the light chain constant region is derived from human kappa, lambda chain or mutant sequence(s) thereof.

In some embodiments, the amino acid sequence of the heavy chain constant region of the antibody in the present disclosure is as shown in SEQ ID NO: 77 or has at least 85% sequence identity to SEQ ID NO: 77, and the amino acid sequence of the light chain constant region is as shown in SEQ ID No. 78 or has at least 85% sequence identity to SEQ ID NO: 78.

In some embodiments, the above-mentioned amino acid sequence having at least 85% sequence identity means an amino acid sequence having at least 86%, 87%, 88%, 89%, 90%, 91%, 92%, 93%, 94%, 95%, 96%, 97%, 98%, or 99% sequence identity.

In some embodiments, the above-mentioned amino acid sequence having at least 85% sequence identity includes those obtained by deletion, insertion or substitution mutation of one or more amino acid(s).

In some embodiments of the anti-CD73 antibody or the antigen-binding fragment thereof of the present disclosure, the antibody is any one selected from the group consisting of iv) to vi):

iv) an anti-CD73 antibody comprising a heavy chain as shown in SEQ ID NO: 79 or having at least 85% sequence identity to SEQ ID NO: 79 and a light chain as shown in SEQ ID NO: 80 or having at least 85% sequence identity to SEQ ID NO: 80;

v) an anti-CD73 antibody comprising a heavy chain as shown in SEQ ID NO: 81 or having at least 85% sequence identity to SEQ ID NO: 81 and a light chain as shown in SEQ ID NO: 82 or having at least 85% sequence identity to SEQ ID NO: 82; and

vi) an anti-CD73 antibody comprising a heavy chain as shown in SEQ ID NO: 83 or having at least 85% sequence identity to SEQ ID NO: 83 and a light chain as shown in SEQ ID NO: 84 or having at least 85% sequence identity to SEQ ID NO: 84.

In some embodiments of the present disclosure, the above-mentioned amino acid sequence having at least 85% sequence identity means an amino acid sequence having at least 86%, 87%, 88%, 89%, 90%, 91%, 92%, 93%, 94%, 95%, 96%, 97%, 98%, or 99% sequence identity.

In some specific embodiments, the above-mentioned amino acid sequence having at least 85% sequence identity includes those obtained by deletion, insertion or substitution mutation of one or more amino acid(s); in some specific embodiments, the above-mentioned amino acid sequence having at least 85% sequence identity is obtained by deletion, insertion or substitution mutation of one or more amino acid(s) in the framework(s) or constant region(s) of the antibody light or heavy chain amino acid sequence(s).

In some embodiments of the anti-CD73 antibody or the antigen-binding fragment thereof of the present disclosure, wherein the antigen-binding fragment is selected from the group consisting of Fab, Fab', F(ab')2, scFv, diabody, and dsFv.

In some embodiments, the present disclosure discloses an isolated monoclonal antibody or antigen-binding fragment thereof, which competitively with any of the antibodies or antigen-binding fragments thereof described above for the binding to human CD73, or binds to the same human CD73 epitope and/or monkey CD73 epitope as any of the antibodies or antigen-binding fragments thereof described above.

In some embodiments, the present disclosure discloses a nucleic acid molecule that encodes any one of the anti-CD73 antibodies or the antigen-binding fragments thereof described above.

In some embodiments, the present disclosure discloses a vector comprising the above-mentioned nucleic acid molecule.

In some embodiments, the present disclosure discloses a host cell, which is obtained by transforming the above-mentioned recombinant vector; the host cell is selected from the group consisting of prokaryotic cells and eukaryotic cells; in some embodiments, eukaryotic cells; in some embodiments, mammalian cells. The host cell does not include any human cell capable of developing into a complete individual, such as human embryonic stem cells, fertilized eggs, and germ cells.

In some embodiments, the present disclosure discloses a method for preparing any anti-CD73 antibody or the antigen-binding fragment thereof as described above, the method comprising:

a step of culturing the above-mentioned host cell in a suitable medium under conditions suitable for growth, recovering and purifying the anti-CD73 antibody or the antigen-binding fragment thereof from the medium.

In some embodiments, the present disclosure discloses a pharmaceutical composition, which comprises the anti-CD73 antibody or the antigen-binding fragment thereof or the nucleic acid molecule expressed by any one of the host cells described above, and one or more pharmaceutically acceptable carrier(s), excipient(s) or diluent(s).

In some embodiments, the pharmaceutical composition can contain 0.01 to 99% by weight of the anti-CD73 antibody or the antigen-binding fragment thereof in unit dose; in some embodiments, the pharmaceutical composition contains an amount of 0.1-2000 mg of the monoclonal antibody or the antigen binding fragment thereof in unit dose; in other embodiments, the pharmaceutical composition contains an amount of 1-1000 mg of the monoclonal antibody or the antigen binding fragment thereof in unit dose.

In some embodiments, the present disclosure discloses an *in vitro* method for detection of human CD73 in a sample to be tested, the method comprising: contacting the sample to be tested with any of the anti-CD73 antibodies or the antigen-binding fragments thereof, or the pharmaceutical composition described above; and determining the presence or level of human CD73 in the sample to be tested.

In some embodiments, the present disclosure discloses use of any of the anti-CD73 antibodies or the antigen-binding fragments, nucleic acid molecules, or pharmaceutical composition described above in the preparation of a medicament for the treatment or prevention of a disease or disorder. In some embodiments, the disease or disorder described is a CD73-related disease or disorder.

In some embodiments, the CD73-related disease or disorder is a cell proliferative disease, and in some embodiments, the cell proliferative disease is a tumor.

In some embodiments, the tumor is selected from the group consisting of breast cancer, glioma, bladder cancer, colon cancer, melanoma, ovarian cancer, thyroid cancer, esophageal cancer, lung cancer, kidney cancer, pancreatic cancer, lymphoma, prostate cancer and gastric cancer. In some embodiments, the tumor is a metastatic tumor.

In some embodiments, the present disclosure discloses a method for the treatment or prevention of a CD73-related disease or disorder, the method comprising administering to a subject a therapeutically effective amount of any of the anti-CD73 antibodies or the antigen binding fragments thereof described above, a nucleic acid molecule encoding the same, or a pharmaceutical composition containing the same; in some embodiments, the disease is a cell proliferative disease; in some embodiments, the cell proliferative disease is a tumor; in some embodiments, the tumor is selected from the group consisting of breast cancer, glioma, bladder cancer, colon cancer, melanoma, ovarian cancer, thyroid cancer, esophageal cancer, lung cancer, kidney cancer, pancreatic cancer, lymphoma, prostate cancer and gastric cancer. In some embodiments, the tumor is a metastatic tumor.

In some embodiments, the present disclosure discloses an anti-CD73 antibody or antigen-binding fragment thereof, or a pharmaceutical composition comprising the same, or a nucleic acid molecule encoding the same for the treatment or prevention of a disease (such as a cell proliferative disease). The treatment or prevention includes administering to a subject a pharmaceutically effective amount of any of the anti-CD73 antibodies or the antigen-binding fragments thereof, or pharmaceutical compositions, or nucleic acid molecules as described above. In some embodiments, the cell proliferative disease is a tumor. In some embodiments, the tumor is selected from the group consisting of breast cancer, glioma, bladder cancer, colon cancer, melanoma, ovarian cancer, thyroid cancer, esophageal cancer, lung cancer, kidney cancer, pancreatic cancer, lymphoma, prostate cancer and gastric cancer. In some embodiments, the tumor is a metastatic tumor.

In some embodiments, the present disclosure discloses the above-mentioned anti-CD73 antibody or antigen-binding fragment thereof, the nucleic acid molecule encoding the same, or the pharmaceutical composition comprising the same for use as a medicament; preferably, the medicament can be used for treatment or prevention of a human CD73-related disease or disorder; more preferably, the medicament can be used for the treatment of a cell proliferative disease, preferably a tumor, more preferably breast cancer, glioma, bladder cancer, colon cancer, melanoma, ovarian cancer, thyroid cancer, esophageal cancer, lung cancer, kidney cancer, pancreatic cancer, lymphoma, prostate cancer or gastric cancer.

**DESCRIPTION OF THE DRAWINGS**

Figure 1: ELISA results of the binding of anti-CD73 antibodies to human CD73 protein;
Figure 2: ELISA results of the binding of anti-CD73 antibodies to cynomolgus monkey CD73 protein;
Figure 3: Results of binding assay of anti-CD73 antibodies to MDA-MB-231 cells;
Figure 4: Experimental results of anti-CD73 antibodies in inhibiting the enzyme activity in MDA-MB-231 cell;
Figure 5: Experimental results of anti-CD73 antibodies in inducing the *in vitro* proliferation of CD4+ T cells;
Figure 6: The effect of anti-CD73 antibodies on the tumor volume of xenograft tumors in MDA-MB-231 tumor-bearing mice;

Figure 7: Inhibition experiment of anti-CD73 antibodies on lung metastatic MDA-MB-231-Luc, the vertical axis shows the bioluminescence value of tumor cells in lung;

Figure 8: Inhibition experiment of anti-CD73 antibodies on lung metastatic MDA-MB-231-Luc, the vertical axis shows the relative bioluminescence value of lung tumor.

## Detailed description

Although there have been reports about anti-CD73 antibodies in the relevant literatures, there is no commercially available CD73 antibody product for clinical use. The inventors of the present application have developed new anti-CD73 antibodies or antigen-binding fragment thereof with favorable biological activities after a large number of experiments.

In order to make the present disclosure be more easily understood, certain technical and scientific terms are specifically defined below. Unless otherwise defined explicitly herein, all other technical and scientific terms used herein have the meaning commonly understood by those skilled in the art to which this disclosure belongs.

Three-letter codes and one-letter codes for amino acids used in the present disclosure are as described in J. biol. chem, 243, p3558(1968).

As used herein, "antibody" refers to immunoglobulin, a four-peptide chain structure formed by two identical heavy chains and two identical light chains connected together by interchain disulfide bond(s). Immunoglobulin heavy chain constant regions exhibit different amino acid compositions and sequences, hence present different antigenicity. Accordingly, immunoglobulins can be divided into five types, or named as immunoglobulin isotypes, namely IgM, IgD, IgG, IgA and IgE, corresponding to heavy chain $\mu$, $\delta$, $\gamma$, $\alpha$ and $\varepsilon$, respectively According to amino acid composition of hinge region and also the number and location of heavy chain disulfide bonds, the same type of Ig can further be divided into different sub-types, for example, IgG can be divided into IgG1, IgG2, IgG3 and IgG4. Light chain can be divided into $\kappa$ or $\lambda$ chain based on different constant regions. Each of the five types of Ig can have a kappa chain or a lambda chain.

In the present disclosure, the antibody light chain described in the present disclosure can further include a light chain constant region, and the light chain constant region includes human or murine kappa, lambda chain or variant(s) thereof.

In the present disclosure, the antibody heavy chain described in the present disclosure can further include a heavy chain constant region, and the heavy chain constant region includes human or murine IgG1, IgG2, IgG3, IgG4 or variant(s) thereof.

About 110 amino acid sequences adjacent to the N-terminus of the antibody heavy and light chains are highly variable, known as variable region (Fv region); the rest of amino acid sequences close to the C-terminus are relatively stable, known as constant region. The variable region includes 3 hypervariable regions (HVRs) and 4 framework regions (FRs) with relatively conservative sequences. The three hypervariable regions which determine the specificity of the antibody are also known as complementarity determining regions (CDRs). Each of the light chain variable region (LCVR) and heavy chain variable region (HCVR) consists of 3 CDR regions and 4 FR regions, with the sequential order from the amino terminus to carboxyl terminus of: FR1, CDR1, FR2, CDR2, FR3, CDR3, and FR4. The three CDR regions of the light chain refer to LCDR1, LCDR2, and LCDR3, and the three CDR regions of the heavy chain refer to HCDR1, HCDR2, and HCDR3. The number and position of the CDR amino acid residues of the LCVR region and the HCVR region of the antibody or antigen-binding fragment described in the present disclosure comply with the known Kabat numbering criteria (LCDR1-3, HCDR1-3).

The term "complementarity determining region", "CDR" or "hypervariable region" refers to one of the six hypervariable regions present in the antibody variable domain that mainly contribute to antigen binding. Generally, there are three CDRs (HCDR1, HCDR2, HCDR3) in each heavy chain variable region, and three CDRs (LCDR1, LCDR2, LCDR3) in each light chain variable region. The amino acid sequence boundaries of CDRs can be determined by any of a variety of well-known schemes, including the "Kabat" numbering criteria (see Kabat et al. (1991), "Sequences of Proteins of Immunological Interest", 5th edition, Public Health Service, National Institutes of Health, Bethesda, MD), "Chothia" numbering criteria (see Al-Lazikani et al., (1997) JMB 273:927-948) and ImmunoGenTics (IMGT) numbering criteria (Lefranc MP, Immunologist, 7, 132- 136 (1999); Lefranc, MP, etc., Dev. Comp. Immunol., 27, 55-77 (2003), and the like. For example, for the classical format, following the Kabat criteria, the CDR amino acid residues in the heavy chain variable domain (VH) are numbered as 31-35 (HCDR1), 50-65 (HCDR2) and 95-102 (HCDR3); the CDR amino acid residues in the light chain variable domain (VL) are numbered as 24-34 (LCDR1), 50-56 (LCDR2), and 89-97 (LCDR3). Following the Chothia criteria, the CDR amino acid residues in VH are numbered as 26-32 (HCDR1), 52-56 (HCDR2) and 95-102 (HCDR3); and the amino acid residues in VL are numbered as 26-32 (LCDR1), 50- 52 (LCDR2) and 91-96 (LCDR3). By combining both Kabat and Chothia to define CDR, CDRs are composed of amino acid residues 26-35 (HCDR1), 50-65 (HCDR2) and 95-102 (HCDR3) in the human VH and amino acid residues 24-34 (LCDR1), 50-56 (LCDR2) and 89-97 (LCDR3) in the human VL. Following IMGT criteria, the CDR amino acid residues in VH are usually numbered as 26-35 (CDR1), 51-57 (CDR2) and 93-102 (CDR3), and the CDR amino acid residues in VL are usually numbered as 27-32 (CDR1), 50-52 (CDR2) and 89-97 (CDR3). Following IMGT criteria, the CDR regions of an antibody can be determined by using IMGT/DomainGap Align Program.

The antibodies of the present disclosure include murine antibodies, chimeric antibodies, and humanized antibodies, preferably humanized antibodies.

As used herein, the term "murine antibody" refers to anti-human PD-L1 monoclonal antibodies prepared according to the knowledge and skills in the art. During the preparation, test subject is injected with CD73 antigen, and then a hybridoma expressing the antibody which possesses desired sequence or functional characteristics is isolated. In a preferable embodiment of the present disclosure, the murine CD73 antibody or antigen binding fragment thereof further comprises a light chain constant region of murine κ, λ chain or variant(s) thereof, or further comprises a heavy chain constant region of murine IgG1, IgG2, IgG3, IgG4 or variant(s) thereof.

The term "chimeric antibody" is an antibody formed by fusing antibody variable region(s) of a species (such as murine) to antibody constant region(s) of another species (such as human), and the chimeric antibody can alleviate the murine antibody-induced immune response. To establish a chimeric antibody, a hybridoma secreting specific murine monoclonal antibody is established firstly and a variable region gene is cloned from the murine hybridoma; then a constant region gene is cloned from human antibody according to the need; The murine variable region gene is connected to the human constant region gene to form a chimeric gene, which can be subsequently inserted into an expression vector. Finally, the chimeric antibody molecule will be expressed in eukaryotic or prokaryotic system. In a preferable embodiment of the present disclosure, the antibody light chain of the CD73 chimeric antibody further comprises a light chain constant region of a human kappa, lambda chain or variant(s) thereof. The antibody heavy chain of the CD73 chimeric antibody further comprises a heavy chain constant region of human IgG1, IgG2, IgG3, IgG4 or variant(s) of IgG1, IgG2, IgG3, IgG4; preferably comprises a heavy chain constant region of human IgG1, IgG2 or IgG4, or variant(s) of IgG1, IgG2 or IgG4 with amino acid mutation(s) (such as YTE or S228P, etc.).

The term "humanized antibody", including CDR-grafted antibody, refers to an antibody generated by grafting murine CDR sequences into human antibody variable region frameworks, i.e., an antibody produced in different types of human germline antibody framework sequences. Humanized antibody can avoid heterologous responses induced by chimeric antibody which carries a large number of murine protein components. Such framework sequences can be obtained from public DNA database covering germline antibody gene sequences or published references. For example, germline DNA sequences of human heavy and light chain variable region genes can be found in "VBase" human germline sequence database (available on www.mrccpe.com.ac.uk/vbase), as well as in Kabat, EA, et al. 1991 Sequences of Proteins of Immunological Interest, 5th Ed. To avoid a decrease in activity caused by the decreased immunogenicity, the framework sequences in human antibody variable region can be subjected to minimal reverse mutation(s) or back mutation(s) to maintain the activity. The humanized antibody of the present disclosure also refers to humanized antibody on which CDR affinity maturation is performed by phage display. To avoid a decrease in activity caused by the decreased immunogenicity, the human antibody variable region can be subjected to minimal reverse mutation(s) (back mutation(s), which means that the amino acid residue(s) on the FR region derived from the human antibody is/are mutated into the amino acid residue(s) of the original source antibody on the corresponding positions) to maintain the activity.

The grafting of CDR can result in the decrease of the affinity of CD73 antibody or antigen binding fragment thereof to the antigen, due to the framework residues contacted with the antigen. Such interactions may be resulted from highly somatic mutations. Therefore, it may still be necessary to graft the donor framework amino acids onto the humanized antibody framework. The amino acid residues involved in antigen binding derived from non-human CD73 antibody or antigen binding fragment thereof can be identified by checking the sequence and structure of murine monoclonal antibody variable region. The amino acid residues which are different between the donor CDR framework and the germ lines may be considered to be related. If it is not possible to determine the most closely related germ line, the sequence may be aligned to the common sequence shared by subtypes or aligned to the common sequence of murine sequences sharing high similarity percentage. Rare framework residues are thought to be the result of a high mutation in somatic cells, and play an important role in binding.

The term "amino acid mutation" refers to the amino acid change or mutation in a protein or polypeptide variant when compared to the original protein or polypeptide, including one or more amino acid insertions, deletions or substitutions on the basis of the original protein or polypeptide. For example, "a variant with no more than 3 amino acid mutation(s)" refer to insertion, deletion or substitution of 3, 2, 1, or 0 amino acid(s), when compared with the original protein or polypeptide.

The term "antigen-binding fragment" or "functional fragment" of an antibody refers to one or more fragments of the antibody that retain the ability to specifically bind to an antigen (e.g., CD73). It has been shown that fragments of a full-length antibody can be used to achieve function of binding to a specific antigen. Examples of the binding fragments contained in the term "antigen-binding fragment" of an antibody include (i) Fab fragment, a monovalent fragment composed of VL, VH, CL and CH1 domains; (ii) F(ab')$_2$ fragment, a bivalent fragment formed by two Fab fragments connected by disulfide bridge(s) in the hinge region, (iii) Fv fragment composed of the VH and VL domains from one arm of the antibody; (iv) single domain or dAb fragment (Ward et al., (1989) Nature341: 544-546), composed of VH domain(s); (v) dsFv, a stable antigen-binding fragment formed by VH and VL via interchain disulfide bond(s); and (vi) diabody, bispecific antibody and multispecific antibody containing fragments such as scFv, dsFv, and Fab. In addition, though the VL domain and VH domain of the Fv fragment are encoded by two separate genes, they can be linked by a synthetic linker by using recombinant methods, to generate a single protein chain in which a monovalent molecular is formed by pairing the VL and VH domain (referred to as single chain Fv (scFv); see, e.g., Bird et al. (1988) Science 242:423-426; and Huston et al (1988) Proc. Natl. Acad. Sci USA 85:5879-5883). Such single chain antibodies are also intended to be included in the term of "antigen binding fragment" of an antibody. Such antibodies are obtained using conventional techniques known in the field, and are screened for functional fragments by using the same method as that for an intact antibody. Antigen binding portions can be produced by recombinant DNA technology or by enzymatic or chemical disruption of an intact immunoglobulin. Antibodies can be in the form of different isotypes, e.g., IgG (e.g., IgG1, IgG2, IgG3 or IgG4 subtype), IgA1, IgA2, IgD, IgE or IgM antibody.

The antigen-binding fragments of the present disclosure include Fab, F(ab')2, Fab', single-chain antibody (scFv), dimerized V region (diabody), disulfide stabilized V region (dsFv) and so on.

Fab is an antibody fragment obtained by treating an IgG antibody molecule with a papain (which cleaves the amino acid residue at position 224 of the H chain). The resulting fragment has a molecular weight of about 50,000 and has antigen binding activity, in which about a half of the N-terminal side of H chain and the entire L chain are bound together through a disulfide bond.

The Fab of the present disclosure can be produced by treating the monoclonal antibody of the present invention (which specifically recognizes human CD73 and binds to the extracellular region amino acid sequences or the three-dimensional structure thereof) with papain. Further, the Fab can be produced by inserting DNA encoding Fab of the antibody into a prokaryotic expression vector or eukaryotic expression vector and introducing the vector into a prokaryote or eukaryote to express the Fab.

"F(ab')$_2$" refers to an antibody fragment with a molecular weight of about 100,000 and antigen-binding activity, obtained by digesting the downstream part of the two disulfide bonds in the hinge region of IgG by pepsin. F(ab')$_2$ contains two Fabs connected at the hinge region.

The F(ab') 2 of the present disclosure can be produced by treating the monoclonal antibody of the present invention (which specifically recognizes human CD73 and binds to the extracellular region amino acid sequences or the three-dimensional structure thereof) with papain. Also, F(ab')2 can be produced by binding the Fab' described below via thioether bond or disulfide bond.

Fab' is an antibody fragment having a molecular weight of about 50,000 and having antigen binding activity, which is obtained by cleaving a disulfide bond at the hinge region of the above-mentioned F(ab')2. The Fab' of the present disclosure can be produced by treating the F(ab')2 of the present disclosure which specifically recognizes CD73 and binds to the extracellular region amino acid sequences or the three-dimensional structure thereof with a reducing agent, such as dithiothreitol.

Further, the Fab' can be produced by inserting DNA encoding Fab' of the antibody into a prokaryotic expression vector or eukaryotic expression vector and introducing the vector into a prokaryote or eukaryote to express the Fab'.

The term "single chain antibody", "single chain Fv" or "scFv" refers to a molecule comprising antibody heavy chain variable domain (or region; VH) connected to antibody light chain variable domain (or region; VL) by a linker. Such scFv molecules have general structure of NH2-VL-linker-VH-COOH or NH$_2$-VH-linker-VL-COOH. A suitable linker in the prior art consists of repeated GGGGS amino acid sequence or variant(s) thereof, for example, variant with 1-4 repeats (Holliger et al. (1993), Proc. Natl. Acad. Sci. USA 90: 6444-6448). Other linkers that can be used in the present disclosure are described by Alfthan et al. (1995), Protein Eng. 8:725-731, Choi et al. (2001), Eur. J. Immunol. 31:94-106, Hu et al. (1996), Cancer Res. 56:3055-3061, Kipriyanov et al. (1999), J. Mol. Biol. 293:41-56 and Roovers et al. (2001), Cancer Immunol.

The scFv of the present disclosure can be produced by the following steps: obtaining cDNAs encoding VH and VL of the monoclonal antibody of the present disclosure which specifically recognizes human CD73 and binds to the extracellular region or three-dimensional structure thereof, constructing DNA encoding scFv, inserting the DNA into a prokaryotic expression vector or eukaryotic expression vector, and then introducing the expression vector into a prokaryote or eukaryote to express the scFv.

Diabody is an antibody fragment wherein the scFv is dimerized, and it is an antibody fragment having divalent antigen binding activity. In the divalent antigen binding activity, the two antigens can be the same or different.

The diabody of the present disclosure can be produced by the following steps: obtaining cDNAs encoding VH and VL of the monoclonal antibody of the present disclosure which specifically recognizes human CD73 and binds to the extracellular region or three-dimensional structure thereof, constructing DNA encoding scFv so that the length of the linker peptide is 8 or less amino acid residues, inserting the DNA into a prokaryotic expression vector or eukaryotic expression vector, and then introducing the expression vector into a prokaryote or eukaryote to express the diabody. dsFv is obtained by substituting one amino acid residue in each of VH and VL with a cysteine residue, and then connecting the substituted polypeptides via a disulfide bond between the two cysteine residues. The amino acid residues to be substituted with a cysteine residue can be selected based on three-dimensional structure prediction of the antibody in accordance with known methods (Protein Engineering, 7, 697 (1994)).

The dsFv of the present disclosure can be produced by the following steps: obtaining cDNAs encoding VH and VL of the monoclonal antibody of the present disclosure which specifically recognizes human CD73 and binds to the extracellular region or three-dimensional structure thereof, constructing DNA encoding dsFv, inserting the DNA into a prokaryotic expression vector or eukaryotic expression vector, and then introducing the expression vector into a prokaryote or eukaryote to express the dsFv.

The CDR-containing peptide of the present disclosure can be produced by the following steps: constructing DNAs encoding VH and VL of the monoclonal antibody of the present disclosure which specifically recognizes human CD73 and binds to the extracellular region amino acid sequences or the three-dimensional structure thereof, inserting the DNA into a prokaryotic expression vector or eukaryotic expression vector, and then introducing the expression vector into a prokaryote or eukaryote to express the peptide. The CDR-containing peptide can also be produced by a chemical synthesis method such as Fmoc method or tBoc method.

As used herein, the term "antibody framework (FR)" refers to a part of the variable domain, either VL or VH, which serves as a scaffold for the antigen binding loops (CDRs) of this variable domain. Essentially, FR is a variable domain without CDRs.

The term "epitope" or "antigenic determinant" refers to a site on an antigen to which an immunoglobulin or antibody specifically binds (e.g., a specific site on CD73 molecule). Epitopes typically include at least 3, 4, 5, 6, 7, 8, 9, 10, 11, 12, 13, 14 or 15 successive or non-successive amino acids in a unique spatial conformation. See, for example, Epitope Mapping Protocols in Methods in Molecular Biology, Vol. 66, G.E. Morris, Ed. (1996).

The term "specifically bind to", "selectively bind to", "selectively binds to" or "specifically binds to" refers to the binding of an antibody to a predetermined epitope on an antigen. Typically, the antibody binds with an affinity (KD) of less than about $10^{-7}$M, for example, less than about $10^{-8}$ M, $10^{-9}$ M, $10^{-10}$ M or $10^{-11}$ M or even less.

The term "KD" or "Kd" refers to the dissociation equilibrium constant for particular antibody-antigen interaction. Typically, the antibody of the present disclosure binds to human CD73 with a dissociation equilibrium constant (KD) of less than about $10^{-7}$M, for example, less than about $10^{-8}$M, $10^{-9}$M or $10^{-10}$M or even less, for example, as determined by Surface Plasma Resonance (SPR) technology in Biacore instrument.

When the term "competition" is used in the context of antigen binding proteins (e.g., neutralizing antigen binding proteins or neutralizing antibodies) that compete for the same epitope, it means that competition occurs between the antigen binding proteins, which is determined by the assays wherein an antigen binding protein to be tested (e.g., an antibody or immunologically functional fragment thereof) prevents or inhibits (e.g., reduces) the specific binding of a reference antigen binding protein (e.g., a ligand or reference antibody) to a common antigen (e.g., a CD73 antigen or fragment thereof). Numerous types of competitive binding assays are available to determine whether an antigen binding protein competes with another. These assays are, for example, solid phase direct or indirect radioimmunoassay (RIA), solid phase direct or indirect enzyme immunoassay (EIA), Sandwich competition assay (see, e.g., Stahli et al, 1983, Methods in Enzymology 9: 242-253); solid phase direct biotin-avidin EIA (see, e.g., Kirkland et al, 1986, J. Immunol. 137: 3614-3619; Cheung, et al, 1990, Virology 176: 546-552), solid phase direct labeling assay, solid phase direct labeling sandwich assay (see, e.g., Harlow and Lane, 1988, Antibodies, A Laboratory Manual, Cold Spring Harbor Press); solid phase direct labeling RIA with 1-125 label (see, e.g., Morel et al, 1988, Molec. Immunol. 25: 7-15); solid phase direct biotin-avidin EIA (see, e.g., Cheung, et al, 1990, Virology 176: 546-552); and direct labeling RIA (Moldenhauer et al, 1990, Scand. J. Immunol. 32: 77-82). Typically, the assay involves the use of a purified antigen capable of binding to both an unlabeled test antigen binding protein and a labeled reference antigen binding protein (the antigen is on a solid surface or cell surface). Competitive inhibition is determined by measuring the amount of label bound to the solid surface or to the cell surface in the presence of the test antigen binding protein. Usually, the test antigen binding protein is present in excess. Antigen binding proteins identified by competitive assay (competing with the antigen binding protein) include: antigen binding proteins that bind to the same epitope as the reference antigen binding protein; and antigen binding proteins that bind to an epitope that is sufficiently close to the epitope to which the reference antigen binding protein binds, wherein the two epitopes spatially interfere with each other to hinder the binding. Additional details regarding methods for determining competitive binding are provided in the Examples herein. Typically, when a competitive antigen binding protein is present in excess, it will inhibit (e.g., reduce) at least 40-45%, 45-50%, 50-55%, 55-60%, 60-65%, 65-70%, 70-75% or even more of the specific binding of the reference antigen binding protein to the common antigen. In some cases, the binding is inhibited by at least 80-85%, 85-90%, 90-95%, 95-97% or even more.

As used herein, the term "nucleic acid molecule" refers to DNA molecules and RNA molecules. The nucleic acid molecule can be single-stranded or double-stranded, but preferably is double-stranded DNA. A nucleic acid is "operably linked" when it is placed into a functional relationship with another nucleic acid sequence. For instance, a promoter or enhancer is considered to be operably linked to a coding sequence, if it affects the transcription of the coding sequence.

The term "vector" refers to a nucleic acid molecule capable of transporting another nucleic acid to which it has been linked. In one embodiment, the vector is a "plasmid," which refers to a circular double stranded DNA loop into which additional DNA segments may be ligated. In another embodiment, the vector is a viral vector, wherein additional DNA segments may be ligated into the viral genome. The vectors disclosed herein are capable of self-replicating in the host cell into which they are introduced (e.g., bacterial vectors having a bacterial replication origin and episomal mammalian vectors), or may be integrated into the genome of a host cell upon introduction into the host cell, and thereby are replicated along with the host genome (e.g., non-episomal mammalian vectors).

Methods for producing and purifying antibodies and antigen-binding fragments are well known in the art, for example, Antibodies: A Laboratory Manual, Cold Spring Harbor, chapters 5-8 and 15. For example, mice can be immunized with human CD73 or fragment thereof, and the resulting antibodies can then be renatured, purified, and sequenced for amino acid sequences by using conventional methods well known in the art. Antigen-binding fragments can also be prepared by conventional methods. The antibodies or the antigen binding fragments of the present disclosure are engineered to incorporate one or more human framework regions onto the CDR regions derived from non-human antibody. Human FR germline sequences can be obtained from ImMunoGeneTics (IMGT) via their website http://imgt.cines.fr, or from The Immunoglobulin Facts, 2001, ISBN012441351, by aligning against IMGT human antibody variable germline gene database using MOE software.

The term "host cell" refers to a cell into which an expression vector has been introduced. Host cells can include bacterial, microbial, plant or animal cells. Bacteria that are susceptible to be transformed include members of *enterobacteriaceae,* such as *Escherichia coli* or *Salmonella* strains; *Bacillaceae* such as *Bacillus subtilis; Pneumococcus; Streptococcus* and *Haemophilus influenzae.* Suitable microorganisms include *Saccharomyces cerevisiae* and *Pichia pastoris.* Suitable animal host cell lines include CHO (Chinese Hamster Ovary Cell Line) and NS0 cells.

The engineered antibodies or antigen binding fragments of the present disclosure can be prepared and purified using known methods. For example, cDNA sequences encoding a heavy chain and a light chain can be cloned and engineered into a GS expression vector. The vectors expressing recombinant immunoglobulin may then be stably transfected into CHO cells. As a more recommended prior art, mammalian expression systems can lead to glycosylation of antibodies, especially in the highly conserved N-terminal sites of the Fc region. Stable clones were obtained by expressing an antibody specifically binding to human CD73. Positive clones may be expanded in serum-free culture medium in bioreactors for antibody production. Culture medium, into which an antibody has been secreted, may be purified by conventional techniques. For example, purification may be performed on Protein A or G Sepharose FF column that has been modified with buffer. The nonspecific binding components are washed out. The bound antibody is eluted by pH gradient and antibody fragments are detected by SDS-PAGE, and then pooled. The antibodies may be filtered and concentrated using common techniques. Soluble mixtures and aggregates may be effectively removed by common techniques, such as size exclusion or ion exchange. The resulting product is then immediately frozen, for example at -70°C, or may be lyophilized.

"Administration" and "treatment," as it applies to an animal, human, experimental subject, cell, tissue, organ, or biological fluid, refers to contacting an exogenous pharmaceutical, therapeutic, diagnostic agent, or composition with the animal, human, subject, cell, tissue, organ, or biological fluid. "Administration" and "treatment" can refer, e.g., to therapeutic, pharmacokinetic, diagnostic, research, and experimental methods. Treatment of a cell involves contacting a reagent with a cell, as well as contacting a reagent with a fluid, where the fluid is in contact with the cell. "Administration" and "treatment" also means *in vitro* or *ex vivo* treatments, e.g., of a cell, with a reagent, diagnostic, binding composition, or with another cell. "Treatment", as it applies to a human, veterinary, or research subject, refers to therapeutic treatment, prophylactic or preventative measures, research and diagnostic applications.

As used herein, "treat" means to administer a therapeutic agent, such as a composition containing any of binding compounds of the present disclosure, internally or externally to a patient having one or more disease symptoms for which the agent has known therapeutic activity. Typically, the agent is administered in an amount effectively to alleviate one or more disease symptoms in the patient or population to be treated, by inducing the regression of or inhibiting the progression of such symptom(s) by any clinically measurable degree. The amount of a therapeutic agent that is effective to alleviate any particular disease symptom (also referred to as the "therapeutically effective amount") may vary according to various factors such as the disease state, age, and body weight of the patient, and the ability of the drug to elicit a desired response in the patient. Whether a disease symptom has been alleviated can be assessed by any clinical measurement typically used by physicians or other skilled healthcare providers to assess the severity or progression status of that symptom. While an embodiment of the present disclosure (e.g., a treatment method or article of manufacture) may not be effective in alleviating the target disease symptom(s) in every subject, it should alleviate the target disease symptom(s) in a statistically significant number of subjects as determined by any statistical test known in the art such as Student's t-test, chi-square test, U-test according to Mann and Whitney, Kruskal-Wallis test (H-test), Jonckheere-Terpstra-test and Wilcoxon-test.

"Conservative modification" or "conservative substitution or replacement" refers to substitutions of amino acids in a protein with other amino acids having similar characteristics (e.g. charge, side-chain size, hydrophobicity/hydrophilicity, backbone conformation and rigidity, etc.), such that the changes can frequently be made without altering the biological activity of the protein. Those skilled in the art recognize that, in general, single amino acid substitution in non-essential regions of a polypeptide does not substantially alter biological activity (see, e.g., Watson et al. (1987) Molecular Biology of the Gene, The Benjamin/Cummings Pub. Co., p. 224 (4th Ed.)). In addition, substitutions with structurally or functionally similar amino acids are less likely to disrupt biological activity. Exemplary conservative substitutions are set forth in the table below.

| Original residue | Conservative substitution |
| --- | --- |
| Ala (A) | Gly; Ser |
| Arg (R) | Lys; His |
| Asn (N) | Gln; His; Asp |
| Asp (D) | Glu; Asn |
| Cys (C) | Ser; Ala; Val |
| Gln(Q) | Asn; Glu |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro(P) | Ala |
| Ser(S) | Thr |
| Thr (T) | Ser |
| Trp (W) | Tyr; Phe |
| Tyr (Y) | Trp; Phe |
| Val (V) | Ile; Leu |

"Effective amount" encompasses an amount sufficient to ameliorate or prevent a symptom or sign of the medical condition. Effective amount also means an amount sufficient to allow or facilitate diagnosis. An effective amount for a particular patient or veterinary subject may vary depending on factors such as the condition being treated, the overall health condition of the patient, the route and dose of administration and the severity of side effects. An effective amount can be the maximal dose or dosing protocol that avoids significant side effects or toxic effects.

"Exogenous" refers to substances produced outside organisms, cells, or humans according to circumstances. "Endogenous" refers to substances produced in cells, organisms, or human bodies according to circumstances.

The "mutated sequence" mentioned in the present disclosure refers to the nucleotide sequence or amino acid sequence having various percentage sequence identity to those of the present disclosure, after modifying the nucleotide sequence and amino acid sequence of the present disclosure by appropriate substitution, insertion or deletion. The sequence identity described in the present disclosure can be at least 85%, 90% or 95%, non-limiting examples include 85%, 86%, 87%, 88%, 89%, 90%, 91%, 92%, 93%, 94%, 95%, 96%, 97%, 98%, 99%, 100%.

"Homology", "identity", and "consistency" are interchangeable herein and refer to the sequence similarity between two polynucleotide sequences or between two polypeptide sequences. When a position in both of the two sequences to be compared is occupied by the same base or amino acid monomer subunit, e.g., if a position in each of two DNA molecules is occupied by the same base, then the molecules are deemed to be homologous at that position. The percent of homology between two sequences is a function of the number of matching or homologous positions shared by the two sequences divided by the number of all positions to be compared, and then multiplied by 100. For example, when two sequences are optimally aligned, if 6 out of 10 positions in the two sequences are matched or homologous, then the two sequences are 60% homologous; if 95 out of 100 positions in the two sequences are matched or homologous, then the two sequences are 95% homologous. Generally, the comparison is performed when two sequences are aligned to give maximum percent homology. In some specific embodiments of the present disclosure, the antibody sequence has at least 85% sequence identity; in other specific embodiments, at least 86%, 87%, 88%, 89%, 90%, 91%, 92%, 93%, 94%, 95%, 96%, 97%, 98%, or 99% sequence identity; in other specific embodiments, 90%, 95%, or 99% or more sequence identity; in other specific embodiments, at least 95% sequence identity. The above-mentioned amino acid sequence having particular sequence identity can be obtained from deletion, insertion or substitution of one or more amino acid(s).

As used herein, the expressions "cell," "cell line," and "cell culture" are used interchangeably and all such designations include progeny thereof. Thus, the words "transformant" and "transformed cell" include the primary subject cells and cultures derived therefrom, regardless of the number of passages. It should be also understood that all progeny may not be precisely identical in DNA content, due to intended or unintended mutation. Mutant progeny thus screened that has the same function or biological activity as that of the originally transformed cells are included in the scope of this term. Where distinct designations are intended to, it will be clearly understood from the context.

As used herein, "polymerase chain reaction" or "PCR" refers to a procedure or technique in which minute amounts of a specific portion of nucleic acid, RNA and/or DNA, are amplified as described in, e.g., U.S. Pat. No. 4,683,195. Generally, sequence information at the ends of or beyond the region of interest is available, such that oligonucleotide primers can be designed; these primers will be identical or similar in sequence to opposite strands of the template to be amplified. The 5' terminal nucleotides of the two primers are identical to the ends of the material to be amplified. PCR can be used to amplify specific RNA sequences, specific DNA sequences from total genomic and cDNA transcribed from total cellular RNA, bacteriophage or plasmid sequences, etc. See generally Mullis et al. (1987) Cold Spring Harbor Symp. Ouant. Biol. 51:263; Erlich ed., (1989) PCR TECHNOLOGY (Stockton Press, NY). The PCR used in the present disclosure is considered to be one, but not the only, example of polymerase reaction method for amplifying a test nucleic acid sample. The method comprises the use of known nucleic acid sequences served as primers together with nucleic acid polymerase to amplify or generate a specific portion of nucleic acid.

"Optional" or "optionally" means that the event or circumstance that follows can occur, but does not necessarily occur, and the description includes the instances in which the event or circumstance does or does not occur. For example, "optionally contains 1-3 antibody heavy chain variable regions" means the antibody heavy chain variable region with specific sequence can, but not necessarily, be present.

"Pharmaceutical composition" refers to a mixture containing one or more antibodies or the antigen binding fragments thereof according to the present disclosure and other chemical components, such as physiologically/pharmaceutically acceptable carrier(s) and excipient(s). The pharmaceutical composition aims at promoting the administration to an organism, facilitating the absorption of the active ingredient and thereby exerting a biological effect.

As used herein, "prevention" includes delaying the development of disease-related symptom(s) and/or reducing the severity of these symptoms that the disease will develop or is expected to develop. The term also includes alleviating the existing symptom(s), preventing additional symptom(s), and alleviating or preventing the factors underlying these symptoms. Therefore, the term means that a beneficial result has been conferred to a vertebrate subject suffering from a disease.

In addition, the present disclosure includes an agent for treating a CD73-related disease, the agent comprising the monoclonal antibody or the antibody fragment thereof of the present disclosure as an active ingredient.

The CD73-related diseases are not to be limited, as long as the disease is related to CD73 (e.g., a disease caused by CD73-expressing cells, or caused by abnormal expression of CD73), for example, a cell proliferative disease that can be suppressed by the molecule of the present disclosure binding to human CD73. In some embodiments, the cell proliferative disease is a tumor. In some embodiments, the tumor is breast cancer, glioma, bladder cancer, colon cancer, melanoma, ovarian cancer, thyroid cancer, esophageal cancer, lung cancer, kidney cancer, pancreatic cancer, lymphoma, prostate cancer, or gastric cancer. In some embodiments, the tumor is a metastatic tumor.

In addition, the present disclosure relates to methods for immunodetection or determination of CD73, reagents for immunodetection or determination of CD73, methods for immunodetection or determination of CD73-expressing cells, and the diagnostic agents for diagnosing diseases associated with CD73, comprising the antibody or the antibody fragment of the present disclosure that specifically recognizes human CD73 as an active ingredient.

In the present disclosure, the method for detecting or measuring the amount of CD73 may be any known method. For example, immunoassay or immunodetection method.

The immunoassay or immunodetection method is a method of detecting or measuring the amount of an antibody or antigen, by using a labeled antigen or antibody. Examples of immunoassay or immunodetection methods include radioactive substance-labeled immuno-antibody method (RIA), enzyme immunoassay (EIA or ELISA), fluorescence immunoassay (FIA), luminescence immunoassay, western blotting, physicochemical method, and the like.

The above-mentioned CD73-related diseases can be diagnosed by detecting or measuring CD73-expressing cells using the antibodies or antibody fragments of the present disclosure.

Cells expressing the polypeptide can be detected by the known immunodetection methods, e.g. by immunoprecipitation, fluorescent cell staining, immunotissue staining, and the like. In addition, the method such as fluorescent antibody staining method with the FMAT8100HTS system (Applied Biosystem) can be used.

In the present disclosure, samples to be detected or measured for CD73 are not particularly limited, as long as they are possible to contain CD73-expressing cells, such as tissue cells, blood, plasma, serum, pancreatic juice, urine, stool, tissue fluid or culture medium.

Dependent on the required diagnostic method, the diagnostic agent comprising the antibody or the antibody fragment thereof of the present disclosure can also comprise reagents for performing an antigen-antibody reaction or reagents for detecting the reaction. The reagents for performing an antigen-antibody reaction include buffers, salts and the like. The reagents for detection include agents commonly used in immunoassay or immunodetection methods, for example, a labeled secondary antibody that recognizes the monoclonal antibody, the antibody fragment or the conjugate thereof, and a substrate corresponding to the label.

The anti-CD73 antibodies or the antigen-binding fragments provided in the examples of the present disclosure are specific to CD73 and have high affinity with human CD73, wherein the humanized anti-CD73 antibodies or the antigen-binding fragments exhibit a greatly reduced immunogenicity and completely maintain the specificity of the murine antibodies, have higher affinity and excellent *in vivo* and *in vitro* activity.

The anti-CD73 antibodies or antigen-binding fragments provided in the examples of the present disclosure have favorable metabolic kinetic characteristics and bioavailability.

## DETAILED DESCRIPTION OF THE INVENTION

The following examples are provided to further describe the present disclosure, but are not intended to limit the scope of the disclosure. Experimental methods for which the specific conditions are not indicated in the examples of the present disclosure are generally carried out according to conventional conditions, such as Sambrook et al., Antibodies: Laboratory Manual, Molecular Cloning, Cold Spring Harbor; or according to the conditions recommended by the manufacturers of materials or products. Reagents for which the sources are not specifically indicated are commercially available reagents.

### Example 1. Preparation of CD73 antigen

### I. Design and expression of antigen

Human CD73 protein (Uniprot number: P21589) was used as the template of CD73 of the present disclosure to design the amino acid sequence of the antigen and detection protein involved in the present disclosure. Optionally, the CD73 protein was fused with various tags, and separately cloned into pTT5 vector or pTarget vector, and transiently expressed in 293 cells or stably expressed in CHO cells to result in the antigens and detection proteins of the present disclosure. The following CD73 antigens refer to human CD73, unless specifically indicated.

Full-length sequence of human CD73 can be used to construct CD73 over-expressing cell lines for immunization and detection, the specific sequence of which is as shown in SEQ ID NO:1:

MCPRAARAPATLLLALGAVLWPAAGA*WELTILHTNDVHSRLEQTSEDSSKCVN ASRCMGGVARLFTKVQQIRRAEPNVLLLDAGDQYQGTIWFTVYKGAEVAHFMNA LRYDAMALGNHEFDNGVEGLIEPLLKEAKFPILSANIKAKGPLASQISGLYLPYKVL PVGDEVVGIVGYTSKETPFLSNPGTNLVFEDEITALQPEVDKLKTLNVNKIIALGHS GFEMDKLIAQKVRGVDVVVGGHSNTFLYTGNPPSKEVPAGKYPFIVTSDDGRKVP VVQAYAFGKYLGYLKIEFDERGNVISSHGNPILLNSSIPEDPSIKADINKWRIKLDNY STQELGKTIVYLDGSSQSCRFRECNMGNLICDAMINNNLRHTDEMFWNHVSMCIL NGGGIRSPIDERNNGTITWENLAAVLPFGGTFDLVQLKGSTLKKAFEHSVHRYGQS TGEFLQVGGIHVVYDLSRKPGDRVVKLDVLCTKCRVPSYDPLKMDEVYKVILPNF LANGGDGFQMIKDELLRHDSGDQDINVVSTYISKMKVIYPAVEGRIK*FSTGSHCH GSFSLIFLSLWAVIFVLYQ*

(Note: The single underlined portion represents signal peptide, the italic represents the extracellular region of human CD73, and the double underlined portion represents propeptide.)

CD73-3Flag (a fusion protein of the CD73 mature protein extracellular domain and three Flag tags) can be used for immunization, the specific sequence of which is as shown in SEQ ID NO: 2:

MEFGLSWLFLVAILKGVQC*WELTILHTNDVHSRLEQTSEDSSKCVNASRCMGGV*
*ARLFTKVQQIRRAEPNVLLLDAGDQYQGTIWFTVYKGAEVAHFMNALRYDAMAL*
*GNHEFDNGVEGLIEPLLKEAKFPILSANIKAKGPLASQISGLYLPYKVLPVGDEVVG*
*IVGYTSKETPFLSNPGTNLVFEDEITALQPEVDKLKTLNVNKIIALGHSGFEMDKLI*
*AQKVRGVDVVVGGHSNTFLYTGNPPSKEVPAGKYPFIVTSDDGRKVPVVQAYAFG*
*KYLGYLKIEFDERGNVISSHGNPILLNSSIPEDPSIKADINKWRIKLDNYSTQELGKTI*
*VYLDGSSQSCRFRECNMGNLICDAMINNNLRHTDEMFWNHVSMCILNGGGIRSPI*
*DERNNGTITWENLAAVLPFGGTFDLVQLKGSTLKKAFEHSVHRYGQSTGEFLQVG*
*GIHVVYDLSRKPGDRVVKLDVLCTKCRVPSYDPLKMDEVYKVILPNFLANGGDGF*
*QMIKDELLRHDSGDQDINVVSTYISKMKVIYPAVEGRIK*DYKDHDGDYKDHDID
YKDDDDK

(Note: The single underlined portion represents signal peptide, the italic represents the extracellular region of human CD73, and the double underlined portion represents the three Flag tags)

CD73-Myc-His (a fusion protein of the CD73 mature protein extracellular domain and Myc tag and His tag) can be used for detection, the specific sequence of which is as shown in SEQ ID NO: 3:

MEFGLSWLFLVAILKGVQCWELTILHTNDVHSRLEQTSEDSSKCVNASRCMG
GVARLFTKVQQIRRAEPNVLLLDAGDQYQGTIWFTVYKGAEVAHFMNALRY
DAMALGNHEFDNGVEGLIEPLLKEAKFPILSANIKAKGPLASQISGLYLPYKV
LPVGDEVVGIVGYTSKETPFLSNPGTNLVFEDEITALQPEVDKLKTLNVNKIIA
LGHSGFEMDKLIAQKVRGVDVVVGGHSNTFLYTGNPPSKEVPAGKYPFIVTS
DDGRKVPVVQAYAFGKYLGYLKIEFDERGNVISSHGNPILLNSSIPEDPSIKAD
INKWRIKLDNYSTQELGKTIVYLDGSSQSCRFRECNMGNLICDAMINNNLRHT
DEMFWNHVSMCILNGGGIRSPIDERNNGTITWENLAAVLPFGGTFDLVQLKG
STLKKAFEHSVHRYGQSTGEFLQVGGIHVVYDLSRKPGDRVVKLDVLCTKCR
VPSYDPLKMDEVYKVILPNFLANGGDGFQMIKDELLRHDSGDQDINVVSTYI
SKMKVIYPAVEGRIKEQKLISEEDLHHHHHH

(Note: The single underlined portion represents signal peptide, the italic represents the extracellular region of human CD73, and the double underlined portion represents Myc-His tag.)

In addition, the cynomolgus monkey CD73/NT5E protein (His tag) was purchased from Sino Biological (Cat. No. 90192-C08H-50).

## II. Purification of CD73-related recombinant proteins, and purification of anti-human CD73 hybridoma antibodies and recombinant antibodies

### 1. Separation and purification of hybridoma supernatant by Protein G affinity chromatography

Purification of mouse hybridoma supernatant was preferably performed with ProteinG affinity chromatography. The cultured hybridoma was centrifuged and the supernatant was taken; based on the volume of the supernatant, 10-15% (by volume) of 1M Tris-HCl (pH 8.0-8.5) was added into the the supernatant to adjust the pH to neutral. Protein G column was washed with 3-5 x column volume of 6M guanidine hydrochloride, and then washed with 3-5 x column volume of pure water; the column was equilibrated with 3-5 x column volume of equilibrium buffer such as 1×PBS (pH7.4) buffer system; the cell supernatant was loaded at a low flow rate for binding, and the flow rate was controlled so that the retention time was about 1 min or longer; the column was washed with 3-5 x column volume of 1×PBS (pH 7.4) until the UV absorption dropped to the baseline; The samples were eluted with 0.1M acetic acid/sodium acetate (pH3.0) buffer, the elution peaks were pooled according to UV detection. The eluted product was preserved for later use, after the pH was adjusted to 5-6 with 1M Tris-HCl (pH8.0). The eluted product can be subjected to solution replacement according to methods well-known to those skilled in the art, for example, replacing the solution with a desired buffer system by ultrafiltration-concentration using an ultrafiltration tube, or replacing the solution with a desired buffer system by using molecular exclusion such as G-25 de-salting column, or removing the aggregation components from the eluted product to improve the purity of the sample by using high-resolution molecular exclusion column (such as Superdex 200).

**2. Purification of antibodies with Protein A affinity Chromatography**

First, the cell culture supernatant expressing the antibody was centrifuged at a high speed to collect the supernatant. The Protein A affinity column was washed with 3-5 x column volume of 6M guanidine hydrochloride, and then washed with 3-5 x column volume of pure water. The column was equilibrated with 3-5 x column volume of 1×PBS (pH7.4) buffer system as equilibrium buffer. The cell supernatant was loaded at a low flow rate, and the flow rate was controlled so that the retention time was about 1 min or longer; after the binding was finished, the column was washed with 3-5 x column volume of 1×PBS (pH 7.4) until the UV absorption dropped to the baseline. Samples were eluted with 0.1M acetic acid/sodium acetate (pH3.0-3.5) buffer, the elution peaks were pooled according to UV detection. The eluted product was preserved for later use after the pH was adjusted to 5-6 with 1M Tris-HCl (pH8.0). The eluted product can be subjected to solution replacement according to methods well-known to those skilled in the art, for example, replacing the solution with a desired buffer system by ultrafiltration-concentration using an ultrafiltration tube, or replacing the solution with a desired buffer system by using molecular exclusion such as G-25 desalting column, or removing the aggregation components from the eluted product to improve the purity of the sample by using high-resolution molecular exclusion column (such as Superdex 200).

**3. Purification of human CD73, CD73-Myc-His, CD73-3Flag and other CD73-related recombinant proteins by nickel column**

The cell expression supernatant sample was centrifuged at high speed to remove impurities, the buffer was replaced with PBS, and imidazole was added to a final concentration of 5mM. The nickel column was equilibrated with PBS solution containing 5mM imidazole, and washed with 2-5 x column volume. The supernatant sample after replacement was loaded onto the column for binding, and the selected medium can be nickel columns of different companies. The column was washed with PBS solution containing 5 mM imidazole until the A280 reading dropped to the baseline. The chromatography column was rinsed with PBS+10mM imidazole to remove the non-specifically bound proteins, and the effluent was collected. The target protein was eluted with PBS solution containing 300 mM imidazole, and the elution peak was collected.

The collected eluted product can be further purified by gel chromatography Superdex200 (GE) after concentration, with PBS served as the mobile phase, aggregates and impurity protein peaks were removed, and the elution peak of the target product was collected. The resulting protein was identified by electrophoresis, peptide mapping and LC-MS, and the verified proteins were aliquoted for use.

**Example 2. Preparation of anti-human CD73 monoclonal antibody**

**I. Immunization**

The anti-human CD73 monoclonal antibodies were produced by immunizing mice. SJL white mice, female, 6-8 weeks old were used for experiment (Beijing Charles River Laboratory Animal Technology Co., Ltd., animal production license number: SCXK (Beijing) 2012-0001). Feeding environment: SPF level. After purchased, the mice were kept in the laboratory environment for 1 week, 12/12 hours light/dark cycle, at temperature of 20-25 °C; humidity of 40-60%. Then, the mice that had been adapted to the environment were immunized according to the following schemes. The immune antigen was CHO cells overexpressing CD73 (SEQ ID NO:1).

Immunization protocol: mice were immunized with CD73-overexpressing CHO cell line, $1\times10^7$ cells/mouse/time, via intraperitoneal injection. The cells were collected and were diluted to $1\times10^8$/ml with PBS, 100 μl/mouse was injected intraperitoneally (IP) on day 0, and then booster immunization was performed every 14 days. Blood samples were collected on day 21, 35, 49 and 63, the antibody titer in mouse serum was determined by ELISA method. After 7 to 9 immunizations, mice that had a high serum antibody titer reaching to a plateau were selected for splenocyte fusion. Three days before the splenocyte fusion, CD73-3Flag antigen solution prepared with saline was intraperitoneally injected (IP), 50 μg/mouse, for booster immunization.

**II. Spleen cell fusion**

Hybridoma cells were obtained by fusing splenic lymphocytes with myeloma Sp2/0 (ATCC® CRL-8287™) by using an optimized PEG-mediated fusion procedure. The fused hybridoma cells were resuspended in complete medium (DMEM medium comprising 20% FBS, 1×HAT, 1×OPI) at a density of 0.5-1×10$^6$ cells/ml, seeded in a 96-well plate with 100 μl/well, incubated at 37°C and 5% $CO_2$ for 3-4 days, supplemented with 100μl/well of HAT complete medium, and continued to be cultured for 3-4 days until forming pinpoint-like clones. The supernatant was removed, 200μl/well of HT complete medium (RPMI-1640 medium comprising 20% FBS, 1×HT and 1×OPI) was added, and incubated at 37°C, 5% $CO_2$ for 3 days and then the cells were subjected to ELISA detection.

**III. Screening of Hybridoma cells**

According to the growth density of hybridoma cells, the hybridoma culture supernatant was detected by binding ELISA method (see Example 3). MDA-MB-231 cell binding assay and cell enzyme activity inhibition assay were performed on cell supernatant collected from positive wells detected by binding ELISA assay (see Example 4). Some of the cells positive for both MDA-MB-231 cell binding assay and cell enzyme activity inhibition assay were timely expanded and cryopreserved; others were sub-cloned for 2 to 3 times until a single cell clone was obtained.
It was also required to perform CD73 binding ELISA, MDA-MB-231 cell binding assay, MDA-MB-231 cell enzyme activity inhibition assay for each subcloning. The hybridoma clones were obtained by screening via the above assays. The antibodies were further prepared by a method using serum-free cell culture. The antibodies were purified according to the Purification Example and used for Test Examples.

**IV. Sequencing of the positive hybridoma clones**

The procedures of cloning the sequences from the positive hybridoma were as follows. Hybridoma cells in logarithmic growth phase were collected. RNAs were extracted with Trizol (Invitrogen, Cat No. 15596-018) according to the kit's instruction and were reversely transcribed with PrimeScript™ Reverse Transcription kit (Takara, Cat No. 2680A). The cDNAs resulting from reverse transcription were amplified by PCR using mouse Ig-Primer Set (Novagen, TB326 Rev. B 0503), and the amplified products were sent to a company for sequencing. After sequencing, murine anti-CD73 antibodies mab108, mab110 and mab127 were obtained. The amino acid sequences of the variable regions are as follows:

mab108 HCVR amino acid sequence is as shown in SEQ ID NO: 4,

*QVQLKQSGPGLVQPSQSLSITCTVFGFSLK*<u>SYGIQ</u>*WVRQSPGKGLEWLG*<u>VIWSGG SADYNAAFIS</u>*RLSISKDNSKSQVFFKMNSLQGDDTAIYYCAG*<u>QYGSV</u>*WGQGTSVT VSS*

mab108 LCVR amino acid sequence is as shown in SEQ ID NO: 5,

*DIQMTQSPSSLSASLGERVSLTC*<u>RASQDIGDRLN</u>*WLQQEPDGTIKRLIY*<u>ATSSLDS</u>*GVPKRFSGSRSGSDYSLTISSLESEDFVDYYC*<u>LQYAGSWT</u>*FGGGTKLEIK*

mab110 HCVR amino acid sequence is as shown in SEQ ID NO: 6,

QVQLQQSGAELARPGASVQMSCKASGYIFT<u>SYTMH</u>WVKQRPGQGLEWIG<u>YI NPNSFYIEYNQKFKD</u>KATLTADTSSSTAYMQLSSLTSEDSAVYYCAR<u>GDYDL FYDMDN</u>WGQGTSVTVSS

mab110 LCVR amino acid sequence is as shown in SEQ ID NO: 7,

*DIQMTQSPSSLSASLGERVSLTC<u>RASQDIGNSLN</u>WLQQEPDGTIKRLIY<u>ATFRLDS</u> GVPKRFSGSRSGSDYSLTISSLESEDFVDYYC<u>QQYASFPWT</u>FGGGTKLEIK*

mab127 HCVR amino acid sequence is as shown in SEQ ID NO: 8,

*EVQLQQSGPELVKPGESVKISCKASGYSFT<u>AYFMN</u>WVMQSHRKSLEWIG<u>RIYHN NGDTFYSQKFKG</u>KATLTVDKSSNTVHMELRSLTSEDSAVYFCAT<u>SYVGD</u>WGQGT TLTVSS*

mab127 LCVR amino acid sequence is as shown in SEQ ID NO: 9,

*DIQMTQSPSSLSASLGERVSLTC<u>RASQDIGDSLN</u>WLQQAPDGTLKRLIY<u>ATSSLDS</u> GVPRRFSGSRSGSDYSLTISSLESEDFVDYYC<u>LQYASFPWT</u>FGGGTKLEIK*

In the above-mentioned mab108, mab110, and mab127 antibody variable region sequences, the FR sequence is in italics, the underline is the CDR sequence determined according to Kabat numbering criteria, and the order of sequence is FR1-CDR1-FR2-CDR2-FR3-CDR3-FR4.

Table 1 A list of amino acid sequences for anti-CD73 antibody heavy chain and light chain CDR regions

| Antibody | Heavy chain | | Light chain | |
|---|---|---|---|---|
| mab108 | HCDR 1 | SYGIQ SEQ ID NO: 10 | LCDR1 | RASQDIGDRLN SEQ ID NO: 13 |
| | HCDR 2 | VIWSGGSADYNAAFIS SEQ ID NO: 11 | LCDR2 | ATSSLDS SEQ ID NO: 14 |
| | HCDR 3 | QYGSV SEQ ID NO: 12 | LCDR3 | LQYAGSWT SEQ ID NO: 15 |
| mab110 | HCDR 1 | SYTMH SEQ ID NO: 16 | LCDR1 | RASQDIGNSLN SEQ ID NO: 19 |
| | HCDR 2 | YINPNSFYIEYNQKFKD SEQ ID NO: 17 | LCDR2 | ATFRLDS SEQ ID NO: 20 |
| | HCDR 3 | GDYDLFYDMDN SEQ ID NO: 18 | LCDR3 | QQYASFPWT SEQ ID NO: 21 |
| mab127 | HCDR 1 | AYFMN SEQ ID NO: 22 | LCDR1 | RASQDIGDSLN SEQ ID NO: 25 |
| | HCDR 2 | RIYHNNGDTFYSQKFKG SEQ ID NO: 23 | LCDR2 | ATSSLDS SEQ ID NO: 14 |
| | HCDR 3 | SYVGD SEQ ID NO: 24 | LCDR3 | LQY ASFPWT SEQ ID NO: 26 |

The resulting antibodies mab108, mab110, and mab127 in the present disclosure have very similar light chain CDR regions, with only a few amino acid differences. These light chain CDR regions can be represented by the general formulas: the antibody light chain LCDR1 is as shown in SEQ ID NO: 27: RASQDIGX$_1$X$_2$LN, wherein $X_1$ is selected from D or N, $X_2$ is selected from R or S; LCDR2 is as shown in SEQ ID NO: 28: ATX$_3$X$_4$LDS, where $X_3$ is selected from F or S, $X_4$ is selected from R or S; LCDR3 is as shown in SEQ ID NO: 29: X$_5$QYAX$_6$X$_7$X$_8$WT, wherein $X_5$ is selected from L or Q, $X_6$ is selected from S or G, $X_7$ is selected from F or absence, and $X_8$ is selected from S or P.

**V. Preparation of human IgG1 chimeric antibody**

The above candidate molecules mab108, mab110 and mab127 obtained by hybridoma screening were amplified and sequenced to obtain the sequences encoding the variable regions. Forward and reverse primers were designed on the basis of the sequences obtained by sequencing, the gene to be sequenced was used as a template to construct the VH/VK gene fragment for each antibody via PCR, and then inserted into the expression vector pHr (with a signal peptide and hIgG1/hkappa constant region gene (CH1-Fc/CL) fragment) via homologous recombination to construct an expression plasmid for the full-length of recombinant chimeric antibody VH-CH1-Fc-pHr/VL-CL-pHr, resulting in three chimeric antibodies CH108, CH110 and CH127.

**VI. Humanization of murine anti-human CD73 antibody**

Human germline heavy chain and light chain variable region genes having high homology with murine antibodies were selected as templates by aligning against IMGT (http://imgt.cines.fr) human antibody variable germline gene database using MOE software (Molecular Operating Environment) software. The murine antibody CDRs were grafted into the corresponding human templates to form a variable region sequence in the order of FR1-CDR1-FR2-CDR2-FR3-CDR3-FR4. As needed, the amino acid(s) in the framework sequence was (were) back-mutated to the amino acids corresponding to the murine antibody to obtain the humanized anti-CD73 monoclonal antibody.

**1. Humanization of mab108**

**1.1 Selection of mab108 framework**

For the murine antibody mab108, the light chain templates for humanization were IGKV1-17*01 and hjk4.1, and the heavy chain templates for humanization were IGHV2-26*01 and hjh4.1. Each of the murine antibody mab108 CDRs was grafted onto the human template and Hab108, a humanized antibody of mab108, was obtained, the variable region sequences thereof are as follows:

Hab108 HCVR (also known as VH-CDR graft) amino acid sequence is as shown in SEQ ID NO: 30:

*EVTLKESGPVLVKPTETLTLTCTVSGFSLS*SYGIQ*WIRQPPGKLLEWLA*VIWSGGS
ADYNAAFIS*RLTISKDTSKSQVVLTMTNMDPVDTATYYCAR*QYGSV*WGQGTLVT
VSS*

Hab108 LCVR (also known as VL-CDR graft) amino acid sequence is as shown in SEQ ID NO: 31:

*DIQMTQSPSSLSASVGDRVTITC*RASQDIGDRLN*WYQQKPGKAPKRLIY*ATSSLDS
*GVPSRFSGSGSGTEFTLTISSLQPEDFATYYC*LQYAGSWT*FGGGTKVEIK*

Note: In the above-mentioned Hab108 antibody sequences, the italics represents FR sequence, the underlined portion represents the CDR sequences determined according to Kabat numbering criteria, and the order of sequence is FR1-CDR1-FR2-CDR2-FR3-CDR3-FR4.

**1.2 Back mutation design for humanized antibody of mab 108:**

As needed, the amino acids in the humanized antibody FR region sequence of mab 108 were back-mutated to the amino acids corresponding to the murine antibody. The specifically designed back-mutations are shown in Table 2.

Table 2: Designed back mutation(s) for the humanized antibody of mab108

| VL | | VH | |
|---|---|---|---|
| Hab108.VL1 | Grafted | Hab108.VH1 | Grafted |
| Hab108.VL2 | Y36L, P44I, F71Y | Hab108.VH2 | R94G |
| Hab108.VL3 | Y36L, K42G, P44I, F71Y | Hab108.VH3 | I37V, A49G, R94G |
| Hab108.VL4 | Y36L, K42G, P44I, T69S, F71Y, T85D | Hab108.VH4 | E6Q, V10G, I37V, L44G, A49G, R94G |
| Hab108.VL5 | Y36L, P44I, S60K, F71Y | Hab108.VH5 | S30K, I37V, A49G, R94G |
| Hab108.VL6 | Y36L, K42G, P44I, S60K, F71Y | | |
| Note: "Grafted" represents the implantation of murine antibody CDRs onto human germline FR region sequences; Y36L represents that Y on position 36 of the "Grafted" was back-mutated to L; others can be interpreted in a similar way. | | | |

**1.3 Humanized antibodies of mAb 108:**

As shown in Table 2, variable regions were obtained after back-mutation of the humanized antibodies of mab108. A variety of humanized antibodies of mab 108 were finally obtained after the combination of the variable regions. The variable region amino acid sequences of each antibody are as follows:

Table 3: The amino acid sequences corresponding to the variable regions of humanized antibody of mab108

|            | Hab108.V H1 | Hab108.VH 2 | Hab108.VH3 | Hab108.VH4 | Hab108.VH5 |
|------------|-------------|-------------|-------------|-------------|-------------|
| Hab108.V L1 | Hab108.11 | Hab 108.12 | Hab 108.13 | Hab108.14 | Hab108.15 |
| Hab108.V L2 | Hab 108.21 | Hab108.22 | Hab108.23 | Hab 108.24 | Hab 108.25 |
| Hab108.V L3 | Hab108.31 | Hab108.32 | Hab108.33 | Hab108.34 | Hab108.35 |
| Hab108.V L4 | Hab 108.41 | Hab108.42 | Hab108.43 | Hab 108.44 | Hab 108.45 |
| Hab108.V L5 | Hab108.51 | Hab108.52 | Hab108.53 | Hab 108.54 | Hab108.55 |
| Hab108.V L6 | Hab108.61 | Hab 108.62 | Hab 108.63 | Hab108.64 | Hab108.65 |

Note: Hab108.64 represents a humanized antibody of mab108, and Hab108.64 has a light chain variable region as shown in Hab108.VL6 and a heavy chain variable region as shown in Hab108.VH4; others can be interpreted in a similar way.

The specific variable region sequences of the humanized antibodies of mAb108 are as follows:

Hab108.VL1 amino acid sequence is as shown in SEQ ID NO: 31 (the same as that of Hab 108 LCVR),

*DIQMTQSPSSLSASVGDRVTITC*RASQDIGDRLN*WYQQKPGKAPKRLIY*ATSSLDS*GVPSRFSGSGSGTEFTLTISSLQPEDFATYYC*LQYAGSWT*FGGGTKVEIK*

Hab108.VL2 amino acid sequence is shown in SEQ ID NO: 32:

*DIQMTQSPSSLSASVGDRVTITC*RASQDIGDRLN*WLQQKPGKAIKRLIY*ATSSLDS*GVPSRFSGSGSGTEYTLTISSLQPEDFATYYC*LQYAGSWT*FGGGTKVEIK*

Hab108.VL3 amino acid sequence is as shown in SEQ ID NO: 33:

*DIQMTQSPSSLSASVGDRVTITC*RASQDIGDRLN*WLQQKPGGAIKRLIY*ATSSLDS*GVPSRFSGSGSGTEYTLTISSLQPEDFATYYC*LQYAGSWT*FGGGTKVEIK*

Hab108.VL4 amino acid sequence is as shown in SEQ ID NO: 34:

*DIQMTQSPSSLSASVGDRVTITC*RASQDIGDRLN*WLQQKPGGAIKRLIY*ATSSLDS*GVPSRFSGSGSGSEYTLTISSLQPEDFADYYC*LQYAGSWT*FGGGTKVEIK*

Hab108.VL5 amino acid sequence is as shown in SEQ ID NO: 35:

*DIQMTQSPSSLSASVGDRVTITC*RASQDIGDRLN*WLQQKPGKAIKRLIY*ATSSLDS*GVPKRFSGSGSGTEYTLTISSLQPEDFATYYC*LQYAGSWT*FGGGTKVEIK*

Hab108.VL6 amino acid sequence is as shown in SEQ ID NO: 36:

*DIQMTQSPSSLSASVGDRVTITC<u>RASQDIGDRLN</u>WLQQKPGGAIKRLIY<u>ATSSLDS</u> GVPKRFSGSGSGTEYTLTISSLQPEDFATYYC<u>LQYAGSWT</u>FGGGTKVEIK*

Hab108.VH1 amino acid sequence is as shown in SEQ ID NO: 30 (the same as that of Hab108 HCVR),

*EVTLKESGPVLVKPTETLTLTCTVSGFSLS<u>SYGIQ</u>WIRQPPGKLLEWLA<u>VIWSGGS ADYNAAFIS</u>RLTISKDTSKSQVVLTMTNMDPVDTATYYCAR<u>QYGSV</u>WGQGTLVT VSS*

Hab108.VH2 amino acid sequence is as shown in SEQ ID NO: 37:

*EVTLKESGPVLVKPTETLTLTCTVSGFSLS<u>SYGIQ</u>WIRQPPGKLLEWLA<u>VIWSGGS ADYNAAFIS</u>RLTISKDTSKSQVVLTMTNMDPVDTATYYCAR<u>QYGSV</u>WGQGTLVT VSS*

Hab108.VH3 amino acid sequence is as shown in SEQ ID NO: 38:

*EVTLKESGPVLVKPTETLTLTCTVSGFSLS<u>SYGIQ</u>WVRQPPGKLLEWLG<u>VIWSGGS ADYNAAFIS</u>RLTISKDTSKSQVVLTMTNMDPVDTATYYCAG<u>QYGSV</u>WGQGTLVT VSS*

Hab108.VH4 amino acid sequence is as shown in SEQ ID NO: 39:

*EVTLKQSGPGLVKPTETLTLTCTVSGFSLS<u>SYGIQ</u>WVRQPPGKGLEWLG<u>VIWSGG SADYNAAFIS</u>RLTISKDTSKSQVVLTMTNMDPVDTATYYCAG<u>QYGSV</u>WGQGTLV TVSS*

Hab108.VH5 amino acid sequence is as shown in SEQ ID NO: 40:

*EVTLKESGPVLVKPTETLTLTCTVSGFSLK<u>SYGIQ</u>WVRQPPGKLLEWLG<u>VIWSGG SADYNAAFIS</u>RLTISKDTSKSQVVLTMTNMDPVDTATYYCAG<u>QYGSV</u>WGQGTLV TVSS.*

## 2. Humanization of mab110

### 2.1 Selection of mab110 framework

For the murine antibody mab110, the light chain templates for humanization were IGKV1-17*01 and hjk4.1, and the heavy chain templates for humanization were IGHV1-3*01 and hjh6.1. Each of the murine antibody mab110 CDRs was grafted onto the human template and Hab110, a humanized antibody of mab110, was obtained, the variable region sequences thereof are as follows:

Hab110 HCVR (also known as VH-CDR graft) amino acid sequence is as shown in SEQ ID NO: 41:

*EVQLVQSGAEVKKPGASVKVSCKASGYTFT<u>SYTMH</u>WVRQAPGQRLEWMG<u>YINP NSFYIEYNQKFKD</u>RVTITRDTSASTAYMELSSLRSEDTAVYYCAR<u>GDYDLFYDMD N</u>WGQGTTVTVSS*

Hab110 LCVR (also known as VL-CDR graft) amino acid sequence is as shown in SEQ ID NO: 42:

*DIQMTQSPSSLSASVGDRVTITC*<u>RASQDIGNSLN</u>*WYQQKPGKAPKRLIY*<u>ATFRLDS</u> *GVPSRFSGSGSGTEFTLTISSLQPEDFATYYC*<u>QQYASFPWT</u>*FGGGTKVEIK*

Note: In the above-mentioned Hab110 antibody sequences, the italics represents the FR sequence, the underlined portion represents the CDR sequences, and the order of sequence is FR1-CDR1-FR2-CDR2-FR3-CDR3-FR4.

**2.2 Back mutation design for mab 110:**

As needed, the amino acids in the humanized antibody FR region sequence of mab 110 were back-mutated to the amino acids corresponding to the murine antibody. The specifically designed back-mutations are shown in Table 4.

Table 4: Designed back mutation(s) for the humanized antibody of mab110

| VL | | VH | |
|---|---|---|---|
| Hab100.VL1 | Grafted | Hab110.VH1 | Grafted |
| Hab110.VL2 | Y36L, P44I, G66R, F71Y | Hab110.VH2 | R71A |
| Hab110.VL3 | Y36L, K42G, P44I, G66R, F71Y, T85D | Hab110.VH3 | M48I, V67A, I69L, R71A |
| | | Hab110.VH4 | M48I, R66K, V67A, I69L, R71A |
| | | Hab110.VH5 | R38K, R44G, M48I, R66K, V67A, I69L, R71A |
| Note: "Grafted" represents the implantation of murine antibody CDRs onto human germline FR region sequences; Y36L represents Y on position 36 of the "Grafted" was back-mutated to L; others can be interpreted in a similar way. | | | |

**2.3 Humanized antibodies of mAb 110** :

The designed back-mutations for humanized antibodies of mab110 as indicated in Table 4 were combined, and then further recombined with human heavy and light constant regions. A variety of humanized antibodies of mab 110 were finally obtained and the variable region amino acid sequences thereof are shown as follows:

Table 5: The amino acid sequences corresponding to the variable regions of humanized antibody of mab110

| | Hab110.VH1 | Hab110.VH2 | Hab110.VH3 | Hab110.VH4 | Hab110.VH5 |
|---|---|---|---|---|---|
| Hab110.VL1 | Hab110.11 | Hab110.12 | Hab 110.13 | Hab 1 10.14 | Hab110.15 |
| Hab110.VL2 | Hab110.21 | Hab110.22 | Hab110.23 | Hab110.24 | Hab110.25 |
| Hab110.VL3 | Hab110.31 | Hab110.32 | Hab110.33 | Hab110.34 | Hab110.35 |
| Note: Hab110.21 represents a humanized antibody of mab110, and Hab110.21 has a light chain variable region as shown in Hab110.VL2 and a heavy chain variable region as shown in Hab110.VH1; others can be interpreted in a similar way. | | | | | |

The specific variable region sequences of the humanized antibodies of mAb1110 are as follows:

Hab110.VL1 amino acid sequence is as shown in SEQ ID NO: 42 (the same as that of Hab110 LCVR):

*DIQMTQSPSSLSASVGDRVTITC*<u>RASQDIGNSLN</u>*WYQQKPGKAPKRLIY*<u>ATFRLDS</u> *GVPSRFSGSGSGTEFTLTISSLQPEDFATYYC*<u>QQYASFPWT</u>*FGGGTKVEIK*

Hab110.VL2 amino acid sequence is as shown in SEQ ID NO: 43:

*DIQMTQSPSSLSASVGDRVTITC*<u>RASQDIGNSLN</u>*WLQQKPGKAIKRLIY*<u>ATFRLDS</u>
*GVPSRFSGSRSGTEYTLTISSLQPEDFATYYC*<u>QQYASFPWT</u>*FGGGTKVEIK*

Hab110.VL3 amino acid sequence is as shown in SEQ ID NO: 44:

*DIQMTQSPSSLSASVGDRVTITC*<u>RASQDIGNSLN</u>*WLQQKPGGAIKRLIY*<u>ATFRLDS</u>
*GVPSRFSGSRSGTEYTLTISSLQPEDFADYYC*<u>QQYASFPWT</u>*FGGGTKVEIK*

Hab110.VH1 amino acid sequence is as shown in SEQ ID NO: 41 (the same as that of Hab110 HCVR):

*EVQLVQSGAEVKKPGASVKVSCKASGYTFT*<u>SYTMH</u>*WVRQAPGQRLEWMG*<u>YINP</u>
<u>NSFYIEYNQKFKD</u>*RVTITRDTSASTAYMELSSLRSEDTAVYYCAR*<u>GDYDLFYDMD</u>
<u>N</u>*WGQGTTVTVSS*

Hab110.VH2 amino acid sequence is as shown in SEQ ID NO: 45:

*EVQLVQSGAEVKKPGASVKVSCKASGYTFT*<u>SYTMH</u>*WVRQAPGQRLEWMG*<u>YINP</u>
<u>NSFYIEYNQKFKD</u>*RVTITADTSASTAYMELSSLRSEDTAVYYCAR*<u>GDYDLFYDMD</u>
<u>N</u>*WGQGTTVTVSS*

Hab110.VH3 amino acid sequence is as shown in SEQ ID NO: 46:

*EVQLVQSGAEVKKPGASVKVSCKASGYTFT*<u>SYTMH</u>*WVRQAPGQRLEWIG*<u>YINPN</u>
<u>SFYIEYNQKFKD</u>*RATLTADTSASTAYMELSSLRSEDTAVYYCAR*<u>GDYDLFYDMDN</u>
*WGQGTTVTVSS*

Hab110.VH4 amino acid sequence is as shown in SEQ ID NO: 47:

*EVQLVQSGAEVKKPGASVKVSCKASGYTFT*<u>SYTMH</u>*WVRQAPGQRLEWIG*<u>YINPN</u>
<u>SFYIEYNQKFKD</u>*KATLTADTSASTAYMELSSLRSEDTAVYYCAR*<u>GDYDLFYDMDN</u>
*WGQGTTVTVSS*

Hab110.VH5 amino acid sequence is as shown in SEQ ID NO: 48:

*EVQLVQSGAEVKKPGASVKVSCKASGYTFT*<u>SYTMH</u>*WVKQAPGQGLEWIG*<u>YINPN</u>
<u>SFYIEYNQKFKD</u>*KATLTADTSASTAYMELSSLRSEDTAVYYCAR*<u>GDYDLFYDMDN</u>
*WGQGTTVTVSS.*

## 3. Humanization of mab127

### 3.1 Selection of mab127 framework

For the murine antibody mab127, the light chain templates for humanization were IGKV1-17*01 and hjk4.1, and the heavy chain templates for humanization were IGHV1-46*01 and hjh6.1. Each of the murine antibody mab127 CDRs was grafted onto the human template and Hab127, a humanized antibody of mab127, was obtained, the variable region sequences thereof are as follows:

Hab127 HCVR (also known as VH-CDR graft) amino acid sequence is as shown in SEQ ID NO: 49:

*EVQLVQSGAEVKKPGASVKVSCKASGYTFT*<u>AYFMN</u>*WVRQAPGQGLEWMG*<u>RIYH</u>
<u>NNGDTFYSQKFKG</u>*RVTMTRDTSTSTVYMELSSLRSEDTAVYYCAR*<u>SYVGD</u>*WGQG*
*TTVTVSS*

Hab127 LCVR (also known as VL-CDR graft) amino acid sequence is as shown in SEQ ID NO: 50:

*DIQMTQSPSSLSASVGDRVTITC*<u>RASQDIGDSLN</u>*WYQQKPGKAPKRLIY*<u>ATSSLDS</u>
*GVPSRFSGSGSGTEFTLTISSLQPEDFATYYC*<u>LQYASFPWT</u>*FGGGTKVEIK*

Note: In the above-mentioned Hab127 antibody sequences, the italics represents FR sequence, the underlined portion represents the CDR sequences (determined and annotated according to Kabat numbering criteria), and the sequence order is FR1-CDR1-FR2-CDR2-FR3-CDR3-FR4.

### 3.2 Back mutation design for mab 127:

As needed, the amino acids in the humanized antibody FR region sequence of mab 127 were back-mutated to the amino acids corresponding to the murine antibody. The specifically designed back-mutations are shown in Table 6.

Table 6: Designed back mutation(s) for the humanized antibody of mab 127

| VL | | VH | |
|---|---|---|---|
| Hab127.VL1 | Grafted | Hab127.VH0 | Grafted |
| Hab127.VL2 | Y36L, F71Y | Hab127.VH1 | R71V, T73K, R94T |
| Hab127.VL3 | Y36L, K42G, P44L, F71Y | Hab127.VH2 | R38M, R71V, T73K, R94T |
| Hab127.VL4 | Y36L, K42G, P44L, T69S, E70D, F71Y | Hab127.VH3 | R38M, M48I, V67A, M69L, R71V, T73K, R94T |
| | | Hab127.VH4 | R38M, G44S, M48I, V67A, M69L, R71V, T73K, S82A R, R94T |
| Note: "Grafted" represents the implantation of murine antibody CDRs onto human germline FR region sequences; Y36L represents Y on position 36 of the "Grafted" was back-mutated to L; others can be interpreted in a similar way. | | | |

### 3.3 Humanized antibodies of mAb 127 :

The designed back-mutations for humanized antibodies of mab127 as indicated in Table 6 were combined and then further recombined with human heavy and light constant regions. A variety of humanized antibodies of mab 127 were finally obtained and the variable region amino acid sequences thereof are shown as follows:

Table 7: The amino acid sequences corresponding to the variable regions of humanized antibody of mab127

| | Hab127.VH1 | Hab127.VH2 | Hab127.VH3 | Hab127.VH4 |
|---|---|---|---|---|
| Hab127.VL1 | Hab 127.11 | Hab 127.12 | Hab127.13 | Hab 127.14 |
| Hab127.VL2 | Hab 127.21 | Hab 127.22 | Hab 127.23 | Hab 127.24 |
| Hab l27.VL3 | Hab 127.31 | Hab 127.32 | Hab 127.33 | Hab 127.34 |
| Hab127.VL4 | Hab 127.41 | Hab 127.42 | Hab 127.43 | Hab 127.44 |
| Note: Hab 127.11 represents a humanized antibody of mab 127, and Hab 127.11 has a light chain variable region as shown in Hab127.VL1 and a heavy chain variable region as shown in Hab110.VH1; others can be interpreted in a similar way. | | | | |

The specific variable region sequences of the humanized antibodies of mab127 are as follows:

Hab127.VL1 amino acid sequence is as shown in SEQ ID NO: 50 (the same as that of Hab 127 LCVR),

*DIQMTQSPSSLSASVGDRVTITC<u>RASQDIGDSLN</u>WYQQKPGKAPKRLIY<u>ATSSLDS</u>*
*GVPSRFSGSGSGTEFTLTISSLQPEDFATYYC<u>LQYASFPWT</u>FGGGTKVEIK*

Hab127.VL2 amino acid sequence is as shown in SEQ ID NO: 51:

*DIQMTQSPSSLSASVGDRVTITC<u>RASQDIGDSLN</u>WLQQKPGKAPKRLIY<u>ATSSLDS</u>*
*GVPSRFSGSGSGTEYTLTISSLQPEDFATYYCLQYASFPWTFGGGTKVEIK*

Hab127.VL3 amino acid sequence is as shown in SEQ ID NO: 52:

*DIQMTQSPSSLSASVGDRVTITC<u>RASQDIGDSLN</u>WLQQKPGGALKRLIY<u>ATSSLDS</u>*
*GVPSRFSGSGSGTEYTLTISSLQPEDFATYYCLQYASFPWTFGGGTKVEIK*

Hab127.VL4 amino acid sequence is as shown in SEQ ID NO: 53:

*DIQMTQSPSSLSASVGDRVTITC<u>RASQDIGDSLN</u>WLQQKPGGALKRLIY<u>ATSSLDS</u>*
*GVPSRFSGSGSGSDYTLTISSLQPEDFATYYCLQYASFPWTFGGGTKVEIK*

Hab127.VH1 amino acid sequence is as shown in SEQ ID NO: 54:

*EVQLVQSGAEVKKPGASVKVSCKASGYTFT<u>AYFMN</u>WVRQAPGQGLEWMG<u>RIYH</u>*
*<u>NNGDTFYSQKFKG</u>RVTMTVDKSTSTVYMELSSLRSEDTAVYYCAT<u>SYVGD</u>WGQG*
*TTVTVSS*

Hab127.VH2 amino acid sequence is as shown in SEQ ID NO: 55:

*EVQLVQSGAEVKKPGASVKVSCKASGYTFT<u>AYFMN</u>WVMQAPGQGLEWMG<u>RIYH</u>*
*<u>NNGDTFYSQKFKG</u>RVTMTVDKSTSTVYMELSSLRSEDTAVYYCAT<u>SYVGD</u>WGQG*
*TTVTVSS*

Hab127.VH3 amino acid sequence is shown in SEQ ID NO: 56:

*EVQLVQSGAEVKKPGASVKVSCKASGYTFT<u>AYFMN</u>WVMQAPGQGLEWIG<u>RIYH</u>*
*<u>NNGDTFYSQKFKG</u>RATLTVDKSTSTVYMELSSLRSEDTAVYYCAT<u>SYVGD</u>WGQG*
*TTVTVSS*

Hab127.VH4 amino acid sequence is as shown in SEQ ID NO: 57:

*EVQLVQSGAEVKKPGASVKVSCKASGYTFT<u>AYFMN</u>WVMQAPGQSLEWIG<u>RIYHN</u>*
*<u>NGDTFYSQKFKG</u>RATLTVDKSTSTVYMELRSLRSEDTAVYYCAT<u>SYVGD</u>WGQGT*
*TVTVSS.*

**3.4 Hot spot mutations of humanized antibody of mab127:**

Amino acid mutations were performed on NNG based on the antibody structure of the mab127 heavy chain HCDR2 by a computer simulation method. In one of the preferred embodiments, we performed amino acid mutations on the NNG of Hab127.VH1 HCDR2. The sequences of Hab127.VH1 after mutation are as follows:

Hab127.VH1a amino acid sequence is shown in SEQ ID NO: 58:

EVQLVQSGAEVKKPGASVKVSCKASGYTFTAYFMNWV**R**QAPGQ**G**LEW**M**G
RIYH<u>NQG</u>DTFYSQKFKGR**VTM**TVDKSTSTVYMEL**S**SLRSEDTAVYYCATSY
VGDWGQGTTVTVSS

Hab127.VH1b amino acid sequence is shown in SEQ ID NO: 59:

EVQLVQSGAEVKKPGASVKVSCKASGYTFTAYFMNWV**R**QAPGQ**G**LEW**M**G
RIYH<u>NLG</u>DTFYSQKFKGR**VTM**TVDKSTSTVYMEL**S**SLRSEDTAVYYCATSYV
GDWGQGTTVTVSS

Hab127.VH1c amino acid sequence is shown in SEQ ID NO: 60:

EVQLVQSGAEVKKPGASVKVSCKASGYTFTAYFMNWV**R**QAPGQ**G**LEW**M**G
RIYH<u>NTG</u>DTFYSQKFKGR**VTM**TVDKSTSTVYMEL**S**SLRSEDTAVYYCATSYV
GDWGQGTTVTVSS

Hab127.VH1d amino acid sequence is shown in SEQ ID NO: 61:

EVQLVQSGAEVKKPGASVKVSCKASGYTFTAYFMNWV**R**QAPGQ**G**LEW**M**G

RIYH<u>NKG</u>DTFYSQKFKGR**VTM**TVDKSTSTVYMEL**S**SLRSEDTAVYYCATSY
VGDWGQGTTVTVSS

Hab127.VH1e amino acid sequence is shown in SEQ ID NO: 62:

EVQLVQSGAEVKKPGASVKVSCKASGYTFTAYFMNWV**R**QAPGQ**G**LEW**M**G
RIYH<u>NEG</u>DTFYSQKFKGR**VTM**TVDKSTSTVYMEL**S**SLRSEDTAVYYCATSYV
GDWGQGTTVTVSS

Hab127.VH1f amino acid sequence is shown in SEQ ID NO: 63:

EVQLVQSGAEVKKPGASVKVSCKASGYTFTAYFMNWV**R**QAPGQ**G**LEW**M**G
RIYH<u>NHG</u>DTFYSQKFKGR**VTM**TVDKSTSTVYMEL**S**SLRSEDTAVYYCATSY
VGDWGQGTTVTVSS

Hab127.VH1g amino acid sequence is shown in SEQ ID NO: 64:

EVQLVQSGAEVKKPGASVKVSCKASGYTFTAYFMNWV**R**QAPGQ**G**LEW**M**G
RIYH<u>QNV</u>DTFYSQKFKGR**VTM**TVDKSTSTVYMEL**S**SLRSEDTAVYYCATSY
VGDWGQGTTVTVSS

Hab127.VH1h amino acid sequence is shown in SEQ ID NO: 65:

EVQLVQSGAEVKKPGASVKVSCKASGYTFTAYFMNWV**R**QAPGQ**G**LEW**M**G
RIYH<u>LNV</u>DTFYSQKFKGR**VTM**TVDKSTSTVYMEL**S**SLRSEDTAVYYCATSYV
GDWGQGTTVTVSS

Habl27.VHli amino acid sequence is shown in SEQ ID NO: 66:

**EVQLVQSGAEVKKPGASVKVSCKASGYTFTAYFMNWVRQAPGQGLEWMG RIYH<u>HNV</u>DTFYSQKFKGRVTMTVDKSTSTVYMELSSLRSEDTAVYYCATSY VGDWGQGTTVTVSS.**

The general formula of the humanized antibody HCDR2 amino acid sequence of mab127 after hot spot mutation is as shown in SEQ ID NO: 67: RIYH X9 X10X11DTFYSQKFKG, wherein X9 is selected from N, Q, L or H, and X10 is selected from N, Q, L, T, K, E or H, X11 is selected from V or G; preferably, the humanized antibody HCDR2 amino acid sequence of mab127 after hot spot mutation is as shown in SEQ ID NOs: 68-76:

SEQ ID NO: 68: RIYHNQGDTFYSQKFKG;
SEQ ID NO: 69: RIYHNLGDTFYSQKFKG;
SEQ ID NO: 70: RIYHNTGDTFYSQKFKG;
SEQ ID NO: 71: RIYHNKGDTFYSQKFKG;
SEQ ID NO: 72: RIYHNEGDTFYSQKFKG;
SEQ ID NO: 73: RIYHNHGDTFYSQKFKG;
SEQ ID NO: 74: RIYHQNVDTFYSQKFKG;
SEQ ID NO: 75: RIYHLNVDTFYSQKFKG;
SEQ ID NO: 76: RIYHHNVDTFYSQKFKG.

Table 8: Humanized antibody variable regions of mab127 corresponding to hot-spot mutations

|  | Hab127.VL1 | Hab127.VL2 | Hab127.VL3 | Hab127.VL4 |
|---|---|---|---|---|
| Hab127.VH1a | Hab127.11a | Hab127.21a | Hab 127.31a | Hab 127.41a |
| Hab127.VH1b | Hab127.11b | Hab127.21b | Hab 127.3 1b | Hab 127.41b |
| Hab127.VH1c | Hab127.11c | Hab127.21c | Hab127.31c | Hab127.41c |
| Hab127.VH1d | Hab127.11d | Hab127.21d | Hab127.31d | Hab127.41d |
| Hab127.VH1e | Hab127.11e | Hab127.21e | Hab127.31e | Hab127.41e |
| Hab127.VH1f | Hab127.11f | Hab127.21f | Hab127.31f | Hab127.41f |
| Hab127.VH1g | Habl27.11g | Hab127.21g | Hab127.31g | Hab127.41g |
| Hab127.VH1h | Hab127.11h | Hab127.21h | Hab127.3 1h | Hab127.41h |
| Hab127.VH1i | Hab127.11i | Hab127.21i | Hab127.3 1i | Hab 127.41i |
| Note: Hab127.31d represents a humanized antibody of mab127, and Hab127.31d has a light chain variable region as shown in Hab127.VL3 and a heavy chain variable region as shown in Hab127.VH1d; others can be interpreted in a similar way. | | | | |

## 4. Construction and expression of anti-human CD73 humanized antibody

Primers were designed, VH/VK gene fragment of each humanized antibody was constructed by PCR and then inserted into the expression vector pHr (with a signal peptide and constant region gene (CH1-FC/CL) fragment) via homologous recombination to construct an expression vector for a full-length antibody VH-CH1-FC-pHr/VK-CL-pHr. For the humanized antibody, the constant region can be selected from the light chain constant region of human κ, λ chain or variant(s) thereof, and can be selected from the heavy chain constant region of IgG1, IgG2, IgG3 or IgG4 or variant(s) thereof. Non-limiting examples include optimizing the constant region of human IgG1, IgG2 or IgG4 to improve antibody function. For example, the ADCC of the antibody can be reduced by point mutations in the constant region such as D265A, N297A, L234A/L235A or L234F/L235E, and the CDC of the antibody can be reduced by P331S or the mutation(s) nearby, and mutants such as S228P, F234A and L235A in constant region.

An exemplary anti-human CD73 humanized antibody constant region sequence is as follows:

The amino acid sequence of the heavy chain constant region is as shown in SEQ ID NO: 77:

ASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKT HTCPPCPAPEFEGGPSVFLFPPKPKDTLMISRTPEVTCVVVDVSHEDPEVKFN WYVDGVEVHNAKTKPREEQYNSTYRVVSVLTVLHQDWLNGKEYKCKVSN KALPASIEKTISKAKGQPREPQVYTLPPSRDELTKNQVSLTCLVKGFYPSDIAV EWESNGQPENNYKTTPPVLDSDGSFFLYSKLTVDKSRWQQGNVFSCSVMHE ALHNHYTQKSLSLSPGK

The amino acid sequence of the light chain constant region of the anti-human CD73 humanized antibody is shown in SEQ ID NO: 78:

RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNR GEC.

An exemplary anti-human CD73 humanized antibody full-length amino acid sequence is as follows:

Hab 108.64 antibody heavy chain amino acid sequence is shown in SEQ ID NO: 79:

EVTLKQSGPGLVKPTETLTLTCTVSGFSLSSYGIQWVRQPPGKGLEWLGVIWS GGSADYNAAFISRLTISKDTSKSQVVLTMTNMDPVDTATYYCAGQYGSVWG QGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKK VEPKSCDKTHTCPPCPAPEFEGGPSVFLFPPKPKDTLMISRTPEVTCVVVDVSH EDPEVKFNWYVDGVEVHNAKTKPREEQYNSTYRVVSVLTVLHQDWLNGKE YKCKVSNKALPASIEKTISKAKGQPREPQVYTLPPSRDELTKNQVSLTCLVKG FYPSDIAVEWESNGQPENNYKTTPPVLDSDGSFFLYSKLTVDKSRWQQGNVF SCSVMHEALHNHYTQKSLSLSPGK

Hab108.64 antibody light chain amino acid sequence is shown in SEQ ID NO: 80:

DIQMTQSPSSLSASVGDRVTITCRASQDIGDRLNWLQQKPGGAIKRLIYATSSL DSGVPKRFSGSGSGTEYTLTISSLQPEDFATYYCLQYAGSWTFGGGTKVEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGE C.

Hab110.21 antibody heavy chain amino acid sequence is shown in SEQ ID NO: 81:

EVQLVQSGAEVKKPGASVKVSCKASGYTFTSYTMHWVRQAPGQRLEWMGY

INPNSFYIEYNQKFKDRVTITRDTSASTAYMELSSLRSEDTAVYYCARGDYDL
FYDMDNWGQGTTVTVSSASTKGPSVFPLAPSSKSTSGGTAALGCLVKDYFPE
PVTVSWNSGALTSGVHTFPAVLQSSGLYSLSSVVTVPSSSLGTQTYICNVNHK
PSNTKVDKKVEPKSCDKTHTCPPCPAPEFEGGPSVFLFPPKPKDTLMISRTPEV
TCVVVDVSHEDPEVKFNWYVDGVEVHNAKTKPREEQYNSTYRVVSVLTVL
HQDWLNGKEYKCKVSNKALPASIEKTISKAKGQPREPQVYTLPPSRDELTKN
QVSLTCLVKGFYPSDIAVEWESNGQPENNYKTTPPVLDSDGSFFLYSKLTVDK
SRWQQGNVFSCSVMHEALHNHYTQKSLSLSPGK

Hab110.21 antibody light chain amino acid sequence is shown in SEQ ID NO: 82:

DIQMTQSPSSLSASVGDRVTITCRASQDIGNSLNWLQQKPGKAIKRLIYATFRL
DSGVPSRFSGSRSGTEYTLTISSLQPEDFATYYCQQYASFPWTFGGGTKVEIKR
TVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C.

Hab127. 31d antibody heavy chain amino acid sequence is shown in SEQ ID NO: 83:

EVQLVQSGAEVKKPGASVKVSCKASGYTFTAYFMNWVRQAPGQGLEWMG
RIYHNKGDTFYSQKFKGRVTMTVDKSTSTVYMELSSLRSEDTAVYYCATSYV
GDWGQGTTVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTK
VDKKVEPKSCDKTHTCPPCPAPEFEGGPSVFLFPPKPKDTLMISRTPEVTCVV
VDVSHEDPEVKFNWYVDGVEVHNAKTKPREEQYNSTYRVVSVLTVLHQDW
LNGKEYKCKVSNKALPASIEKTISKAKGQPREPQVYTLPPSRDELTKNQVSLT
CLVKGFYPSDIAVEWESNGQPENNYKTTPPVLDSDGSFFLYSKLTVDKSRWQ
QGNVFSCSVMHEALHNHYTQKSLSLSPGK

Hab127.31d antibody light chain amino acid sequence is shown in SEQ ID NO: 84:

DIQMTQSPSSLSASVGDRVTITCRASQDIGDSLNWLQQKPGGALKRLIYATSS
LDSGVPSRFSGSGSGTEYTLTISSLQPEDFATYYCLQYASFPWTFGGGTKVEIK
RTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNR
GEC.

**Example 3. Detection of binding activity of anti-CD73 antibody**

1. ELISA assay of the binding of anti-CD73 antibodies to human CD73 protein;

The binding of anti-CD73 antibodies to human CD73 protein was tested by ELISA assay. The biotinylated CD73 fusion protein was immobilized onto a 96-well microtiter plate by binding to the Streptavidin coated on the microtiter plate. The signal strength after the addition of the antibody was used to determine the binding activity of the antibody to CD73. The specific experimental procedures were as follows:

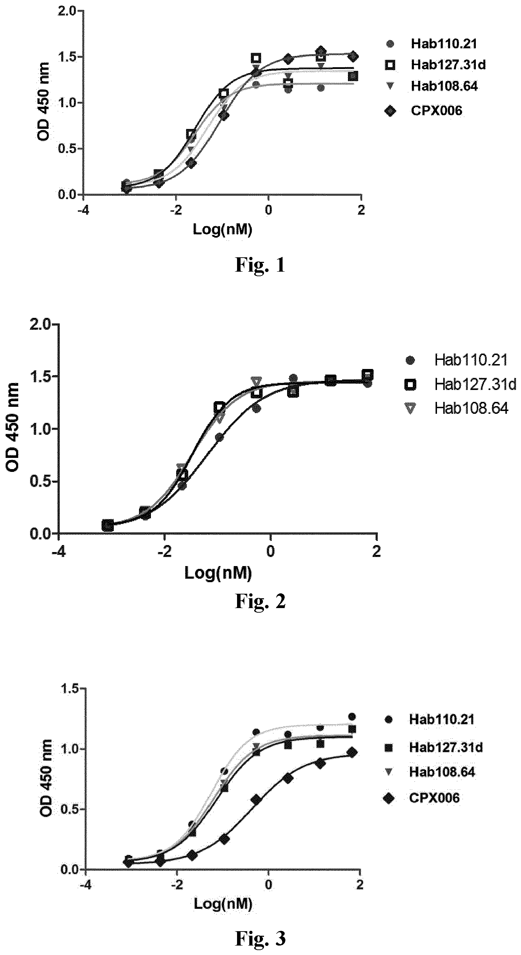

Streptavidin (Sigma, Cat No. S4762-5MG) was diluted with PBS, pH7.4 (Shanghai BasalMedia, Cat No. B320) buffer to a concentration of 3 $\mu$g/ml, was added into the 96-well microtiter plate (Corning, Cat No. CLS3590-100EA), 50$\mu$l/well, and incubated in an incubator at 37°C for 2 hours. The liquid was discarded, 250$\mu$l/well blocking solution (5% skim milk (BD skim milk, Cat No.232100) diluted in PBS) was added, and incubated in an incubator at 37°C for 3 hours or at 4°C overnight (16-18 hours) for blocking. After blocking, the blocking solution was discarded; the plate was washed for 5 times with PBST buffer (PBS containing 0.05% tween-20, pH7.4), 50μl/well of 0.5μg/ml biotinylated CD73-myc-his fusion protein (Tojin Chemical, Cat No. LK03, biotinylation method was according to the kit's instruction) diluted with sample diluent (PBS containing 1% BSA, pH7.4) was added, and incubated in an incubator at 37°C 1 hour or at 4°C overnight. After the incubation was finished, the reaction solution was removed from the microtiter plate, the plate was washed for 5 times with PBST, 50μl/well of various concentrations of each of the test antibodies (purified hybridoma antibodies or humanized antibodies, diluted with sample diluent) were added, and incubated in an incubator at 37°C for 1 hour. After the incubation was finished, the plate was washed for 5 times with PBST, 50μl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample diluent was added, and incubated at 37°C for 1 hour. The plate was washed for 5 times with PBST, 50μl/well of TMB chromogenic substrate (KPL, Cat No. 52-00-03) was added, incubated at room temperature for 5-10min, and 50μl/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of anti-CD73 antibody to human CD73 protein was calculated as EC50 value. The experimental results are shown in Figure 1 and Table 9.

Figure 9: ELISA results of the binding of anti-CD73 antibodies to human CD73 protein

| Test samples | EC50 (nM) in ELISA assay showing the binding to human CD73 protein |
| --- | --- |
| Hab110.21 | 0.026 |
| Hab127.31d | 0.026 |
| Hab108.64 | 0.047 |
| CPX006 | 0.088 |

The experimental results show that the anti-CD73 antibodies Hab110.21, Hab127.31d and Hab 108.64 of the present disclosure all have favorable binding activity to human CD73 protein, and the EC50 values of the three anti-CD73 antibodies to human CD73 protein are all lower than that of the positive control antibody CPX006 (antibody CPX-006 disclosed in patent application WO2018013611).

**2. ELISA assay showing the binding of anti-CD73 antibodies to cynomolgus monkey CD73 protein**

The cross-binding activity of anti-CD73 antibodies to cynomolgus monkey CD73 protein was tested by ELISA assay. The cynomolgus monkey CD73 fusion protein was directly coated onto a 96-well microtiter plate. The signal strength after the addition of the antibody was used to determine the binding activity of the antibody to cynomolgus monkey CD73. The specific experimental procedures were as follows:
Cynomolgus monkey CD73 (Sino Biological, Cat No. 90192-C08H) was diluted with PBS, pH7.4 (Shanghai BasalMedia, Cat No. B320) buffer to a concentration of 2 μg/ml, was added into the 96-well microtiter plate at 50μl/well, and was incubated in an incubator at 37°C for 2 hours. The liquid was discarded, 250μl/well blocking solution (5% skim milk diluted in PBS) was added, and incubated in an incubator at 37°C for 3 hours or at 4°C overnight (16-18 hours) for blocking. After the blocking was finished, the blocking solution was discarded, the plate was washed for 5 times with PBST buffer, 50μl/well of various concentrations of each of the test antibodies (purified hybridoma antibodies or humanized antibodies, diluted with sample diluent) were added, and incubated in an incubator at 37°C for 1 hour. After the incubation was finished, the plate was washed for 5 times with PBST, 50μl/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample diluent was added, and incubated at 37°C for 1 hour. The plate was washed for 5 times with PBST, 50μl/well of TMB chromogenic substrate (KPL, Cat No. 52-00-03) was added, incubated at room temperature for 5-10min, and 50μl/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of anti-CD73 antibody to monkey CD73 was calculated as EC50 value. The experimental results are shown in Figure 2 and Table 10.

Figure 10: ELISA results of the binding of anti-CD73 antibodies to cynomolgus monkey CD73 protein

| Test sample | EC50 (nM) in ELISA assay showing the binding to cynomolgus monkey CD73 |
|---|---|
| Hab 110.21 | 0.064 |
| Hab127.31d | 0.033 |
| Hab108.64 | 0.033 |

The experimental results show that the anti-CD73 antibodies Hab110.21, Hab11d, and Hab108.64 of the present disclosure all have favorable binding effects with monkey CD73 protein.

### 3. Binding assay of anti-CD73 antibodies to MDA-MB-231 cells

The binding of anti-CD73 antibodies to tumor cell line MDA-MB-231 that highly expresses human CD73 was tested by ELISA assay. The MDA-MB-231 cells were directly coated onto a 96-well microtiter plate. The signal strength after the addition of the antibody was used to determine the binding activity of the antibody to MDA-MB-231 cells. The specific experimental procedures were as follows:

MDA-MB-231 cells (ATCC, Cat No HTB-26) were seeded in the 96-well plate (Corning, Cat No. CLS3599-100EA) at a density of $6 \times 10^5$/ml, 100 $\mu$l/well, and cultured overnight. The supernatant was discarded, the plate was washed with PBS for three times, 100$\mu$l/well of cell immune fixed solution (Beyotime, Cat No. P0098) was added and fixed for half an hour at room temperature, and the plate washed with PBS for four times. The liquid was discarded, 250$\mu$l/well blocking solution (5% skim milk diluted in PBS) was added, and incubated in an incubator at 37°C for 3 hours. After the blocking was finished, the blocking solution was discarded, the plate was washed for 5 times with PBST buffer, 50%l/well of various concentrations of each of the test antibodies were added, and incubated in an incubator at 37°C for 1 hour. After the incubation was finished, the plate was washed for 5 times with PBST, 50$\mu$l/well of HRP-labeled goat anti-mouse secondary antibody (Jackson Immuno Research, Cat No. 115-035-003) or goat anti-human secondary antibody (Jackson Immuno Research, Cat No. 109-035-003) diluted with sample diluent was added, and incubated at 37°C for 1 hour. The plate was washed for 5 times with PBST, 50$\mu$l/well of TMB chromogenic substrate (KPL, Cat No. 52-00-03) was added, incubated at room temperature for 5-15min, and 50$\mu$l/well of 1M $H_2SO_4$ was added to stop the reaction. The absorbance value was read by a microplate reader (Molecular Devices, VERSA max) at a wavelength of 450 nm, and the data was analyzed with GraphPad Prism 5. The binding of anti-CD73 antibody to MDA-MB-231 cells over-expressing CD73 was calculated as EC50 value. The experimental results are shown in Figure 3 and Table 11.

Table 11: Results of binding assay of anti-CD73 antibodies to MDA-MB-231 cells

| Test sample | EC50 (nM) in ELISA assay showing the binding to MDA-MB-231 cell |
|---|---|
| Hab110.21 | 0.056 |
| Hab127.31d | 0.072 |
| Hab108.64 | 0.062 |
| CPX006 | 0.433 |

The experimental results show that the anti-CD73 antibodies Hab110.21, Hab11d, and Hab108.64 of the present disclosure all have favorable binding effect with the tumor cell line MDA-MB-231.

### 4. Biacore detection of anti-CD73 antibodies

In this experiment, Biacore instrument was used to determine the affinity of the humanized anti-CD73 antibodies to be tested with human CD73 (huCD73) and monkey CD73 (cynoCD73).

A certain amount of test antibody was affinity-captured with Protein A biosensing chip (Cat. # 29127556, GE), and then a certain concentration of human or monkey CD73 antigen was allowed to flow through the surface of the chip. The reaction signals were detected in real time with Biacore instrument (Biacore T200, GE) to generate a binding and dissociation curve. At the end of each cycle, after the dissociation was completed, the biosensor chip was washed and regenerated with Glycine-HCl, pH1.5 (Cat. # BR-1003-54, GE). The running buffer for the assay was 1×HBS-EP buffer solution (Cat. # BR-1001-88, GE).

The data obtained from the experiment was fitted against (1:1) Langmuir model using GE Biacore T200 Evaluation version 3.0 software, and the affinity values were obtained, as shown in Table 12 and Table 13 for details.

Table 12: Reaction affinity of anti-CD73 antibody to huCD73 protein

| Antibody | ka (1/Ms) | kd (1/s) | KD (M) |
|---|---|---|---|
| CPX006 | 1.99E+05 | 3.68E-04 | 1.85E-09 |
| Hab110.21 | 6.50E+05 | 6.14E-05 | 9.44E-11 |
| Hab127.31d | 2.26E+05 | 5.81E-05 | 2.57E-10 |
| Hab108.64 | 2.24E+05 | 9.24E-05 | 4.12E-10 |
| CH108 | 1.60E+05 | 5.82E-05 | 3.65E-10 |
| CH110 | 5.56E+05 | 1.47E-04 | 2.65E-10 |
| CH 127 | 2.39E+05 | 3.31E-05 | 1.39E-10 |
| Hab 108.23 | 2.05E+05 | 1.87E-04 | 9.13E-10 |
| Hab108.34 | 1.06E+05 | 1.06E-04 | 9.99E-10 |
| Hab108.33 | 2.55E+05 | 1.23E-04 | 4.81E-10 |
| Hab 108.52 | 2.60E+05 | 2.41E-04 | 9.30E-10 |
| Hab108.53 | 2.57E+05 | 1.56E-04 | 6.09E-10 |
| Hab108.62 | 2.27E+05 | 8.68E-05 | 3.82E-10 |
| Hab108.63 | 2.28E+05 | 1.17E-04 | 5.13E-10 |
| Hab108.65 | 2.81E+05 | 6.93E-05 | 2.46E-10 |
| Hab108.15 | 2.49E+05 | 1.21E-04 | 4.85E-10 |
| Hab108.25 | 2.90E+05 | 1.01E-04 | 3.49E-10 |
| Hab108.35 | 2.90E+05 | 8.09E-05 | 2.80E-10 |
| Hab108.45 | 2.92E+05 | 7.38E-05 | 2.53E-10 |
| Hab110.22 | 3.38E+05 | 4.95E-05 | 1.47E-10 |
| Hab110.23 | 3.27E+05 | 5.43E-05 | 1.66E-10 |
| Hab110.24 | 3.30E+05 | 6.20E-05 | 1.88E-10 |
| Hab110.25 | 3.49E+05 | 7.79E-05 | 2.23E-10 |
| Hab110.31 | 3.64E+05 | 5.86E-05 | 1.61E-10 |
| Hab110.32 | 3.70E+05 | 6.94E-05 | 1.88E-10 |
| Hab110.34 | 3.58E+05 | 8.16E-05 | 2.28E-10 |
| Hab110.35 | 3.85E+05 | 9.40E-05 | 2.44E-10 |
| Hab127.13 | 1.50E+05 | 1.19E-04 | 7.90E-10 |
| Hab127.14 | 1.40E+05 | 1.18E-04 | 8.47E-10 |
| Hab127.21 | 1.62E+05 | 5.40E-05 | 3.33E-10 |
| Hab127.22 | 1.66E+05 | 4.86E-05 | 2.92E-10 |
| Hab127.23 | 1.66E+05 | 4.59E-05 | 2.76E-10 |
| Hab127.24 | 1.60E+05 | 4.48E-05 | 2.80E-10 |
| Hab127.31 | 1.63E+05 | 4.20E-05 | 2.57E-10 |
| Hab127.32 | 1.66E+05 | 4.60E-05 | 2.77E-10 |
| Hab127.33 | 1.75E+05 | 3.69E-05 | 2.11E-10 |
| Hab127.34 | 1.63E+05 | 3.58E-05 | 2.20E-10 |

(continued)

| Antibody | ka (1/Ms) | kd (1/s) | KD (M) |
|---|---|---|---|
| Hab127.41 | 2.30E+05 | 4.39E-05 | 1.91E-10 |
| Hab127.42 | 2.38E+05 | 3.94E-05 | 1.66E-10 |
| Hab127.43 | 2.39E+05 | 3.58E-05 | 1.50E-10 |
| Hab127.44 | 2.30E+05 | 4.15E-05 | 1.80E-10 |
| Hab127.31a | 2.19E+05 | 4.80E-05 | 2.19E-10 |
| Hab 127.3 1b | 2.20E+05 | 4.48E-05 | 2.04E-10 |
| Hab127.31c | 2.32E+05 | 4.76E-05 | 2.05E-10 |
| Hab127.31e | 1.77E+05 | 3.75E-05 | 2.12E-10 |
| Hab127.31f | 2.24E+05 | 4.33E-05 | 1.93E-10 |
| Hab127.31g | 1.63E+05 | 5.18E-05 | 3.19E-10 |
| Hab127.31h | 1.51E+05 | 6.66E-05 | 4.41E-10 |
| Hab127.31i | 1.36E+05 | 7.93E-05 | 5.85E-10 |

Table 13. Reaction affinity of anti-CD73 antibodies to cynoCD73 protein

| Antibody | ka (1/Ms) | kd (1/s) | KD (M) |
|---|---|---|---|
| Hab110.21 | 7.04E+05 | 3.45E-05 | 4.89E-11 |
| Hab 127.31d | 2.66E+05 | 1.86E-05 | 6.99E-11 |
| Hab108.64 | 2.56E+05 | 4.24E-05 | 1.66E-10 |

The experimental results show that the anti-CD73 antibodies Hab110.21, Hab127.31d and Hab108.64 of the present disclosure can all bind to human CD73 and monkey CD73 with high affinity.

**Example 4. Test of activity of anti-CD73 antibodies in inhibiting the enzyme activity**

The inhibitory effect of anti-CD73 antibodies on the enzyme activity was measured by chemiluminescence method, wherein the chemiluminescence method was used to detect the activity of antibodies in inhibiting the enzyme activity in MDA-MB-231 cells which highly express human CD73. The antibody was added into MDA-MB-231 cells, and the inhibitory effect on CD73 enzyme activity was determined by measuring the remaining amount of active substrate of CD73 enzyme. The specific experimental procedures were as follows:
MDA-MB-231 cells (ATCC, HTB-26) were seeded in a 96-well plate at a density of $2\times10^5$/ml, 100 $\mu$l/well, and cultured overnight. The supernatant was discarded, the plate was washed with PBS once, 50$\mu$l/well of various concentrations of each of the test antibodies (purified hybridoma antibodies or humanized antibodies, diluted with cell culture medium (RPMI-1640, Hyclone, Cat No SH30809.01)) were added, and incubated in an incubator at 37°C for 0.5 hour. Then 50$\mu$l/well of adenosine-5'-monophosphate (Sigma, Cat No A1752-5MG, diluted with cell culture medium) was added, and incubated in an incubator in $CO_2$ at 37°C for 3 hours. After the incubation was finished, 25$\mu$l/well of the reaction solution was transferred into a new 96-well plate (PerkinElmer, Cat No 6005290), 25$\mu$l/well of 5'-adenosine triphosphate (Sigma, Cat No A7699-5MG, diluted in cell culture medium) was added, and finally 50$\mu$l/well of CellTiter Glo (Promega, Cat No G7573) was added. The chemiluminescence values were read under Cytation5 (Biotek), data were analyzed with GraphPad Prism5, and the inhibition of enzyme activity in MDA-MB-231 cells by anti-CD73 antibodies was calculated as IC50 value.

Table 14: Experimental results of anti-CD73 antibodies in inhibiting the enzyme activity in MDA-MB-231 cell

| Test sample | IC50 (nM) in the assay showing the inhibition of enzyme activity in MDA-MB-231 cell |
|---|---|
| Hab110.21 | 0.329 |

(continued)

| Test sample | IC50 (nM) in the assay showing the inhibition of enzyme activity in MDA-MB-231 cell |
|---|---|
| Hab127.31d | 0.505 |
| Hab108.64 | 0.530 |
| CPX006 | 10.16 |
| MEDI9447 | 0.565 |

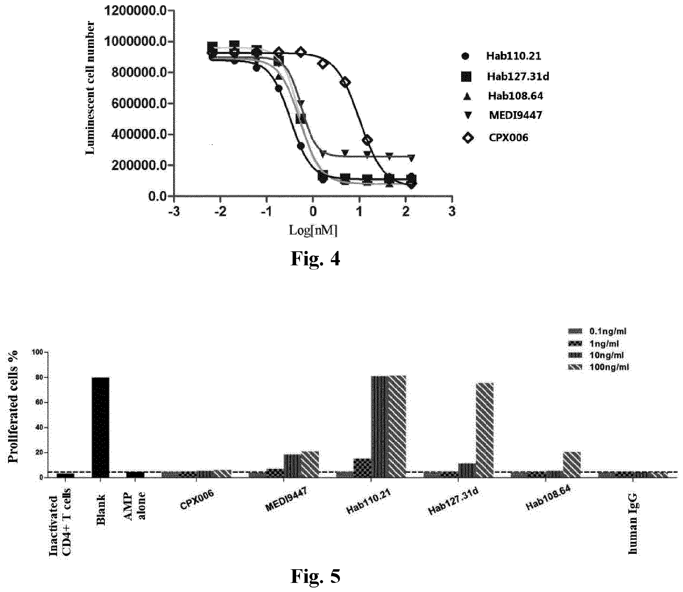

The experimental results show that the anti-CD73 antibodies Hab110.21, Hab127.31d, and Hab108.64 of the present disclosure all can significantly inhibit the enzyme activity of CD73 (expressed on tumor cells MDA-MB-231) on its substrates, and the maximal inhibitory effect on enzyme activity is higher than that of MEDI9447, a positive control antibody (see antibody MEDI9447 disclosed in patent application WO2016075099) (see Figure 4). The IC50 values of enzymatic hydrolysis are significantly lower than that of CPX006, a positive control antibody (see Table 14), indicating that the anti-CD73 antibodies of the present disclosure exhibit a higher ability in inhibiting CD73 enzyme activity than that of the two positive control antibodies MEDI9447 and CPX006.

## Example 5. *In vitro* experiment of CD4+ T cells proliferation induced by anti-CD73 antibody

CD4+ T cells were isolated and purified from PBMC prepared from human whole blood by using CD4 Microbeads (CD4 microspheres, Miltenyi Biotec, Cat# 130-045-101). CD4+ T cells were stained with Cell Proliferation Dye eFluor™ 670 (Invitrogen, Cat#65-0840). Human CD3/CD28 Dynabeads (CD3/CD28 immunomagnetic beads, Gibco, Cat#11131D) and 150 U/ml recombinant human IL-2 (PeproTech, Cat#200-02) were added to the stained CD4+T cells for activation, and then 50,000 cells per well (80 $\mu$l per well) were added into a round-bottom 96-well plate, and incubated in an incubator at 37°C, 5% $CO_2$ for 1 hour. Then 10 $\mu$l of CD73 antibody or control molecules (including MEDI9447, CPX006 positive control, medium blank control, and isotype human IgG negative control) was added into each well, with the final concentration of 100 ng/ml, 10 ng/ml, 1 ng/ ml and 0.1 ng/ml, and incubated in an incubator at 37°C, 5% $CO_2$, for an hour as pre-incubation, finally 10 $\mu$l of AMP (adenosine-5'-sodium monophosphate, SIGMA, Cat#A1752-1G) was added to each well, with a final concentration of 400 $\mu$M, and cultured in an incubator at 37°C, 5% $CO_2$ for 4 days. The medium used for the culture of CD4+ T cells was X-VIVO™ 15 serum-free medium (Lonza, Cat#04-418QCN). After 4 days, the cells were washed with flow buffer (PBS containing 2.5% FBS), and the cells were detected by flow cytometry to obtain APC Fluorescence value.

The experimental results are shown in Figure 5. The results show that the non-activated CD4+ T cells do not proliferate. For the activated cells, about 80% of the CD4+ T cells in the blank control wells (medium alone) proliferate, whereas 400 $\mu$M AMP significantly inhibits the proliferation of CD4+ T cells, and only about 5% of the cells proliferate. Anti-CD73 antibodies Hab110.21, Hab127.31d and Hab108.64 can reverse the effect of AMP on inhibiting CD4+ T cell proliferation to varying degrees.

## Example 6: *In vivo* biological activity of anti-CD73 antibody in animals

## 1. Experiment of anti-CD73 antibody inhibiting MDA-MB-231 subcutaneous xenograft tumor

Experimental method: 100 $\mu$l of MDA-MB-231 (ATCC bank) cells ($2.0 \times 10^6$ cells) were subcutaneously inoculated into the right flank of 50 NSG mice (B-NDG® mice, purchased from Beijing Biocytogene Biotechnology Co., Ltd.). After tumor was developed (~160 mm$^3$), the mice with too large or too small body weight or tumor volume were excluded. The remaining mice were randomly divided, based the tumor volume, into 6 groups, 6 mice per group., as shown in Table 15. PBMCs (Peripheral Blood Mononuclear Cells) were isolated from the blood of two volunteers, and mixed at a ratio of 1:1 without stimulation. $0.715 \times 10^7$/100 $\mu$l were injected intraperitoneally into mice. From the next day, the anti-CD73 antibodies of the present disclosure and control molecules (including isotype human IgG blank control, MEDI9447 positive control, CPX006 positive control) were injected intraperitoneally, twice a week, for a total of 3 weeks. The tumor volume and body weight were measured twice a week, and the data were recorded. Grouping and dosing are shown in Table 15.

Table 15: Test grouping and dosing

| Group | Animal Number | medicament | Dose (mg/ml) | Route of administ ration | Frequency of administration |
|---|---|---|---|---|---|
| 1 | 6 | Human IgG | 10 | i.p. | Twice a week |
| 2 | 6 | MEDI9447 | 10 | i.p. | Twice a week |
| 3 | 6 | CPX006 | 10 | i.p. | Twice a week |
| 4 | 6 | Hab110.21 | 10 | i.p. | Twice a week |
| 5 | 6 | Hab 127.31d | 10 | i.p. | Twice a week |
| 6 | 6 | Hab108.64 | 10 | i.p. | Twice a week |

Note: i.p. means intraperitoneal injection.

Excel statistical software was used: the average value was calculated as avg (Average); SD (Standard deviation) value was calculated as STDEV; SEM (Standard Error of Mean) value was calculated as STDEV/SQRT (Square root) (number of animals in each group); GraphPad Prism software was used for graphing, Two-way ANOVA (two-way analysis of variance) or One-way ANOVA (one-way analysis of variance) was used for statistical analysis of data.

The tumor volume (V) was calculated according to the following formula:

$$V = 1/2 \times L_{long} \times L_{short}^2$$

$$\text{Relative volume (RTV)} = V_T / V_0$$

$$\text{Tumor inhibition rate (\%)} = (C_{RTV} - T_{RTV})/C_{RTV} \, (\%)$$

Wherein, $V_0$ and $V_T$ represent the tumor volume at the beginning of and at the end of the experiment, respectively. $C_{RTV}$ and $T_{RTV}$ represent the relative tumor volume of the blank control group (Vehicle) and the test group at the end of the experiment, respectively.

**Experimental results:**

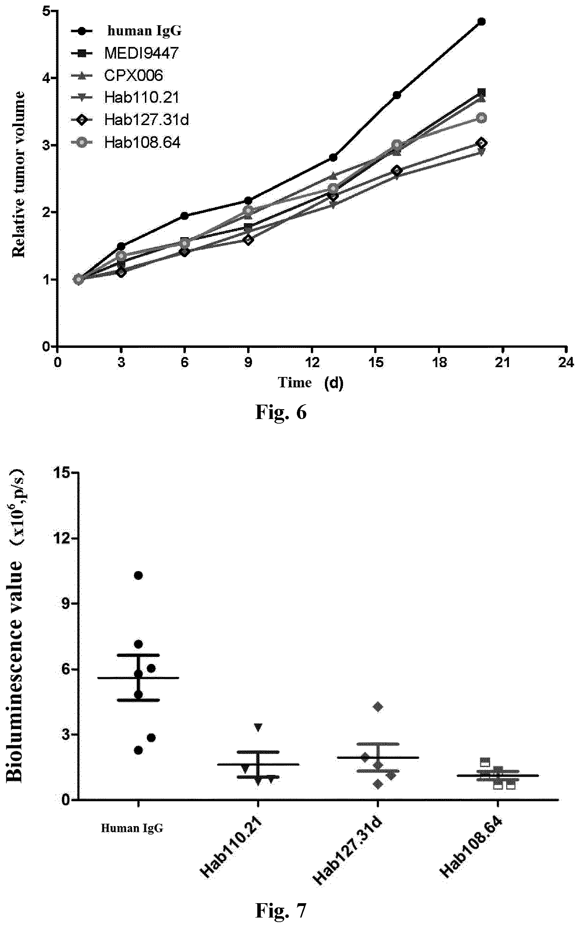

From D1 to D20, subcutaneous injection was performed twice a week, and the effect of anti-CD73 antibodies on inhibiting the growth of MDA-MB-231 tumor was detected. The experimental results are shown in Table 16 and Figure 6. The body weight of mice in each group did not change significantly. When compared with the isotype human IgG blank control group, the tumor inhibition rates of group MEDI9447, CPX006, Hab110.21, Hab127.31d and Hab108.64 group were 16%, 18%, 36%, 33%, and 25%, respectively. Hab110.21, Hab127.31d and Hab108.64 groups can significantly inhibit the growth of MDA-MB-231 tumors (p <0.01).

Table 16: Experimental results showing the inhibition effect of anti-CD73 antibodies on xenograft tumor in MDA-MB-231 mice

| Grouping | Average tumor volume ($mm^3$) | | Average tumor volume ($mm^3$) | | Relative tumor volume ($mm^3$) | | % Tumor inhibiti on rate | P (vs blank control) |
|---|---|---|---|---|---|---|---|---|
| | D0 | SEM | D20 | SEM | D20 | SEM | D20 | |
| Human IgG | 168.85 | 18.23 | 797.12 | 47.92 | 4.53 | 0.14 | - | - |
| MEDI9447 | 160.64 | 16.18 | 593.95 | 54.06 | 3.78 | 0.28 | 16% | p<0.01 |
| CPX006 | 163.63 | 18.46 | 591.71 | 44.45 | 3.7 | 0.15 | 18% | p<0.01 |
| Hab110.21 | 172.47 | 15.06 | 496.26 | 75.81 | 2.89 | 0.38 | 36% | p<0.01 |
| Hab 127.31d | 159.82 | 15.62 | 498.86 | 89.56 | 3.04 | 0.31 | 33% | p<0.01 |
| Hab108.64 | 163.54 | 14.41 | 537.56 | 49.29 | 3.4 | 0.41 | 25% | p<0.01 |

**2. Inhibition experiment of anti-CD73 antibody on lung metastatic MDA-MB-231-Luc**

**experimental method:**

MDA-MB-231-LUC cells (Caliper, USA) were cultivated, and incubated with human isotype IgG or CD73 antibody for 2 hours before inoculation, the final concentration of the antibody was 10 μg/ml, and then 200μl ($2.5 \times 10^5$ cells) was injected into the tail vein of each of the NSG mice. The mice were divided into 4 groups, 5-7 mice per group, as shown in Table 17. PBMCs (Peripheral Blood Mononuclear Cells) were isolated from the blood of two volunteers, and mixed at a ratio of 1:1 without stimulation. $0.57 \times 10^7/100$ μl were injected intraperitoneally into mice. From the next day, the antibody was injected intraperitoneally, twice a week for a total of 3 weeks. The *in vivo* bioluminescence values were measured twice a week, the body weight was measured and the data were recorded. Grouping and dosing are shown in Table 17.

Table 17: Test grouingp and dosing

| Group | Animal Number | medicament | Dose (mg/ml) | Route of administra tion | Frequency of administration |
|---|---|---|---|---|---|
| 1 | 7 | Human IgG | 10 | i.p. | Twice a week |
| 2 | 5 | Hab110.21 | 10 | i.p. | Twice a week |
| 3 | 6 | Hab 127.31d | 10 | i.p. | Twice a week |
| 4 | 6 | Hab108.64 | 10 | i.p. | Twice a week |

Note: i.p. means intraperitoneal injection.

Excel statistical software was used: both the body weight of each animal group, and the tumor bioluminescence value ROI value (region of interest; unit p/s (photons per second, the number of photons emitted from the animal body surface per unit time)) are represented as Mean ± SEM. GraphPad Prism software was used for graphing, and the data were statistically analyzed by Two-way ANOVA.

relative lung bioluminescence value: relative ROI (R-ROI)=$ROI_T/ROI_0$, $ROI_T$ is the lung bioluminescence value at the end of the experiment, and ROIo is the lung bioluminescence value at the beginning of the experiment.

$$\text{Tumor inhibition rate (\%)} = (T_{R\text{-}ROI} - C_{R\text{-}ROI})/C_{R\text{-}ROI}\,(\%)$$

Wherein, $T_{R\text{-}ROI}$ and $C_{R\text{-}ROI}$ are the relative lung bioluminescence values of the administration group and the control group at the end of the experiment, respectively.

**Experimental results:**

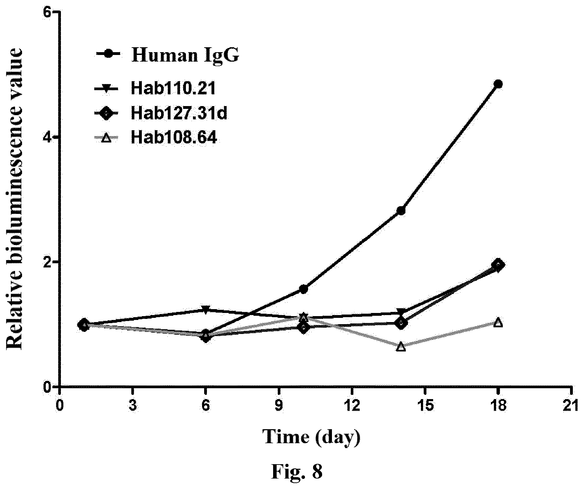

From D1 to D18, subcutaneous injection was performed twice a week, and the effect of anti-CD73 antibodies on inhibiting the lung metastatic MDA-MB-231-Luc was detected. The experimental results are shown in Table 18, Figure 7 and Figure 8. When compared with the human isotype IgG blank control group, the tumor inhibition rates of the Hab110.21, Hab127.31d, and Hab108.64 groups are 61% (p < 0.001), 60% (p < 0.001) and 78% (p < 0.001) respectively. All the groups can significantly inhibit the lung metastasis and growth of MDA-MB-231-LUC cells.

Table 18: Efficacy of anti-CD73 antibodies on lung metastasis in mice inoculated with MDA-MB-231-LUC

| Grouping | Average bioluminescence value ROI (Unit: $\times 10^6$) | | | | Relative bioluminescence value ROI | | % Tumor inhibition rate | P (vs blank control) |
|---|---|---|---|---|---|---|---|---|
| | D0 | SEM | D18 | SEM | D18 | SEM | D18 | |
| Human IgG | 1.26 | 0.12 | 5.6 | 1.02 | 4.85 | 1.14 | - | - |
| Hab110.21 | 1.04 | 0.25 | 1.62 | 0.51 | 1.89 | 0.7 | 61% | p<0.01 |
| Hab127.31d | 1.07 | 0.18 | 1.94 | 0.56 | 1.96 | 0.63 | 60% | p<0.01 |
| Hab108.64 | 1.17 | 0.2 | 1.12 | 0.18 | 1.05 | 0.22 | 78% | p<0.01 |

Example 7: *In vivo* pharmacokinetic experiment of anti-CD73 antibody in mice

Eighteen C57 mice, females, were purchased from Sippr-BK Lab Animal Co., Ltd. Mice were adapted to the laboratory environment for 4-day, free access to food and water, 12/12 hour light/dark cycle, at a temperature of 16-26 °C, relative humidity 40-70%, and the administration was started when the body weight ranged from 18g to 22g. On the day of the experiment, the mice were equally divided into 6 groups, wherein three groups were injected with the agents to be tested (Hab108.64, Hab110.21, and Hab 127.31d) via tail vein (IV), and the other three groups were injected subcutaneously (SC) with the agents to be tested (Hab108.64, Hab110.21 and Hab127.31d). The administration dose was 3mg/kg, and the administration volume was 10mL/kg.

Blood samples were collected at various time points, i.e., prior to administration, and 5min, 8h, 1d, 2d, 4d, 7d, 10d, 14d, 21d and 28d after administration. About 0.1 ml whole blood was drawn from each animal without adding anticoagulant. The blood samples were placed at 4°C for 30 minutes, centrifuged at 1000g for 15 minutes, and the upper serum was transferred into an EP tube and stored at -80°C.

The concentration of the antibodies in serum was detected by ELISA method, and the pharmacokinetic parameters of the agents to be tested were calculated by Winnolin software. The experimental results are shown in Table 19. The results show that the Hab108.64, Hab110.21 and Hab127.31d of the present disclosure have favorable *in vivo* stability and high bioavailability, and are suitable for subcutaneous injection.

Table 19: *In vivo* pharmacokinetic test results of anti-CD73 antibodies in mice

| | Hab 108.64 IV | | Hab 108.64 Sc | | Hab110.21 IV | | Hab110.21 Sc | | Hab 127.31d IV | | Hab 127.31d Sc | |
|---|---|---|---|---|---|---|---|---|---|---|---|---|
| Dose(mg/kg) | 3 | RSD | 3 | RSD | 3 | RSD | 3 | RSD | 3 | RSD | 3 | RSD |
| C max (ug/ml) | 79.2 | 7.4% | 19.0 | 15.8% | 59.6 | 2.0% | 20.0 | 2.0% | 76.1 | 2.0% | 20.0 | 6.9% |
| AUC 0-% (ug/ml*h) | 6178.7 | 15.9% | 5358.2 | 11.5% | 5214.3 | 7.5% | 5960.3 | 7.1% | 4904.7 | 6.3% | 5107.4 | 9.0% |
| **Bioavailability** | | | 84.2% | | | | 112.6% | | | | 104.2% | |
| $t_{1/2}$ (h) | 197.4 | 17.5% | 213.4 | 12.1% | 227.9 | 16.3% | 219.8 | 15.9% | 209.9 | 5.5% | 191.8 | 8.4% |
| $t_{1/2}$ (d) | 8.2 | 17.5% | 8.9 | 12.1% | 9.5 | 16.3% | 9.2 | 15.9% | 8.7 | 5.5% | 8.0 | 8.4% |

Note: Dose (administration dose), Cmax (maximum blood concentration), $AUC_{0-\infty}$ (area under the drug-time curve), Bioavailability (bioavailability), t1/2(h): half-life (in hours), T1/2(d): half-life (in days), RSD (relative standard deviation), IV (intravenous injection), Sc (subcutaneous injection).

Sequence listing

<110> JIANGSU HENGRUI MEDICINE CO., LTD.;
      SHANGHAI HENGRUI PHARMACEUTICAL CO., LTD.

<120> Anti-CD73 antibody, antigen binding fragment thereof and application thereof

<130> 719090CPCT

<160> 84

<170> SIPOSequenceListing 1.0

<210> 1
<211> 574
<212> PRT
<213> Artificial Sequence

<220>
<221> DOMAIN
<223> human CD73 full-length amino acid sequence

<400> 1
Met Cys Pro Arg Ala Ala Arg Ala Pro Ala Thr Leu Leu Leu Ala Leu
1               5                   10                  15
Gly Ala Val Leu Trp Pro Ala Ala Gly Ala Trp Glu Leu Thr Ile Leu
            20                  25                  30
His Thr Asn Asp Val His Ser Arg Leu Glu Gln Thr Ser Glu Asp Ser
        35                  40                  45
Ser Lys Cys Val Asn Ala Ser Arg Cys Met Gly Gly Val Ala Arg Leu
    50                  55                  60
Phe Thr Lys Val Gln Gln Ile Arg Arg Ala Glu Pro Asn Val Leu Leu
65                  70                  75                  80
Leu Asp Ala Gly Asp Gln Tyr Gln Gly Thr Ile Trp Phe Thr Val Tyr
                85                  90                  95
Lys Gly Ala Glu Val Ala His Phe Met Asn Ala Leu Arg Tyr Asp Ala
            100                 105                 110
Met Ala Leu Gly Asn His Glu Phe Asp Asn Gly Val Glu Gly Leu Ile
        115                 120                 125
Glu Pro Leu Leu Lys Glu Ala Lys Phe Pro Ile Leu Ser Ala Asn Ile
    130                 135                 140
Lys Ala Lys Gly Pro Leu Ala Ser Gln Ile Ser Gly Leu Tyr Leu Pro
145                 150                 155                 160
Tyr Lys Val Leu Pro Val Gly Asp Glu Val Val Gly Ile Val Gly Tyr
                165                 170                 175
Thr Ser Lys Glu Thr Pro Phe Leu Ser Asn Pro Gly Thr Asn Leu Val
            180                 185                 190
Phe Glu Asp Glu Ile Thr Ala Leu Gln Pro Glu Val Asp Lys Leu Lys
            195                 200                 205
Thr Leu Asn Val Asn Lys Ile Ile Ala Leu Gly His Ser Gly Phe Glu
        210                 215                 220
Met Asp Lys Leu Ile Ala Gln Lys Val Arg Gly Val Asp Val Val Val
225                 230                 235                 240
Gly Gly His Ser Asn Thr Phe Leu Tyr Thr Gly Asn Pro Pro Ser Lys
                245                 250                 255
Glu Val Pro Ala Gly Lys Tyr Pro Phe Ile Val Thr Ser Asp Asp Gly
            260                 265                 270
Arg Lys Val Pro Val Val Gln Ala Tyr Ala Phe Gly Lys Tyr Leu Gly
            275                 280                 285
Tyr Leu Lys Ile Glu Phe Asp Glu Arg Gly Asn Val Ile Ser Ser His
    290                 295                 300
Gly Asn Pro Ile Leu Leu Asn Ser Ser Ile Pro Glu Asp Pro Ser Ile

```
305                     310                     315                     320
Lys Ala Asp Ile Asn Lys Trp Arg Ile Lys Leu Asp Asn Tyr Ser Thr
                325                     330                     335
Gln Glu Leu Gly Lys Thr Ile Val Tyr Leu Asp Gly Ser Ser Gln Ser
                340                     345                     350
Cys Arg Phe Arg Glu Cys Asn Met Gly Asn Leu Ile Cys Asp Ala Met
                355                     360                     365
Ile Asn Asn Asn Leu Arg His Thr Asp Glu Met Phe Trp Asn His Val
        370                     375                     380
Ser Met Cys Ile Leu Asn Gly Gly Gly Ile Arg Ser Pro Ile Asp Glu
385                     390                     395                     400
Arg Asn Asn Gly Thr Ile Thr Trp Glu Asn Leu Ala Ala Val Leu Pro
                405                     410                     415
Phe Gly Gly Thr Phe Asp Leu Val Gln Leu Lys Gly Ser Thr Leu Lys
                420                     425                     430
Lys Ala Phe Glu His Ser Val His Arg Tyr Gly Gln Ser Thr Gly Glu
        435                     440                     445
Phe Leu Gln Val Gly Gly Ile His Val Val Tyr Asp Leu Ser Arg Lys
        450                     455                     460
Pro Gly Asp Arg Val Val Lys Leu Asp Val Leu Cys Thr Lys Cys Arg
465                     470                     475                     480
Val Pro Ser Tyr Asp Pro Leu Lys Met Asp Glu Val Tyr Lys Val Ile
                485                     490                     495
Leu Pro Asn Phe Leu Ala Asn Gly Gly Asp Gly Phe Gln Met Ile Lys
                500                     505                     510
Asp Glu Leu Leu Arg His Asp Ser Gly Asp Gln Asp Ile Asn Val Val
        515                     520                     525
Ser Thr Tyr Ile Ser Lys Met Lys Val Ile Tyr Pro Ala Val Glu Gly
        530                     535                     540
Arg Ile Lys Phe Ser Thr Gly Ser His Cys His Gly Ser Phe Ser Leu
545                     550                     555                     560
Ile Phe Leu Ser Leu Trp Ala Val Ile Phe Val Leu Tyr Gln
                565                     570
```

<210> 2
<211> 562
<212> PRT
<213> Artificial Sequence

<220>
<221> DOMAIN
<223> CD73-3Flag amino acid sequence

<400> 2

```
Met Glu Phe Gly Leu Ser Trp Leu Phe Leu Val Ala Ile Leu Lys Gly
1                   5                       10                      15
Val Gln Cys Trp Glu Leu Thr Ile Leu His Thr Asn Asp Val His Ser
                20                      25                      30
Arg Leu Glu Gln Thr Ser Glu Asp Ser Ser Lys Cys Val Asn Ala Ser
        35                      40                      45
Arg Cys Met Gly Gly Val Ala Arg Leu Phe Thr Lys Val Gln Gln Ile
        50                      55                      60
Arg Arg Ala Glu Pro Asn Val Leu Leu Leu Asp Ala Gly Asp Gln Tyr
65                      70                      75                      80
Gln Gly Thr Ile Trp Phe Thr Val Tyr Lys Gly Ala Glu Val Ala His
                85                      90                      95
Phe Met Asn Ala Leu Arg Tyr Asp Ala Met Ala Leu Gly Asn His Glu
                100                     105                     110
Phe Asp Asn Gly Val Glu Gly Leu Ile Glu Pro Leu Leu Lys Glu Ala
        115                     120                     125
Lys Phe Pro Ile Leu Ser Ala Asn Ile Lys Ala Lys Gly Pro Leu Ala
        130                     135                     140
```

```
Ser Gln Ile Ser Gly Leu Tyr Leu Pro Tyr Lys Val Leu Pro Val Gly
145             150             155             160
Asp Glu Val Val Gly Ile Val Gly Tyr Thr Ser Lys Glu Thr Pro Phe
            165             170             175
Leu Ser Asn Pro Gly Thr Asn Leu Val Phe Glu Asp Glu Ile Thr Ala
        180             185             190
Leu Gln Pro Glu Val Asp Lys Leu Lys Thr Leu Asn Val Asn Lys Ile
    195             200             205
Ile Ala Leu Gly His Ser Gly Phe Glu Met Asp Lys Leu Ile Ala Gln
210             215             220
Lys Val Arg Gly Val Asp Val Val Val Gly Gly His Ser Asn Thr Phe
225             230             235             240
Leu Tyr Thr Gly Asn Pro Pro Ser Lys Glu Val Pro Ala Gly Lys Tyr
            245             250             255
Pro Phe Ile Val Thr Ser Asp Asp Gly Arg Lys Val Pro Val Val Gln
            260             265             270
Ala Tyr Ala Phe Gly Lys Tyr Leu Gly Tyr Leu Lys Ile Glu Phe Asp
        275             280             285
Glu Arg Gly Asn Val Ile Ser Ser His Gly Asn Pro Ile Leu Leu Asn
290             295             300
Ser Ser Ile Pro Glu Asp Pro Ser Ile Lys Ala Asp Ile Asn Lys Trp
305             310             315             320
Arg Ile Lys Leu Asp Asn Tyr Ser Thr Gln Glu Leu Gly Lys Thr Ile
            325             330             335
Val Tyr Leu Asp Gly Ser Ser Gln Ser Cys Arg Phe Arg Glu Cys Asn
        340             345             350
Met Gly Asn Leu Ile Cys Asp Ala Met Ile Asn Asn Asn Leu Arg His
        355             360             365
Thr Asp Glu Met Phe Trp Asn His Val Ser Met Cys Ile Leu Asn Gly
370             375             380
Gly Gly Ile Arg Ser Pro Ile Asp Glu Arg Asn Asn Gly Thr Ile Thr
385             390             395             400
Trp Glu Asn Leu Ala Ala Val Leu Pro Phe Gly Gly Thr Phe Asp Leu
            405             410             415
Val Gln Leu Lys Gly Ser Thr Leu Lys Lys Ala Phe Glu His Ser Val
        420             425             430
His Arg Tyr Gly Gln Ser Thr Gly Glu Phe Leu Gln Val Gly Gly Ile
        435             440             445
His Val Val Tyr Asp Leu Ser Arg Lys Pro Gly Asp Arg Val Val Lys
    450             455             460
Leu Asp Val Leu Cys Thr Lys Cys Arg Val Pro Ser Tyr Asp Pro Leu
465             470             475             480
Lys Met Asp Glu Val Tyr Lys Val Ile Leu Pro Asn Phe Leu Ala Asn
            485             490             495
Gly Gly Asp Gly Phe Gln Met Ile Lys Asp Glu Leu Leu Arg His Asp
        500             505             510
Ser Gly Asp Gln Asp Ile Asn Val Val Ser Thr Tyr Ile Ser Lys Met
    515             520             525
Lys Val Ile Tyr Pro Ala Val Glu Gly Arg Ile Lys Asp Tyr Lys Asp
    530             535             540
His Asp Gly Asp Tyr Lys Asp His Asp Ile Asp Tyr Lys Asp Asp Asp
545             550             555             560
Asp Lys
```

```
<210>  3
<211>  556
<212>  PRT
<213>  Artificial Sequence

<220>
<221>  DOMAIN
<223>  CD73-Myc-His amino acid sequence
```

```
<400>  3
Met Glu Phe Gly Leu Ser Trp Leu Phe Leu Val Ala Ile Leu Lys Gly
1               5                   10                  15
Val Gln Cys Trp Glu Leu Thr Ile Leu His Thr Asn Asp Val His Ser
            20                  25                  30
Arg Leu Glu Gln Thr Ser Glu Asp Ser Ser Lys Cys Val Asn Ala Ser
        35                  40                  45
Arg Cys Met Gly Gly Val Ala Arg Leu Phe Thr Lys Val Gln Gln Ile
        50                  55                  60
Arg Arg Ala Glu Pro Asn Val Leu Leu Leu Asp Ala Gly Asp Gln Tyr
65                  70                  75                  80
Gln Gly Thr Ile Trp Phe Thr Val Tyr Lys Gly Ala Glu Val Ala His
                85                  90                  95
Phe Met Asn Ala Leu Arg Tyr Asp Ala Met Ala Leu Gly Asn His Glu
            100                 105                 110
Phe Asp Asn Gly Val Glu Gly Leu Ile Glu Pro Leu Leu Lys Glu Ala
        115                 120                 125
Lys Phe Pro Ile Leu Ser Ala Asn Ile Lys Ala Lys Gly Pro Leu Ala
        130                 135                 140
Ser Gln Ile Ser Gly Leu Tyr Leu Pro Tyr Lys Val Leu Pro Val Gly
145                 150                 155                 160
Asp Glu Val Val Gly Ile Val Gly Tyr Thr Ser Lys Glu Thr Pro Phe
                165                 170                 175
Leu Ser Asn Pro Gly Thr Asn Leu Val Phe Glu Asp Glu Ile Thr Ala
            180                 185                 190
Leu Gln Pro Glu Val Asp Lys Leu Lys Thr Leu Asn Val Asn Lys Ile
        195                 200                 205
Ile Ala Leu Gly His Ser Gly Phe Glu Met Asp Lys Leu Ile Ala Gln
        210                 215                 220
Lys Val Arg Gly Val Asp Val Val Val Gly Gly His Ser Asn Thr Phe
225                 230                 235                 240
Leu Tyr Thr Gly Asn Pro Pro Ser Lys Glu Val Pro Ala Gly Lys Tyr
                245                 250                 255
Pro Phe Ile Val Thr Ser Asp Asp Gly Arg Lys Val Pro Val Val Gln
            260                 265                 270
Ala Tyr Ala Phe Gly Lys Tyr Leu Gly Tyr Leu Lys Ile Glu Phe Asp
        275                 280                 285
Glu Arg Gly Asn Val Ile Ser Ser His Gly Asn Pro Ile Leu Leu Asn
        290                 295                 300
Ser Ser Ile Pro Glu Asp Pro Ser Ile Lys Ala Asp Ile Asn Lys Trp
305                 310                 315                 320
Arg Ile Lys Leu Asp Asn Tyr Ser Thr Gln Glu Leu Gly Lys Thr Ile
                325                 330                 335
Val Tyr Leu Asp Gly Ser Ser Gln Ser Cys Arg Phe Arg Glu Cys Asn
            340                 345                 350
Met Gly Asn Leu Ile Cys Asp Ala Met Ile Asn Asn Asn Leu Arg His
        355                 360                 365
Thr Asp Glu Met Phe Trp Asn His Val Ser Met Cys Ile Leu Asn Gly
        370                 375                 380
Gly Gly Ile Arg Ser Pro Ile Asp Glu Arg Asn Asn Gly Thr Ile Thr
385                 390                 395                 400
Trp Glu Asn Leu Ala Ala Val Leu Pro Phe Gly Gly Thr Phe Asp Leu
                405                 410                 415
Val Gln Leu Lys Gly Ser Thr Leu Lys Lys Ala Phe Glu His Ser Val
            420                 425                 430
His Arg Tyr Gly Gln Ser Thr Gly Glu Phe Leu Gln Val Gly Gly Ile
        435                 440                 445
His Val Val Tyr Asp Leu Ser Arg Lys Pro Gly Asp Arg Val Val Lys
        450                 455                 460
Leu Asp Val Leu Cys Thr Lys Cys Arg Val Pro Ser Tyr Asp Pro Leu
465                 470                 475                 480
```

```
Lys Met Asp Glu Val Tyr Lys Val Ile Leu Pro Asn Phe Leu Ala Asn
            485                 490                 495
Gly Gly Asp Gly Phe Gln Met Ile Lys Asp Glu Leu Leu Arg His Asp
            500                 505                 510
Ser Gly Asp Gln Asp Ile Asn Val Ser Thr Tyr Ile Ser Lys Met
            515                 520                 525
Lys Val Ile Tyr Pro Ala Val Glu Gly Arg Ile Lys Glu Gln Lys Leu
            530                 535                 540
Ile Ser Glu Glu Asp Leu His His His His His His
545                 550                 555
```

<210> 4
<211> 113
<212> PRT
<213> Artificial Sequence

<220>
<221> DOMAIN
<223> mab108 HCVR amino acid sequence

```
<400> 4
Gln Val Gln Leu Lys Gln Ser Gly Pro Gly Leu Val Gln Pro Ser Gln
1               5                   10                  15
Ser Leu Ser Ile Thr Cys Thr Val Phe Gly Phe Ser Leu Lys Ser Tyr
            20                  25                  30
Gly Ile Gln Trp Val Arg Gln Ser Pro Gly Lys Gly Leu Glu Trp Leu
            35                  40                  45
Gly Val Ile Trp Ser Gly Gly Ser Ala Asp Tyr Asn Ala Ala Phe Ile
        50                  55                  60
Ser Arg Leu Ser Ile Ser Lys Asp Asn Ser Lys Ser Gln Val Phe Phe
65                  70                  75                  80
Lys Met Asn Ser Leu Gln Gly Asp Asp Thr Ala Ile Tyr Tyr Cys Ala
                85                  90                  95
Gly Gln Tyr Gly Ser Val Trp Gly Gln Gly Thr Ser Val Thr Val Ser
            100                 105                 110
Ser
```

<210> 5
<211> 106
<212> PRT
<213> Artificial Sequence

<220>
<221> DOMAIN
<223> mab108 LCVR amino acid sequence

```
<400> 5
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Asp Arg
            20                  25                  30
Leu Asn Trp Leu Gln Gln Glu Pro Asp Gly Thr Ile Lys Arg Leu Ile
            35                  40                  45
Tyr Ala Thr Ser Ser Leu Asp Ser Gly Val Pro Lys Arg Phe Ser Gly
        50                  55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Gly Ser Trp Thr
                85                  90                  95
Phe Gly Gly Gly Thr Lys Leu Glu Ile Lys
```

100                     105

```
<210>  6
<211>  120
<212>  PRT
<213>  Artificial Sequence

<220>
<221>  DOMAIN
<223>  mab110 HCVR amino acid sequence


<400>  6
Gln Val Gln Leu Gln Gln Ser Gly Ala Glu Leu Ala Arg Pro Gly Ala
1               5                   10                  15
Ser Val Gln Met Ser Cys Lys Ala Ser Gly Tyr Ile Phe Thr Ser Tyr
            20                  25                  30
Thr Met His Trp Val Lys Gln Arg Pro Gly Gln Gly Leu Glu Trp Ile
        35                  40                  45
Gly Tyr Ile Asn Pro Asn Ser Phe Tyr Ile Glu Tyr Asn Gln Lys Phe
    50                  55                  60
Lys Asp Lys Ala Thr Leu Thr Ala Asp Thr Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Gly Asp Tyr Asp Leu Phe Tyr Asp Met Asp Asn Trp Gly Gln
            100                 105                 110
Gly Thr Ser Val Thr Val Ser Ser
            115                 120


<210>  7
<211>  107
<212>  PRT
<213>  Artificial Sequence

<220>
<221>  DOMAIN
<223>  mab110  LCVR amino acid sequence


<400>  7
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Asn Ser
            20                  25                  30
Leu Asn Trp Leu Gln Gln Glu Pro Asp Gly Thr Ile Lys Arg Leu Ile
        35                  40                  45
Tyr Ala Thr Phe Arg Leu Asp Ser Gly Val Pro Lys Arg Phe Ser Gly
    50                  55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Gln Gln Tyr Ala Ser Phe Pro Trp
                85                  90                  95
Thr Phe Gly Gly Gly Thr Lys Leu Glu Ile Lys
            100                 105


<210>  8
<211>  114
<212>  PRT
<213>  Artificial Sequence

<220>
<221>  DOMAIN
```

<223> mab127 HCVR amino acid sequence


<400> 8
```
Glu Val Gln Leu Gln Gln Ser Gly Pro Glu Leu Val Lys Pro Gly Glu
1               5                   10                  15
Ser Val Lys Ile Ser Cys Lys Ala Ser Gly Tyr Ser Phe Thr Ala Tyr
            20              25                  30
Phe Met Asn Trp Val Met Gln Ser His Arg Lys Ser Leu Glu Trp Ile
        35                  40                  45
Gly Arg Ile Tyr His Asn Asn Gly Asp Thr Phe Tyr Ser Gln Lys Phe
        50              55                  60
Lys Gly Lys Ala Thr Leu Thr Val Asp Lys Ser Asn Thr Val His
65                  70                  75                  80
Met Glu Leu Arg Ser Leu Thr Ser Glu Asp Ser Ala Val Tyr Phe Cys
                85                  90                  95
Ala Thr Ser Tyr Val Gly Asp Trp Gly Gln Gly Thr Thr Leu Thr Val
                100                 105                 110
Ser Ser
```


<210> 9
<211> 107
<212> PRT
<213> Artificial Sequence


<220>
<221> DOMAIN
<223> mab127 LCVR amino acid sequence


<400> 9
```
Asp Ile Gln Met Thr Gln Ser Pro Ser Ser Leu Ser Ala Ser Leu Gly
1               5                   10                  15
Glu Arg Val Ser Leu Thr Cys Arg Ala Ser Gln Asp Ile Gly Asp Ser
            20              25                  30
Leu Asn Trp Leu Gln Gln Ala Pro Asp Gly Thr Leu Lys Arg Leu Ile
        35                  40                  45
Tyr Ala Thr Ser Ser Leu Asp Ser Gly Val Pro Arg Arg Phe Ser Gly
        50              55                  60
Ser Arg Ser Gly Ser Asp Tyr Ser Leu Thr Ile Ser Ser Leu Glu Ser
65                  70                  75                  80
Glu Asp Phe Val Asp Tyr Tyr Cys Leu Gln Tyr Ala Ser Phe Pro Trp
                85                  90                  95
Thr Phe Gly Gly Gly Thr Lys Leu Glu Ile Lys
                100                 105
```


<210> 10
<211> 5
<212> PRT
<213> Artificial Sequence


<220>
<221> DOMAIN
<223> mab108 HCDR1 amino acid sequence


<400> 10
```
Ser Tyr Gly Ile Gln
1               5
```


<210> 11
<211> 16
```

<212> PRT
<213> Artificial Sequence

<220>
<221> DOMAIN
<223> mab108 HCDR2 amino acid sequence

<400> 11
Val Ile Trp Ser Gly Gly Ser Ala Asp Tyr Asn Ala Ala Phe Ile Ser
1               5                   10                  15

<210> 12
<211> 5
<212> PRT
<213> Artificial Sequence

<220>
<221> DOMAIN
<223> mab108 HCDR3 amino acid sequence

<400> 12
Gln Tyr Gly Ser Val
1               5

<210> 13
<211> 11
<212> PRT
<213> Artificial Sequence

<220>
<221> DOMAIN
<223> mab108 LCDR1 amino acid sequence

<400> 13
Arg Ala Ser Gln Asp Ile Gly Asp Arg Leu Asn
1               5                   10

<210> 14
<211> 7
<212> PRT
<213> Artificial Sequence

<220>
<221> DOMAIN
<223> mab108, mab127 LCDR2 amino acid sequence

<400> 14
Ala Thr Ser Ser Leu Asp Ser
1               5

<210> 15
<211> 8
<212> PRT
<213> Artificial Sequence

<220>
<221> DOMAIN
<223> mab108 LCDR3 amino acid sequence

<400> 15
Leu Gln Tyr Ala Gly Ser Trp Thr
1               5

<210> 16
<211> 5
<212> PRT
<213> Artificial Sequence

<220>
<221> DOMAIN
<223> mab110 HCDR1 amino acid sequence

<400> 16
Ser Tyr Thr Met His
1               5

<210> 17
<211> 17
<212> PRT
<213> Artificial Sequence

<220>
<221> DOMAIN
<223> mab110 HCDR2 amino acid sequence

<400> 17
Tyr Ile Asn Pro Asn Ser Phe Tyr Ile Glu Tyr Asn Gln Lys Phe Lys
1               5                       10                      15
Asp

<210> 18
<211> 11
<212> PRT
<213> Artificial Sequence

<220>
<221> DOMAIN
<223> mab110 HCDR3 amino acid sequence

<400> 18
Gly Asp Tyr Asp Leu Phe Tyr Asp Met Asp Asn
1               5                       10

<210> 19
<211> 11
<212> PRT
<213> Artificial Sequence

<220>
<221> DOMAIN
<223> mab110 LCDR1 amino acid sequence

<400> 19
Arg Ala Ser Gln Asp Ile Gly Asn Ser Leu Asn
1               5                       10

48

<210> 20
<211> 7
<212> PRT
<213> Artificial Sequence

<220>
<221> DOMAIN
<223> mab110 LCDR2 amino acid sequence


<400> 20
Ala Thr Phe Arg Leu Asp Ser
1               5


<210> 21
<211> 9
<212> PRT
<213> Artificial Sequence

<220>
<221> DOMAIN
<223> mab110 LCDR3 amino acid sequence


<400> 21
Gln Gln Tyr Ala Ser Phe Pro Trp Thr
1               5


<210> 22
<211> 5
<212> PRT
<213> Artificial Sequence

<220>
<221> DOMAIN
<223> mab127 HCDR1 amino acid sequence


<400> 22
Ala Tyr Phe Met Asn
1               5


<210> 23
<211> 17
<212> PRT
<213> Artificial Sequence

<220>
<221> DOMAIN
<223> mab127 HCDR2 amino acid sequence


<400> 23
Arg Ile Tyr His Asn Asn Gly Asp Thr Phe Tyr Ser Gln Lys Phe Lys
1               5                   10                  15
Gly


<210> 24
<211> 5
<212> PRT
<213> Artificial Sequence

```
<220>
<221>  DOMAIN
<223>  mab127 HCDR3 amino acid sequence


<400>  24
Ser Tyr Val Gly Asp
1               5


<210>  25
<211>  11
<212>  PRT
<213>  Artificial Sequence


<220>
<221>  DOMAIN
<223>  mab127 LCDR1 amino acid sequence


<400>  25
Arg Ala Ser Gln Asp Ile Gly Asp Ser Leu Asn
1               5                   10


<210>  26
<211>  9
<212>  PRT
<213>  Artificial Sequence


<220>
<221>  DOMAIN
<223>  mab127 LCDR3 amino acid sequence


<400>  26
Leu Gln Tyr Ala Ser Phe Pro Trp Thr
1               5


<210>  27
<211>  11
<212>  PRT
<213>  Artificial Sequence


<220>
<221>  DOMAIN
<223>  mab108, mab110 and mab127 LCDR1 amino acid sequence general formula


<220>
<221>  DOMAIN
<222>  (8)..(8)
<223>  Xaa is selected from Asp or Asn


<220>
<221>  DOMAIN
<222>  (9)..(9)
<223>  Xaa is selected from Arg or Ser


<400>  27
Arg Ala Ser Gln Asp Ile Gly Xaa Xaa Leu Asn
1               5                   10
```

```
<210>  28
<211>  7
<212>  PRT
<213>  Artificial Sequence

<220>
<221>  DOMAIN
<223>  mab108, mab110 and mab127 LCDR2 amino acid sequence general formula


<220>
<221>  DOMAIN
<222>  (3)..(3)
<223>  Xaa is selected from Phe or Ser


<220>
<221>  DOMAIN
<222>  (4)..(4)
<223>  Xaa is selected from Arg or Ser


<400>  28
Ala Thr Xaa Xaa Leu Asp Ser
1               5

<210>  29
<211>  9
<212>  PRT
<213>  Artificial Sequence

<220>
<221>  DOMAIN
<223>  mab108, mab110 and mab127 LCDR2 amino acid sequence general formula


<220>
<221>  DOMAIN
<222>  (1)..(1)
<223>  Xaa is selected from Leu or Gln


<220>
<221>  DOMAIN
<222>  (5)..(5)
<223>  Xaa is selected from Ser or Gly


<220>
<221>  DOMAIN
<222>  (6)..(6)
<223>  Xaa is selected from Phe or absence


<220>
<221>  DOMAIN
<222>  (7)..(7)
<223>  Xaa is selected from Ser or Pro


<400>  29
Xaa Gln Tyr Ala Xaa Xaa Xaa Trp Thr
1               5
```

```
<210>   30
<211>   113
<212>   PRT
<213>   Artificial Sequence

<220>
<221>   DOMAIN
<223>   Hab108 HCVR, Hab108.VH1 amino acid sequence


<400>   30
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Ile Gln Trp Ile Arg Gln Pro Pro Gly Lys Leu Leu Glu Trp Leu
            35                  40                  45
Ala Val Ile Trp Ser Gly Gly Ser Ala Asp Tyr Asn Ala Ala Phe Ile
        50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Arg Gln Tyr Gly Ser Val Trp Gly Gln Gly Thr Leu Val Thr Val Ser
            100                 105                 110
Ser


<210>   31
<211>   106
<212>   PRT
<213>   Artificial Sequence

<220>
<221>   DOMAIN
<223>   Hab108 LCVR, Hab108.VL1 amino acid sequence


<400>   31
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
            20                  25                  30
Leu Asn Trp Tyr Gln Gln Lys Pro Gly Lys Ala Pro Lys Arg Leu Ile
            35                  40                  45
Tyr Ala Thr Ser Ser Leu Asp Ser Gly Val Pro Ser Arg Phe Ser Gly
        50                  55                  60
Ser Gly Ser Gly Thr Glu Phe Thr Leu Thr Ile Ser Ser Leu Gln Pro
65                  70                  75                  80
Glu Asp Phe Ala Thr Tyr Tyr Cys Leu Gln Tyr Ala Gly Ser Trp Thr
                85                  90                  95
Phe Gly Gly Gly Thr Lys Val Glu Ile Lys
            100                 105

<210>   32
<211>   106
<212>   PRT
<213>   Artificial Sequence

<220>
<221>   DOMAIN
<223>   Hab108.VL2 amino acid sequence
```

<400>  32
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
            20                  25                  30
Leu Asn Trp Leu Gln Gln Lys Pro Gly Lys Ala Ile Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Gly Ser Trp Thr
                85                  90                  95
Phe Gly Gly Gly Thr Lys Val Glu Ile Lys
            100                 105


<210>  33
<211>  106
<212>  PRT
<213>  Artificial Sequence

<220>
<221>  DOMAIN
<223>  Hab108.VL3   amino acid sequence


<400>  33
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
            20                  25                  30
Leu Asn Trp Leu Gln Gln Lys Pro Gly Gly Ala Ile Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Gly Ser Trp Thr
                85                  90                  95
Phe Gly Gly Gly Thr Lys Val Glu Ile Lys
            100                 105


<210>  34
<211>  106
<212>  PRT
<213>  Artificial Sequence

<220>
<221>  DOMAIN
<223>  Hab108.VL4   amino acid sequence


<400>  34
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
            20                  25                  30
Leu Asn Trp Leu Gln Gln Lys Pro Gly Gly Ala Ile Lys Arg Leu Ile
        35                  40                  45
Tyr Ala Thr Ser Ser Leu Asp Ser Gly Val Pro Ser Arg Phe Ser Gly
    50                  55                  60

```
Ser Gly Ser Gly Ser Glu Tyr Thr Leu Thr Ile Ser Ser Leu Gln Pro
65              70                  75                      80
Glu Asp Phe Ala Asp Tyr Tyr Cys Leu Gln Tyr Ala Gly Ser Trp Thr
                85              90                      95
Phe Gly Gly Gly Thr Lys Val Glu Ile Lys
            100                 105
```

```
<210>   35
<211>   106
<212>   PRT
<213>   Artificial Sequence

<220>
<221>   DOMAIN
<223>   Hab108.VL5   amino acid sequence
```

```
<400>   35
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
                20                  25                  30
Leu Asn Trp Leu Gln Gln Lys Pro Gly Lys Ala Ile Lys Arg Leu Ile
            35                  40                  45
Tyr Ala Thr Ser Ser Leu Asp Ser Gly Val Pro Lys Arg Phe Ser Gly
        50                  55                  60
Ser Gly Ser Gly Thr Glu Tyr Thr Leu Thr Ile Ser Ser Leu Gln Pro
65              70                  75                      80
Glu Asp Phe Ala Thr Tyr Tyr Cys Leu Gln Tyr Ala Gly Ser Trp Thr
                85              90                      95
Phe Gly Gly Gly Thr Lys Val Glu Ile Lys
            100                 105
```

```
<210>   36
<211>   106
<212>   PRT
<213>   Artificial Sequence

<220>
<221>   DOMAIN
<223>   Hab108.VL6   amino acid sequence
```

```
<400>   36
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
                20                  25                  30
Leu Asn Trp Leu Gln Gln Lys Pro Gly Gly Ala Ile Lys Arg Leu Ile
            35                  40                  45
Tyr Ala Thr Ser Ser Leu Asp Ser Gly Val Pro Lys Arg Phe Ser Gly
        50                  55                  60
Ser Gly Ser Gly Thr Glu Tyr Thr Leu Thr Ile Ser Ser Leu Gln Pro
65              70                  75                      80
Glu Asp Phe Ala Thr Tyr Tyr Cys Leu Gln Tyr Ala Gly Ser Trp Thr
                85              90                      95
Phe Gly Gly Gly Thr Lys Val Glu Ile Lys
            100                 105
```

```
<210>   37
<211>   113
<212>   PRT
<213>   Artificial Sequence
```

```
<220>
<221>  DOMAIN
<223>  Hab108.VH2   amino acid sequence


<400>  37
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Ile Gln Trp Ile Arg Gln Pro Pro Gly Lys Leu Leu Glu Trp Leu
        35                  40                  45
Ala Val Ile Trp Ser Gly Gly Ser Ala Asp Tyr Asn Ala Ala Phe Ile
    50                  55                  60
Ser Arg Leu Thr Ile Ser Lys Asp Thr Ser Lys Ser Gln Val Val Leu
65                  70                  75                      80
Thr Met Thr Asn Met Asp Pro Val Asp Thr Ala Thr Tyr Tyr Cys Ala
                85                  90                  95
Gly Gln Tyr Gly Ser Val Trp Gly Gln Gly Thr Leu Val Thr Val Ser
            100                 105                 110
Ser


<210>  38
<211>  113
<212>  PRT
<213>  Artificial Sequence

<220>
<221>  DOMAIN
<223>  Hab108.VH3   amino acid sequence


<400>  38
Glu Val Thr Leu Lys Glu Ser Gly Pro Val Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Ile Gln Trp Val Arg Gln Pro Pro Gly Lys Leu Leu Glu Trp Leu
        35                  40                  45
Gly Val Ile Trp Ser Gly Gly Ser Ala Asp Tyr Asn Ala Ala Phe Ile
    50                  55                  60
Ser Arg Leu Thr Ile Ser Lys Asp Thr Ser Lys Ser Gln Val Val Leu
65                  70                  75                      80
Thr Met Thr Asn Met Asp Pro Val Asp Thr Ala Thr Tyr Tyr Cys Ala
                85                  90                  95
Gly Gln Tyr Gly Ser Val Trp Gly Gln Gly Thr Leu Val Thr Val Ser
            100                 105                 110
Ser


<210>  39
<211>  113
<212>  PRT
<213>  Artificial Sequence

<220>
<221>  DOMAIN
<223>  Hab108.VH4   amino acid sequence


<400>  39
```

```
Glu Val Thr Leu Lys Gln Ser Gly Pro Gly Leu Val Lys Pro Thr Glu
1               5               10              15
Thr Leu Thr Leu Thr Cys Thr Val Ser Gly Phe Ser Leu Ser Ser Tyr
            20              25              30
Gly Ile Gln Trp Val Arg Gln Pro Pro Gly Lys Gly Leu Glu Trp Leu
        35              40              45
Gly Val Ile Trp Ser Gly Gly Ser Ala Asp Tyr Asn Ala Ala Phe Ile
    50              55              60
Ser Arg Leu Thr Ile Ser Lys Asp Thr Ser Lys Ser Gln Val Val Leu
65              70              75              80
Thr Met Thr Asn Met Asp Pro Val Asp Thr Ala Thr Tyr Tyr Cys Ala
            85              90              95
Gly Gln Tyr Gly Ser Val Trp Gly Gln Gly Thr Leu Val Thr Val Ser
            100             105             110
Ser
```

```
<210>   40
<211>   113
<212>   PRT
<213>   Artificial Sequence

<220>
<221>   DOMAIN
<223>   Hab108.VH5   amino acid sequence


<400>   40
Glu Val Thr Leu Lys Glu Ser Gly Pro Val Leu Val Lys Pro Thr Glu
1               5               10              15
Thr Leu Thr Leu Thr Cys Thr Val Ser Gly Phe Ser Leu Lys Ser Tyr
            20              25              30
Gly Ile Gln Trp Val Arg Gln Pro Pro Gly Lys Leu Leu Glu Trp Leu
        35              40              45
Gly Val Ile Trp Ser Gly Gly Ser Ala Asp Tyr Asn Ala Ala Phe Ile
    50              55              60
Ser Arg Leu Thr Ile Ser Lys Asp Thr Ser Lys Ser Gln Val Val Leu
65              70              75              80
Thr Met Thr Asn Met Asp Pro Val Asp Thr Ala Thr Tyr Tyr Cys Ala
            85              90              95
Gly Gln Tyr Gly Ser Val Trp Gly Gln Gly Thr Leu Val Thr Val Ser
            100             105             110
Ser
```

```
<210>   41
<211>   120
<212>   PRT
<213>   Artificial Sequence

<220>
<221>   DOMAIN
<223>   Hab110 HCVR, Hab110.VH1   amino acid sequence


<400>   41
Glu Val Gln Leu Val Gln Ser Gly Ala Glu Val Lys Lys Pro Gly Ala
1               5               10              15
Ser Val Lys Val Ser Cys Lys Ala Ser Gly Tyr Thr Phe Thr Ser Tyr
            20              25              30
Thr Met His Trp Val Arg Gln Ala Pro Gly Gln Arg Leu Glu Trp Met
        35              40              45
Gly Tyr Ile Asn Pro Asn Ser Phe Tyr Ile Glu Tyr Asn Gln Lys Phe
```

56

```
            50                      55                      60
    Lys Asp Arg Val Thr Ile Thr Arg Asp Thr Ser Ala Ser Thr Ala Tyr
    65                      70                      75                      80
    Met Glu Leu Ser Ser Leu Arg Ser Glu Asp Thr Ala Val Tyr Tyr Cys
                            85                      90                      95
    Ala Arg Gly Asp Tyr Asp Leu Phe Tyr Asp Met Asp Asn Trp Gly Gln
                    100                     105                     110
    Gly Thr Thr Val Thr Val Ser Ser
                    115                     120


    <210>   42
    <211>   107
    <212>   PRT
    <213>   Artificial Sequence

    <220>
    <221>   DOMAIN
    <223>   Hab110 LCVR, Hab110.VL1   amino acid sequence


    <400>   42
    Asp Ile Gln Met Thr Gln Ser Pro Ser Ser Leu Ser Ala Ser Val Gly
    1                       5                       10                      15
    Asp Arg Val Thr Ile Thr Cys Arg Ala Ser Gln Asp Ile Gly Asn Ser
                    20                      25                      30
    Leu Asn Trp Tyr Gln Gln Lys Pro Gly Lys Ala Pro Lys Arg Leu Ile
                35                      40                      45
    Tyr Ala Thr Phe Arg Leu Asp Ser Gly Val Pro Ser Arg Phe Ser Gly
            50                      55                      60
    Ser Gly Ser Gly Thr Glu Phe Thr Leu Thr Ile Ser Ser Leu Gln Pro
    65                      70                      75                      80
    Glu Asp Phe Ala Thr Tyr Tyr Cys Gln Gln Tyr Ala Ser Phe Pro Trp
                            85                      90                      95
    Thr Phe Gly Gly Gly Thr Lys Val Glu Ile Lys
                    100                     105


    <210>   43
    <211>   107
    <212>   PRT
    <213>   Artificial Sequence

    <220>
    <221>   DOMAIN
    <223>   Hab110.VL2   amino acid sequence


    <400>   43
    Asp Ile Gln Met Thr Gln Ser Pro Ser Ser Leu Ser Ala Ser Val Gly
    1                       5                       10                      15
    Asp Arg Val Thr Ile Thr Cys Arg Ala Ser Gln Asp Ile Gly Asn Ser
                    20                      25                      30
    Leu Asn Trp Leu Gln Gln Lys Pro Gly Lys Ala Ile Lys Arg Leu Ile
                35                      40                      45
    Tyr Ala Thr Phe Arg Leu Asp Ser Gly Val Pro Ser Arg Phe Ser Gly
            50                      55                      60
    Ser Arg Ser Gly Thr Glu Tyr Thr Leu Thr Ile Ser Ser Leu Gln Pro
    65                      70                      75                      80
    Glu Asp Phe Ala Thr Tyr Tyr Cys Gln Gln Tyr Ala Ser Phe Pro Trp
                            85                      90                      95
    Thr Phe Gly Gly Gly Thr Lys Val Glu Ile Lys
                    100                     105


    <210>   44
```

```
<211>   107
<212>   PRT
<213>   Artificial Sequence

<220>
<221>   DOMAIN
<223>   Hab110.VL3   amino acid sequence


<400>   44
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asn Ser
                20                  25                  30
Leu Asn Trp Leu Gln Gln Lys Pro Gly Gly Ala Ile Lys Arg Leu Ile
            35                  40                  45
Tyr Ala Thr Phe Arg Leu Asp Ser Gly Val Pro Ser Arg Phe Ser Gly
        50                  55                  60
Ser Arg Ser Gly Thr Glu Tyr Thr Leu Thr Ile Ser Ser Leu Gln Pro
65                  70                  75                  80
Glu Asp Phe Ala Asp Tyr Tyr Cys Gln Gln Tyr Ala Ser Phe Pro Trp
                85                  90                  95
Thr Phe Gly Gly Gly Thr Lys Val Glu Ile Lys
            100                 105


<210>   45
<211>   120
<212>   PRT
<213>   Artificial Sequence

<220>
<221>   DOMAIN
<223>   Hab110.VH2   amino acid sequence


<400>   45
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
                20                  25                  30
Thr Met His Trp Val Arg Gln Ala Pro Gly Gln Arg Leu Glu Trp Met
            35                  40                  45
Gly Tyr Ile Asn Pro Asn Ser Phe Tyr Ile Glu Tyr Asn Gln Lys Phe
        50                  55                  60
Lys Asp Arg Val Thr Ile Thr Ala Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Asp Tyr Asp Leu Phe Tyr Asp Met Asp Asn Trp Gly Gln
            100                 105                 110
Gly Thr Thr Val Thr Val Ser Ser
            115                 120

<210>   46
<211>   120
<212>   PRT
<213>   Artificial Sequence

<220>
<221>   DOMAIN
<223>   Hab110.VH3   amino acid sequence
```

```
<400>   46
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Thr Met His Trp Val Arg Gln Ala Pro Gly Gln Arg Leu Glu Trp Ile
        35                  40                  45
Gly Tyr Ile Asn Pro Asn Ser Phe Tyr Ile Glu Tyr Asn Gln Lys Phe
    50                  55                  60
Lys Asp Arg Ala Thr Leu Thr Ala Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Asp Tyr Asp Leu Phe Tyr Asp Met Asp Asn Trp Gly Gln
            100                 105                 110
Gly Thr Thr Val Thr Val Ser Ser
        115                 120


<210>   47
<211>   120
<212>   PRT
<213>   Artificial Sequence

<220>
<221>   DOMAIN
<223>   Hab110.VH4   amino acid sequence


<400>   47
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Thr Met His Trp Val Arg Gln Ala Pro Gly Gln Arg Leu Glu Trp Ile
        35                  40                  45
Gly Tyr Ile Asn Pro Asn Ser Phe Tyr Ile Glu Tyr Asn Gln Lys Phe
    50                  55                  60
Lys Asp Lys Ala Thr Leu Thr Ala Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Asp Tyr Asp Leu Phe Tyr Asp Met Asp Asn Trp Gly Gln
            100                 105                 110
Gly Thr Thr Val Thr Val Ser Ser
        115                 120


<210>   48
<211>   120
<212>   PRT
<213>   Artificial Sequence

<220>
<221>   DOMAIN
<223>   Hab110.VH5   amino acid sequence


<400>   48
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Thr Met His Trp Val Lys Gln Ala Pro Gly Gln Gly Leu Glu Trp Ile
        35                  40                  45
```

```
Gly Tyr Ile Asn Pro Asn Ser Phe Tyr Ile Glu Tyr Asn Gln Lys Phe
    50                      55                  60
Lys Asp Lys Ala Thr Leu Thr Ala Asp Thr Ser Ala Ser Thr Ala Tyr
65                  70                  75                      80
Met Glu Leu Ser Ser Leu Arg Ser Glu Asp Thr Ala Val Tyr Tyr Cys
                85                  90                      95
Ala Arg Gly Asp Tyr Asp Leu Phe Tyr Asp Met Asp Asn Trp Gly Gln
            100                 105                 110
Gly Thr Thr Val Thr Val Ser Ser
        115                 120
```

```
<210>   49
<211>   114
<212>   PRT
<213>   Artificial Sequence

<220>
<221>   DOMAIN
<223>   Hab127 HCVR amino acid sequence
```

```
<400>   49
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Asn Gly Asp Thr Phe Tyr Ser Gln Lys Phe
    50                      55                  60
Lys Gly Arg Val Thr Met Thr Arg Asp Thr Ser Thr Ser Thr Val Tyr
65                  70                  75                      80
Met Glu Leu Ser Ser Leu Arg Ser Glu Asp Thr Ala Val Tyr Tyr Cys
                85                  90                      95
Ala Arg Ser Tyr Val Gly Asp Trp Gly Gln Gly Thr Thr Val Thr Val
            100                 105                 110
Ser Ser
```

```
<210>   50
<211>   107
<212>   PRT
<213>   Artificial Sequence

<220>
<221>   DOMAIN
<223>   Hab127 LCVR, Hab127.VL1   amino acid sequence
```

```
<400>   50
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Ser
            20                  25                  30
Leu Asn Trp Tyr Gln Gln Lys Pro Gly Lys Ala Pro Lys Arg Leu Ile
        35                  40                  45
Tyr Ala Thr Ser Ser Leu Asp Ser Gly Val Pro Ser Arg Phe Ser Gly
    50                      55                  60
Ser Gly Ser Gly Thr Glu Phe Thr Leu Thr Ile Ser Ser Leu Gln Pro
65                  70                  75                      80
Glu Asp Phe Ala Thr Tyr Tyr Cys Leu Gln Tyr Ala Ser Phe Pro Trp
                85                  90                      95
Thr Phe Gly Gly Gly Thr Lys Val Glu Ile Lys
```

100                    105

```
<210>  51
<211>  107
<212>  PRT
<213>  Artificial Sequence

<220>
<221>  DOMAIN
<223>  Hab127.VL2  amino acid sequence

<400>  51
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Ser
            20                  25                  30
Leu Asn Trp Leu Gln Gln Lys Pro Gly Lys Ala Pro Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Ser Phe Pro Trp
                85                  90                  95
Thr Phe Gly Gly Gly Thr Lys Val Glu Ile Lys
            100                 105


<210>  52
<211>  107
<212>  PRT
<213>  Artificial Sequence

<220>
<221>  DOMAIN
<223>  Hab127.VL3  amino acid sequence


<400>  52
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Ser
            20                  25                  30
Leu Asn Trp Leu Gln Gln Lys Pro Gly Gly Ala Leu Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Ser Phe Pro Trp
                85                  90                  95
Thr Phe Gly Gly Gly Thr Lys Val Glu Ile Lys
            100                 105

<210>  53
<211>  107
<212>  PRT
<213>  Artificial Sequence

<220>
<221>  DOMAIN
<223>  Hab127.VL4  amino acid sequence
```

<400> 53
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Ser
            20                  25                  30
Leu Asn Trp Leu Gln Gln Lys Pro Gly Gly Ala Leu Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Ser Asp Tyr Thr Leu Thr Ile Ser Ser Leu Gln Pro
65                  70                  75                  80
Glu Asp Phe Ala Thr Tyr Tyr Cys Leu Gln Tyr Ala Ser Phe Pro Trp
            85                  90                  95
Thr Phe Gly Gly Gly Thr Lys Val Glu Ile Lys
            100                 105


<210>   54
<211>   114
<212>   PRT
<213>   Artificial Sequence


<220>
<221>   DOMAIN
<223>   Hab127.VH1   amino acid sequence


<400>   54
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Asn Gly Asp Thr Phe Tyr Ser Gln Lys Phe
    50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
            85                  90                  95
Ala Thr Ser Tyr Val Gly Asp Trp Gly Gln Gly Thr Thr Val Thr Val
            100                 105                 110
Ser Ser


<210>   55
<211>   114
<212>   PRT
<213>   Artificial Sequence


<220>
<221>   DOMAIN
<223>   Hab127.VH2   amino acid sequence


<400>   55
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Met Gln Ala Pro Gly Gln Gly Leu Glu Trp Met
        35                  40                  45
Gly Arg Ile Tyr His Asn Asn Gly Asp Thr Phe Tyr Ser Gln Lys Phe

```
                50                    55                    60
Lys Gly Arg Val Thr Met Thr Val Asp Lys Ser Thr Ser Thr Val Tyr
65                    70                    75                    80
Met Glu Leu Ser Ser Leu Arg Ser Glu Asp Thr Ala Val Tyr Tyr Cys
                      85                    90                    95
Ala Thr Ser Tyr Val Gly Asp Trp Gly Gln Gly Thr Thr Val Thr Val
                100                   105                   110
Ser Ser


<210>  56
<211>  114
<212>  PRT
<213>  Artificial Sequence

<220>
<221>  DOMAIN
<223>  Hab127.VH3 amino acid sequence


<400>  56
Glu Val Gln Leu Val Gln Ser Gly Ala Glu Val Lys Lys Pro Gly Ala
1               5                    10                    15
Ser Val Lys Val Ser Cys Lys Ala Ser Gly Tyr Thr Phe Thr Ala Tyr
              20                    25                    30
Phe Met Asn Trp Val Met Gln Ala Pro Gly Gln Gly Leu Glu Trp Ile
          35                    40                    45
Gly Arg Ile Tyr His Asn Asn Gly Asp Thr Phe Tyr Ser Gln Lys Phe
      50                    55                    60
Lys Gly Arg Ala Thr Leu Thr Val Asp Lys Ser Thr Ser Thr Val Tyr
65                    70                    75                    80
Met Glu Leu Ser Ser Leu Arg Ser Glu Asp Thr Ala Val Tyr Tyr Cys
                      85                    90                    95
Ala Thr Ser Tyr Val Gly Asp Trp Gly Gln Gly Thr Thr Val Thr Val
                100                   105                   110
Ser Ser


<210>  57
<211>  114
<212>  PRT
<213>  Artificial Sequence

<220>
<221>  DOMAIN
<223>  Hab127.VH4   amino acid sequence


<400>  57
Glu Val Gln Leu Val Gln Ser Gly Ala Glu Val Lys Lys Pro Gly Ala
1               5                    10                    15
Ser Val Lys Val Ser Cys Lys Ala Ser Gly Tyr Thr Phe Thr Ala Tyr
              20                    25                    30
Phe Met Asn Trp Val Met Gln Ala Pro Gly Gln Ser Leu Glu Trp Ile
          35                    40                    45
Gly Arg Ile Tyr His Asn Asn Gly Asp Thr Phe Tyr Ser Gln Lys Phe
      50                    55                    60
Lys Gly Arg Ala Thr Leu Thr Val Asp Lys Ser Thr Ser Thr Val Tyr
65                    70                    75                    80
Met Glu Leu Arg Ser Leu Arg Ser Glu Asp Thr Ala Val Tyr Tyr Cys
                      85                    90                    95
Ala Thr Ser Tyr Val Gly Asp Trp Gly Gln Gly Thr Thr Val Thr Val
                100                   105                   110
```

Ser Ser


<210> 58
<211> 114
<212> PRT
<213> Artificial Sequence


<220>
<221> DOMAIN
<223> Hab127.VH1a amino acid sequence


<400> 58
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Gln Gly Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser


<210> 59
<211> 114
<212> PRT
<213> Artificial Sequence


<220>
<221> DOMAIN
<223> Hab127.VH1b amino acid sequence


<400> 59
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Leu Gly Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser


<210> 60
<211> 114
<212> PRT
<213> Artificial Sequence

```
<220>
<221>  DOMAIN
<223>  Hab127.VH1c amino acid sequence


<400>  60
Glu Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Thr Gly Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser


<210>  61
<211>  114
<212>  PRT
<213>  Artificial Sequence

<220>
<221>  DOMAIN
<223>  Hab127.VH1d amino acid sequence


<400>  61
Glu Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Lys Gly Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser


<210>  62
<211>  114
<212>  PRT
<213>  Artificial Sequence

<220>
<221>  DOMAIN
<223>  Hab127.VH1e amino acid sequence


<400>  62
```

```
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn Glu Gly Asp Thr Phe Tyr Ser Gln Lys Phe
    50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser
```

```
<210>  63
<211>  114
<212>  PRT
<213>  Artificial Sequence

<220>
<221>  DOMAIN
<223>  Hab127.VH1f amino acid sequence


<400>  63
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Asn His Gly Asp Thr Phe Tyr Ser Gln Lys Phe
    50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser
```

```
<210>  64
<211>  114
<212>  PRT
<213>  Artificial Sequence

<220>
<221>  DOMAIN
<223>  Hab127.VH1g amino acid sequence


<400>  64
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ala Tyr
            20                  25                  30
Phe Met Asn Trp Val Arg Gln Ala Pro Gly Gln Gly Leu Glu Trp Met
        35                  40                  45
Gly Arg Ile Tyr His Gln Asn Val Asp Thr Phe Tyr Ser Gln Lys Phe
```

```
        50                    55                    60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser
```

```
<210>   65
<211>   114
<212>   PRT
<213>   Artificial Sequence

<220>
<221>   DOMAIN
<223>   Hab127.VH1h amino acid sequence


<400>   65
Glu Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Ala Tyr
                20                  25                  30
Phe Met Asn Trp Val Arg Gln Ala Pro Gly Gln Gly Leu Glu Trp Met
            35                  40                  45
Gly Arg Ile Tyr His Leu Asn Val Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
Ser Ser
```

```
<210>   66
<211>   114
<212>   PRT
<213>   Artificial Sequence

<220>
<221>   DOMAIN
<223>   Hab127.VH1i amino acid sequence


<400>   66
Glu Val Gln Leu Val Gln Ser Gly Ala Glu Val Lys Lys Pro Gly Ala
1                   5                   10                  15
Ser Val Lys Val Ser Cys Lys Ala Ser Gly Tyr Thr Phe Thr Ala Tyr
                20                  25                  30
Phe Met Asn Trp Val Arg Gln Ala Pro Gly Gln Gly Leu Glu Trp Met
            35                  40                  45
Gly Arg Ile Tyr His His Asn Val Asp Thr Phe Tyr Ser Gln Lys Phe
        50                  55                  60
Lys Gly Arg Val Thr Met Thr Val Asp Lys Ser Thr Ser Thr Val Tyr
65                  70                  75                  80
Met Glu Leu Ser Ser Leu Arg Ser Glu Asp Thr Ala Val Tyr Tyr Cys
                85                  90                  95
Ala Thr Ser Tyr Val Gly Asp Trp Gly Gln Gly Thr Thr Val Thr Val
            100                 105                 110
```

Ser Ser


```
<210>  67
<211>  17
<212>  PRT
<213>  Artificial Sequence


<220>
<221>  DOMAIN
<223>  After hot spot mutation, mab127 humanized antibody HCDR2 amino acid
sequence general formula


<220>
<221>  DOMAIN
<222>  (5)..(5)
<223>  Xaa is selected from Asn, Gln, Leu or His


<220>
<221>  DOMAIN
<222>  (6)..(6)
<223>  Xaa is selected from Asn, Gln, Leu, Thr, Lys, Glu or His


<220>
<221>  DOMAIN
<222>  (7)..(7)
<223>  Xaa is selected from Val or Gly


<400>  67
Arg Ile Tyr His Xaa Xaa Xaa Asp Thr Phe Tyr Ser Gln Lys Phe Lys
1               5                   10                  15
Gly


<210>  68
<211>  17
<212>  PRT
<213>  Artificial Sequence


<220>
<221>  DOMAIN
<223>  hot spot mutation a of mab127 humanized antibody, HCDR2 amino acid
sequence


<400>  68
Arg Ile Tyr His Asn Gln Gly Asp Thr Phe Tyr Ser Gln Lys Phe Lys
1               5                   10                  15
Gly


<210>  69
<211>  17
<212>  PRT
<213>  Artificial Sequence


<220>
<221>  DOMAIN
<223>  hot spot mutation b of mab127 humanized antibody, HCDR2 amino acid
```

sequence

<400> 69
Arg Ile Tyr His Asn Leu Gly Asp Thr Phe Tyr Ser Gln Lys Phe Lys
1               5                   10                  15
Gly


<210> 70
<211> 17
<212> PRT
<213> Artificial Sequence

<220>
<221> DOMAIN
<223> hot spot mutation c of mab127 humanized antibody, HCDR2 amino acid
sequence


<400> 70
Arg Ile Tyr His Asn Thr Gly Asp Thr Phe Tyr Ser Gln Lys Phe Lys
1               5                   10                  15
Gly


<210> 71
<211> 17
<212> PRT
<213> Artificial Sequence

<220>
<221> DOMAIN
<223> hot spot mutation d of mab127 humanized antibody, HCDR2 amino acid
sequence


<400> 71
Arg Ile Tyr His Asn Lys Gly Asp Thr Phe Tyr Ser Gln Lys Phe Lys
1               5                   10                  15
Gly


<210> 72
<211> 17
<212> PRT
<213> Artificial Sequence

<220>
<221> DOMAIN
<223> hot spot mutation e of mab127 humanized antibody, HCDR2 amino acid
sequence


<400> 72
Arg Ile Tyr His Asn Glu Gly Asp Thr Phe Tyr Ser Gln Lys Phe Lys
1               5                   10                  15
Gly


<210> 73
<211> 17
<212> PRT

<213> Artificial Sequence

<220>
<221> DOMAIN
<223> hot spot mutation f of mab127 humanized antibody, HCDR2 amino acid sequence


<400> 73
Arg Ile Tyr His Asn His Gly Asp Thr Phe Tyr Ser Gln Lys Phe Lys
1               5                   10                  15
Gly


<210> 74
<211> 17
<212> PRT
<213> Artificial Sequence

<220>
<221> DOMAIN
<223> hot spot mutation g of mab127 humanized antibody, HCDR2 amino acid sequence


<400> 74
Arg Ile Tyr His Gln Asn Val Asp Thr Phe Tyr Ser Gln Lys Phe Lys
1               5                   10                  15
Gly


<210> 75
<211> 17
<212> PRT
<213> Artificial Sequence

<220>
<221> DOMAIN
<223> hot spot mutation h of mab127 humanized antibody, HCDR2 amino acid sequence


<400> 75
Arg Ile Tyr His Leu Asn Val Asp Thr Phe Tyr Ser Gln Lys Phe Lys
1               5                   10                  15
Gly


<210> 76
<211> 17
<212> PRT
<213> Artificial Sequence

<220>
<221> DOMAIN
<223> hot spot mutation i of mab127 humanized antibody, HCDR2 amino acid sequence


<400> 76
Arg Ile Tyr His His Asn Val Asp Thr Phe Tyr Ser Gln Lys Phe Lys
1               5                   10                  15
Gly

70

<210> 77
<211> 330
<212> PRT
<213> Artificial Sequence

<220>
<221> DOMAIN
<223> anti-CD73 humanized antibody heavy chain constant region amino acid sequence

<400> 77

```
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
        50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Phe Glu Gly Gly Pro Ser Val Phe Leu Phe Pro Pro
            115                 120                 125
Lys Pro Lys Asp Thr Leu Met Ile Ser Arg Thr Pro Glu Val Thr Cys
        130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
            195                 200                 205
Lys Ala Leu Pro Ala Ser Ile Glu Lys Thr Ile Ser Lys Ala Lys Gly
        210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
            275                 280                 285
Leu Tyr Ser Lys Leu Thr Val Asp Lys Ser Arg Trp Gln Gln Gly Asn
            290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330
```

<210> 78
<211> 107
<212> PRT
<213> Artificial Sequence

<220>

<221> DOMAIN
<223> anti-CD73 humanized antibody light chain constant region amino acid sequence


<400> 78

```
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```

<210> 79
<211> 443
<212> PRT
<213> Artificial Sequence

<220>
<221> DOMAIN
<223> Hab108.64 antibody heavy chain amino acid sequence


<400> 79

```
Glu Val Thr Leu Lys Gln Ser Gly Pro Gly Leu Val Lys Pro Thr Glu
1               5                   10                  15
Thr Leu Thr Leu Thr Cys Thr Val Ser Gly Phe Ser Leu Ser Ser Tyr
            20                  25                  30
Gly Ile Gln Trp Val Arg Gln Pro Pro Gly Lys Gly Leu Glu Trp Leu
        35                  40                  45
Gly Val Ile Trp Ser Gly Gly Ser Ala Asp Tyr Asn Ala Ala Phe Ile
    50                  55                  60
Ser Arg Leu Thr Ile Ser Lys Asp Thr Ser Lys Ser Gln Val Val Leu
65                  70                  75                  80
Thr Met Thr Asn Met Asp Pro Val Asp Thr Ala Thr Tyr Tyr Cys Ala
                85                  90                  95
Gly Gln Tyr Gly Ser Val Trp Gly Gln Gly Thr Leu Val Thr Val Ser
            100                 105                 110
Ser Ala Ser Thr Lys Gly Pro Ser Val Phe Pro Leu Ala Pro Ser Ser
            115                 120                 125
Lys Ser Thr Ser Gly Gly Thr Ala Ala Leu Gly Cys Leu Val Lys Asp
            130                 135                 140
Tyr Phe Pro Glu Pro Val Thr Val Ser Trp Asn Ser Gly Ala Leu Thr
145                 150                 155                 160
Ser Gly Val His Thr Phe Pro Ala Val Leu Gln Ser Ser Gly Leu Tyr
                165                 170                 175
Ser Leu Ser Ser Val Val Thr Val Pro Ser Ser Ser Leu Gly Thr Gln
            180                 185                 190
Thr Tyr Ile Cys Asn Val Asn His Lys Pro Ser Asn Thr Lys Val Asp
            195                 200                 205
Lys Lys Val Glu Pro Lys Ser Cys Asp Lys Thr His Thr Cys Pro Pro
    210                 215                 220
Cys Pro Ala Pro Glu Phe Glu Gly Gly Pro Ser Val Phe Leu Phe Pro
225                 230                 235                 240
Pro Lys Pro Lys Asp Thr Leu Met Ile Ser Arg Thr Pro Glu Val Thr
```

```
                           245                        250                        255
        Cys Val Val Val Asp Val Ser His Glu Asp Pro Glu Val Lys Phe Asn
                        260                        265                        270
        Trp Tyr Val Asp Gly Val Glu Val His Asn Ala Lys Thr Lys Pro Arg
                        275                        280                        285
        Glu Glu Gln Tyr Asn Ser Thr Tyr Arg Val Val Ser Val Leu Thr Val
            290                        295                        300
        Leu His Gln Asp Trp Leu Asn Gly Lys Glu Tyr Lys Cys Lys Val Ser
        305                        310                        315                        320
        Asn Lys Ala Leu Pro Ala Ser Ile Glu Lys Thr Ile Ser Lys Ala Lys
                        325                        330                        335
        Gly Gln Pro Arg Glu Pro Gln Val Tyr Thr Leu Pro Pro Ser Arg Asp
                        340                        345                        350
        Glu Leu Thr Lys Asn Gln Val Ser Leu Thr Cys Leu Val Lys Gly Phe
                        355                        360                        365
        Tyr Pro Ser Asp Ile Ala Val Glu Trp Glu Ser Asn Gly Gln Pro Glu
            370                        375                        380
        Asn Asn Tyr Lys Thr Thr Pro Pro Val Leu Asp Ser Asp Gly Ser Phe
        385                        390                        395                        400
        Phe Leu Tyr Ser Lys Leu Thr Val Asp Lys Ser Arg Trp Gln Gln Gly
                        405                        410                        415
        Asn Val Phe Ser Cys Ser Val Met His Glu Ala Leu His Asn His Tyr
                        420                        425                        430
        Thr Gln Lys Ser Leu Ser Leu Ser Pro Gly Lys
                        435                        440
```

```
<210>  80
<211>  213
<212>  PRT
<213>  Artificial Sequence

<220>
<221>  DOMAIN
<223>  Hab108.64 antibody light chain amino acid sequence


<400>  80
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Arg
                20                  25                  30
Leu Asn Trp Leu Gln Gln Lys Pro Gly Gly Ala Ile Lys Arg Leu Ile
            35                  40                  45
Tyr Ala Thr Ser Ser Leu Asp Ser Gly Val Pro Lys Arg Phe Ser Gly
        50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Gly Ser Trp Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
```

```
Asn Arg Gly Glu Cys
    210


<210>   81
<211>   450
<212>   PRT
<213>   Artificial Sequence


<220>
<221>   DOMAIN
<223>   Hab110.21 antibody heavy chain amino acid sequence


<400>   81
Glu Val Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Ser Tyr
            20                  25                  30
Thr Met His Trp Val Arg Gln Ala Pro Gly Gln Arg Leu Glu Trp Met
        35                  40                  45
Gly Tyr Ile Asn Pro Asn Ser Phe Tyr Ile Glu Tyr Asn Gln Lys Phe
    50                  55                  60
Lys Asp Arg Val Thr Ile Thr Arg Asp Thr Ser Ala Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Gly Asp Tyr Asp Leu Phe Tyr Asp Met Asp Asn Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Phe Glu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
                260                 265                 270
Asp Pro Glu Val Lys Phe Asn Trp Tyr Val Asp Gly Val Glu Val His
            275                 280                 285
Asn Ala Lys Thr Lys Pro Arg Glu Glu Gln Tyr Asn Ser Thr Tyr Arg
        290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Ser Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Asp Glu Leu Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
```

```
         385                   390                   395                   400
         Leu Asp Ser Asp Gly Ser Phe Phe Leu Tyr Ser Lys Leu Thr Val Asp
                         405                   410                   415
         Lys Ser Arg Trp Gln Gln Gly Asn Val Phe Ser Cys Ser Val Met His
                     420                   425                   430
         Glu Ala Leu His Asn His Tyr Thr Gln Lys Ser Leu Ser Leu Ser Pro
                     435                   440                   445
         Gly Lys
             450


         <210>   82
         <211>   214
         <212>   PRT
         <213>   Artificial Sequence

         <220>
         <221>   DOMAIN
         <223>   Hab110.21 antibody light chain amino acid sequence


         <400>   82
         Asp Ile Gln Met Thr Gln Ser Pro Ser Ser Leu Ser Ala Ser Val Gly
         1               5                   10                  15
         Asp Arg Val Thr Ile Thr Cys Arg Ala Ser Gln Asp Ile Gly Asn Ser
                     20                  25                  30
         Leu Asn Trp Leu Gln Gln Lys Pro Gly Lys Ala Ile Lys Arg Leu Ile
                     35                  40                  45
         Tyr Ala Thr Phe Arg Leu Asp Ser Gly Val Pro Ser Arg Phe Ser Gly
             50                  55                  60
         Ser Arg Ser Gly Thr Glu Tyr Thr Leu Thr Ile Ser Ser Leu Gln Pro
         65                  70                  75                  80
         Glu Asp Phe Ala Thr Tyr Tyr Cys Gln Gln Tyr Ala Ser Phe Pro Trp
                         85                  90                  95
         Thr Phe Gly Gly Gly Thr Lys Val Glu Ile Lys Arg Thr Val Ala Ala
                     100                 105                 110
         Pro Ser Val Phe Ile Phe Pro Pro Ser Asp Glu Gln Leu Lys Ser Gly
                     115                 120                 125
         Thr Ala Ser Val Val Cys Leu Leu Asn Asn Phe Tyr Pro Arg Glu Ala
                     130                 135                 140
         Lys Val Gln Trp Lys Val Asp Asn Ala Leu Gln Ser Gly Asn Ser Gln
         145                 150                 155                 160
         Glu Ser Val Thr Glu Gln Asp Ser Lys Asp Ser Thr Tyr Ser Leu Ser
                     165                 170                 175
         Ser Thr Leu Thr Leu Ser Lys Ala Asp Tyr Glu Lys His Lys Val Tyr
                     180                 185                 190
         Ala Cys Glu Val Thr His Gln Gly Leu Ser Ser Pro Val Thr Lys Ser
                     195                 200                 205
         Phe Asn Arg Gly Glu Cys
             210

         <210>   83
         <211>   444
         <212>   PRT
         <213>   Artificial Sequence

         <220>
         <221>   DOMAIN
         <223>   Hab127. 31d antibody heavy chain amino acid sequence


         <400>   83
         Glu Val Gln Leu Val Gln Ser Gly Ala Glu Val Lys Lys Pro Gly Ala
         1               5                   10                  15
```

75

```
Ser Val Lys Val Ser Cys Lys Ala Ser Gly Tyr Thr Phe Thr Ala Tyr
        20              25              30
Phe Met Asn Trp Val Arg Gln Ala Pro Gly Gln Gly Leu Glu Trp Met
        35              40              45
Gly Arg Ile Tyr His Asn Lys Gly Asp Thr Phe Tyr Ser Gln Lys Phe
    50              55              60
Lys Gly Arg Val Thr Met Thr Val Asp Lys Ser Thr Ser Thr Val Tyr
65              70              75              80
Met Glu Leu Ser Ser Leu Arg Ser Glu Asp Thr Ala Val Tyr Tyr Cys
            85              90              95
Ala Thr Ser Tyr Val Gly Asp Trp Gly Gln Gly Thr Thr Val Thr Val
        100             105             110
Ser Ser Ala Ser Thr Lys Gly Pro Ser Val Phe Pro Leu Ala Pro Ser
    115             120             125
Ser Lys Ser Thr Ser Gly Gly Thr Ala Ala Leu Gly Cys Leu Val Lys
130             135             140
Asp Tyr Phe Pro Glu Pro Val Thr Val Ser Trp Asn Ser Gly Ala Leu
145             150             155             160
Thr Ser Gly Val His Thr Phe Pro Ala Val Leu Gln Ser Ser Gly Leu
            165             170             175
Tyr Ser Leu Ser Ser Val Val Thr Val Pro Ser Ser Ser Leu Gly Thr
        180             185             190
Gln Thr Tyr Ile Cys Asn Val Asn His Lys Pro Ser Asn Thr Lys Val
        195             200             205
Asp Lys Lys Val Glu Pro Lys Ser Cys Asp Lys Thr His Thr Cys Pro
    210             215             220
Pro Cys Pro Ala Pro Glu Phe Glu Gly Gly Pro Ser Val Phe Leu Phe
225             230             235             240
Pro Pro Lys Pro Lys Asp Thr Leu Met Ile Ser Arg Thr Pro Glu Val
            245             250             255
Thr Cys Val Val Val Asp Val Ser His Glu Asp Pro Glu Val Lys Phe
        260             265             270
Asn Trp Tyr Val Asp Gly Val Glu Val His Asn Ala Lys Thr Lys Pro
    275             280             285
Arg Glu Glu Gln Tyr Asn Ser Thr Tyr Arg Val Val Ser Val Leu Thr
    290             295             300
Val Leu His Gln Asp Trp Leu Asn Gly Lys Glu Tyr Lys Cys Lys Val
305             310             315             320
Ser Asn Lys Ala Leu Pro Ala Ser Ile Glu Lys Thr Ile Ser Lys Ala
            325             330             335
Lys Gly Gln Pro Arg Glu Pro Gln Val Tyr Thr Leu Pro Pro Ser Arg
            340             345             350
Asp Glu Leu Thr Lys Asn Gln Val Ser Leu Thr Cys Leu Val Lys Gly
            355             360             365
Phe Tyr Pro Ser Asp Ile Ala Val Glu Trp Glu Ser Asn Gly Gln Pro
    370             375             380
Glu Asn Asn Tyr Lys Thr Thr Pro Pro Val Leu Asp Ser Asp Gly Ser
385             390             395             400
Phe Phe Leu Tyr Ser Lys Leu Thr Val Asp Lys Ser Arg Trp Gln Gln
            405             410             415
Gly Asn Val Phe Ser Cys Ser Val Met His Glu Ala Leu His Asn His
        420             425                     430
Tyr Thr Gln Lys Ser Leu Ser Leu Ser Pro Gly Lys
    435             440
```

```
<210>   84
<211>   214
<212>   PRT
<213>   Artificial Sequence

<220>
<221>   DOMAIN
<223>   Hab127. 31d antibody light chain amino acid sequence
```

```
<400>  84
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Ile Gly Asp Ser
            20                  25                  30
Leu Asn Trp Leu Gln Gln Lys Pro Gly Gly Ala Leu Lys Arg Leu Ile
        35                  40                  45
Tyr Ala Thr Ser Ser Leu Asp Ser Gly Val Pro Ser Arg Phe Ser Gly
    50                  55                  60
Ser Gly Ser Gly Thr Glu Tyr Thr Leu Thr Ile Ser Ser Leu Gln Pro
65                  70                  75                  80
Glu Asp Phe Ala Thr Tyr Tyr Cys Leu Gln Tyr Ala Ser Phe Pro Trp
            85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
            115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
            165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
            195                 200                 205
Phe Asn Arg Gly Glu Cys
            210
```

## Claims

1. An anti-CD73 antibody or an antigen-binding fragment thereof specifically binding to human CD73, wherein the anti-CD73 antibody or the antigen-binding fragment thereof comprises a light chain variable region and a heavy chain variable region, wherein:

   the light chain variable region comprises light chain LCDR1, LCDR2 and LCDR3 as shown in SEQ ID NOs: 27, 28 and 29 respectively, or comprises LCDR variant(s) having no more than 3 amino acid mutation(s) relative to the sequence as shown in SEQ ID NOs: 27, 28 and 29 respectively; and
   the heavy chain variable region comprises:

   i) HCDR1, HCDR2 and HCDR3 as shown in SEQ ID NOs: 10, 11 and 12 respectively or HCDR variant(s) having no more than 3 amino acid mutation(s) relative to the sequence as shown in SEQ ID NOs: 10, 11 and 12 respectively; or
   ii) HCDR1, HCDR2 and HCDR3 as shown in SEQ ID NOs: 16, 17 and 18 respectively or HCDR variant(s) having no more than 3 amino acid mutation(s) relative to the sequence as shown in SEQ ID NOs: 16, 17 and 18 respectively; or
   iii) HCDR1, HCDR2 and HCDR3 as shown in SEQ ID NOs: 22, 67 and 24 respectively or HCDR variant(s) having no more than 3 amino acid mutation(s) relative to the sequence as shown in SEQ ID NOs: 22, 67 and 24 respectively.

2. The anti-CD73 antibody or the antigen-binding fragment thereof according to claim 1, wherein the anti-CD73 antibody or the antigen-binding fragment thereof binds to human CD73 with a dissociation equilibrium constant equal to or less than $10^{-9}$ M.

3. The anti-CD73 antibody or the antigen-binding fragment thereof according to claim 1 or 2, wherein the anti-CD73 antibody or the antigen-binding fragment thereof comprises:

iv) HCDR1, HCDR2, HCDR3, LCDR1, LCDR2 and LCDR3 as shown in SEQ ID NOs: 10, 11, 12, 13, 14 and 15, respectively; or

v) HCDR1, HCDR2, HCDR3, LCDR1, LCDR2 and LCDR3 as shown in SEQ ID NOs: 16, 17, 18, 19, 20 and 21, respectively; or

vi) HCDR2 as shown in SEQ ID NOs: 23, 68, 69, 70, 71, 72, 73, 74, 75 or 76 and HCDR1, HCDR3, LCDR1, LCDR2 and LCDR3 as shown in SEQ ID NOs: 22, 24, 25, 14 and 26, respectively.

4. The anti-CD73 antibody or the antigen-binding fragment thereof according to any one of claims 1-3, wherein the antibody is a murine antibody, a chimeric antibody or a humanized antibody.

5. The anti-CD73 antibody or the antigen-binding fragment thereof according to claim 4, wherein the murine or the chimeric antibody or the antigen-binding fragment thereof is any one selected from the group consisting of A-C:

(A) an anti-CD73 antibody or an antigen-binding fragment thereof comprising a heavy chain variable region as shown in SEQ ID NO: 4 and a light chain variable region as shown in SEQ ID NO: 5;
(B) an anti-CD73 antibody or an antigen-binding fragment thereof comprising a heavy chain variable region as shown in SEQ ID NO: 6 and a light chain variable region as shown in SEQ ID NO: 7; and
(C) an anti-CD73 antibody or an antigen-binding fragment thereof comprising a heavy chain variable region as shown in SEQ ID NO: 8 and a light chain variable region as shown in SEQ ID NO: 9.

6. The anti-CD73 antibody or the antigen-binding fragment thereof according to claim 4, wherein the antibody is a humanized antibody, and the light chain framework region (FR) and the heavy chain framework region (FR) of the antibody are respectively derived from human germline light chain and heavy chain or mutant sequence thereof; preferably, the humanized antibody comprises any one selected from the group consisting of D-F:

(D) HCDR1, HCDR2, HCDR3, LCDR1, LCDR2 and LCDR3 as shown in SEQ ID NOs: 10, 11, 12, 13, 14 and 15, respectively, and heavy chain framework region(s) and light chain framework region(s); and wherein the heavy chain framework region(s) comprise(s) one or more back mutation(s) selected from the group consisting of 6Q, 10G, 30K, 37V, 44G, 49G and 94G; and wherein the light chain framework region(s) comprise(s) one or more back mutation(s) selected from the group consisting of 36L, 42G, 441, 60K, 69S, 71Y and 85D;
(E) HCDR1, HCDR2, HCDR3, LCDR1, LCDR2 and LCDR3 as shown in SEQ ID NOs: 16, 17, 18, 19, 20 and 21, respectively, and heavy chain framework region(s) and light chain framework region(s); and wherein the heavy chain framework region(s) comprise(s) one or more back mutation(s) selected from the group consisting of 38K, 44G, 481, 66K, 67A, 69L and 71A; and wherein the light chain framework region(s) comprise(s) one or more back mutation(s) selected from the group consisting of 36L, 42G, 441, 66R, 71Y and 85D; and
(F) HCDR2 as shown in SEQ ID NO: 23, 68, 69, 70, 71, 72, 73, 74, 75 or 76, and HCDR1, HCDR3, LCDR1, LCDR2 and LCDR3 as shown in SEQ ID NOs: 22, 24, 25, 14 and 26 respectively, and heavy chain framework region(s) and light chain framework region(s); and wherein the heavy chain framework region(s) comprise(s) one or more back mutation(s) selected from the group consisting of 38M, 44S, 481, 67A, 69L, 71V, 73K, 82A R and 94T; and wherein the light chain framework region(s) comprise(s) one or more back mutation(s) selected from the group consisting of 36L, 42G, 44L, 69S, 70D and 71Y;

wherein, the back-mutation sites are numbered according to Kabat numbering criteria.

7. The anti-CD73 antibody or the antigen-binding fragment thereof according to claim 6, wherein the humanized antibody comprises a heavy chain variable region as shown in SEQ ID NO: 30, 41 or 49 or variant(s) thereof.

8. The anti-CD73 antibody or the antigen-binding fragment thereof according to claim 7, wherein the heavy chain variable region variant has 1-10 amino acid back mutation(s) on the FR region(s) of the heavy chain variable region as shown in SEQ ID NO: 30, 41 or 49; preferably, the heavy chain variable region variant is any one selected from the group consisting of G-I:

(G) a heavy chain variable region variant, having one or more back mutation(s) selected from the group consisting of E6Q, V10G, S30K, I37V, L44G, A49G and R94G on the FR region(s) of the heavy chain variable region as shown in SEQ ID NO: 30;
(H) a heavy chain variable region variant, having one or more back mutation(s) selected from the group consisting of R38K, R44G, M48I, R66K, V67A, I69L and R71A on the FR region(s) of the heavy chain variable region as shown in SEQ ID NO: 41; and

(I) a heavy chain variable region variant, having one or more back mutation(s) selected from the group consisting of R38M, G44S, M48I, V67A, M69L, R71V, T73K, S82A R and R94T on the FR region(s) of the heavy chain variable region as shown in SEQ ID NO: 49.

9. The anti-CD73 antibody or the antigen-binding fragment thereof according to any one of claims 6 to 8, wherein the antibody comprises a heavy chain variable region of any one selected from the group consisting of J-L:

(J) a heavy chain variable region as shown in SEQ ID NO: 30, 37, 38, 39 or 40;
(K) a heavy chain variable region as shown in SEQ ID NO: 41, 45, 46, 47 or 48; and
(L) a heavy chain variable region as shown in SEQ ID NO: 49, 54, 55, 56, 57, 58, 59, 60, 61, 62, 63, 64, 65 or 66.

10. The anti-CD73 antibody or the antigen-binding fragment thereof according to claim 6, wherein the antibody comprises a light chain variable region as shown in SEQ ID NO: 31, 42 or 50 or variant(s) thereof.

11. The anti-CD73 antibody or the antigen-binding fragment thereof according to claim 10, wherein the light chain variable region variant has 1-10 amino acid back mutation(s) on the FR region(s) of the light chain variable region as shown in SEQ ID NO: 31, 42 or 50; preferably, the light chain variable region variant is any one selected from the group consisting of M-O:

(M) a light chain variable region variant, having one or more back mutation(s) selected from the group consisting of Y36L, K42G, P44I, S60K, T69S, F71Y and T85D on the FR region(s) of the light chain variable region as shown in SEQ ID NO: 31;
(N) a light chain variable region variant, having one or more back mutation(s) selected from the group consisting of Y36L, K42G, P44I, G66R, F71Y and T85D on the FR region(s) of the light chain variable region as shown in SEQ ID NO: 42; and
(O) a light chain variable region variant, having one or more back mutation(s) selected from the group consisting of Y36L, K42G, P44L, T69S, E70D and F71Y on the FR region(s) of the light chain variable region as shown in SEQ ID NO: 50.

12. The anti-CD73 antibody or the antigen-binding fragment thereof according to any one of claims 6, 10 and 11, wherein the antibody comprises a light chain variable region of any one selected from the group consisting of P-R:

(P) a light chain variable region as shown in SEQ ID NO: 31, 32, 33, 34, 35 or 36;
(Q) a light chain variable region as shown in SEQ ID NO: 42, 43 or 44; and
(R) a light chain variable region as shown in SEQ ID NO: 50, 51, 52 or 53.

13. The anti-CD73 antibody or the antigen-binding fragment thereof according to any one of claims 6 to 12, wherein the antibody or the antigen-binding fragment thereof comprises a heavy chain variable region and a light chain variable region of any one selected from the group consisting of S-U:

(S) a heavy chain variable region as shown in amino acid sequence SEQ ID NO: 30, 37, 38, 39 or 40 or having at least 95% sequence identity to SEQ ID NO: 30, 37, 38, 39 or 40, and a light chain variable region as shown in amino acid sequence SEQ ID NO: 31, 32, 33, 34, 35 or 36 or having at least 95% sequence identity to SEQ ID NO: 31, 32, 33, 34, 35 or 36;
(T) a heavy chain variable region as shown in amino acid sequence SEQ ID NO: 41, 45, 46, 47 or 48 or having at least 95% sequence identity to SEQ ID NO: 41, 45, 46, 47 or 48, and a light chain variable region as shown in amino acid sequence SEQ ID NO: 42, 43 or 44 or having at least 95% sequence identity to SEQ ID NO: 42, 43 or 44; and
(U) a heavy chain variable region as shown in amino acid sequence SEQ ID NO: 49, 54, 55, 56, 57, 58, 59, 60, 61, 62, 63, 64, 65 or 66 or having at least 95% sequence identity to SEQ ID NO: 49, 54, 55, 56, 57, 58, 59, 60, 61, 62, 63, 64, 65 or 66, and a light chain variable region as shown in amino acid sequence SEQ ID NO: 50, 51, 52 or 53 or having at least 95% sequence identity to SEQ ID NO: 50, 51, 52 or 53; preferably, the antibody or the antigen-binding fragment thereof comprises any one selected from the group consisting of V-X:
(V) a heavy chain variable region as shown in SEQ ID NO: 39 and a light chain variable region as shown in SEQ ID NO: 36;
(W) a heavy chain variable region as shown in SEQ ID NO: 41 and a light chain variable region as shown in SEQ ID NO: 43; and

(X) a heavy chain variable region as shown in SEQ ID NO: 61 and a light chain variable region as shown in SEQ ID NO: 52.

14. The anti-CD73 antibody or the antigen-binding fragment thereof according to any one of claims 1 to 13, wherein the antibody comprises constant region(s); preferably, the heavy chain constant region of the antibody is derived from human IgG1, IgG2, IgG3 or IgG4, or mutant sequence(s) of IgG1, IgG2, IgG3 or IgG4, the light chain constant region is derived from human κ, λ chain or mutant sequence(s) thereof; more preferably, the amino acid sequence of the antibody heavy chain constant region is as shown in SEQ ID NO: 77, or has at least 85% sequence identity to SEQ ID NO:77, and the amino acid sequence of the light chain constant region is as shown in SEQ ID NO:78 or has at least 85% sequence identity to SEQ ID NO:78.

15. The anti-CD73 antibody or the antigen-binding fragment thereof according to claim 14, wherein the antibody is any one selected from the group consisting of iv)-vi):

iv) an anti-CD73 antibody comprising a heavy chain as shown in SEQ ID NO: 79 or having at least 85% sequence identity to SEQ ID NO: 79 and a light chain as shown in SEQ ID NO: 80 or having at least 85% sequence identity to SEQ ID NO: 80;
v) an anti-CD73 antibody comprising a heavy chain as shown in SEQ ID NO: 81 or having at least 85% sequence identity to SEQ ID NO: 81 and a light chain as shown in SEQ ID NO: 82 or having at least 85% sequence identity to SEQ ID NO: 82; and
vi) an anti-CD73 antibody comprising a heavy chain as shown in SEQ ID NO: 83 or having at least 85% sequence identity to SEQ ID NO: 83 and a light chain as shown in SEQ ID NO: 84 or having at least 85% sequence identity to SEQ ID NO: 84.

16. The anti-CD73 antibody or the antigen-binding fragment thereof according to any one of claims 1 to 15, wherein the antigen-binding fragment is selected from the group consisting of Fab, Fab', F(ab')2, scFv, diabody, and dsFv.

17. An isolated monoclonal antibody or an antigen-binding fragment thereof, wherein the antibody or the antigen-binding fragment thereof competitively with the antibody or the antigen-binding fragment thereof according to any one of claims 1 to 16, for binding to human CD73.

18. A nucleic acid molecule encoding the anti-CD73 antibody or the antigen-binding fragment thereof according to any one of claims 1 to 17.

19. A vector comprising the nucleic acid molecule of claim 18.

20. A host cell obtained by transforming the vector of claim 19, wherein the host cell is selected from the group consisting of prokaryotic cells and eukaryotic cells, preferably eukaryotic cells, more preferably mammalian cells.

21. A method for preparing the anti-CD73 antibody or the antigen-binding fragment thereof according to any one of claims 1 to 17, comprising steps of:
culturing the host cell of claim 20 in a suitable medium under conditions suitable for growth, recovering and purifying the anti-CD73 antibody or the antigen-binding fragment thereof expressed by the host cell from the medium.

22. A pharmaceutical composition comprising the anti-CD73 antibody or the antigen-binding fragment thereof according to any one of claims 1 to 17, or the nucleic acid molecule according to claim 18, and one or more pharmaceutically acceptable carrier(s), excipient(s) or diluent(s).

23. An *in vitro* method for detection of human CD73 in a sample to be tested, the method comprising:

contacting the anti-CD73 antibody or the antigen-binding fragment thereof according to any one of claims 1 to 17, or the pharmaceutical composition of claim 22 with the sample to be tested; and
determining the presence or level of human CD73 in the sample to be tested.

24. Use of the anti-CD73 antibody or the antigen-binding fragment thereof according to any one of claims 1 to 17, or the nucleic acid molecule according to claim 18, or the pharmaceutical composition according to claim 22 for preparation of a medicament for the treatment or prevention of a disease or disorder.

**25.** The use according to claim 24, wherein the disease or disorder is related to human CD73.

**26.** The use according to claim 24 or 25, wherein the disease or disorder is a tumor, preferably is breast cancer, glioma, bladder cancer, colon cancer, melanoma, ovarian cancer, thyroid cancer, esophageal cancer, lung cancer, kidney cancer, pancreatic cancer, lymphoma, prostate cancer or gastric cancer.

Fig. 1

Fig. 2

Fig. 3

**Fig. 4**

**Fig. 5**

**Fig. 6**

**Fig. 7**

Fig. 8

## INTERNATIONAL SEARCH REPORT

| International application No. |
|---|
| **PCT/CN2019/117114** |

**A. CLASSIFICATION OF SUBJECT MATTER**

C07K 16/28(2006.01)i; C07K 16/46(2006.01)i; A61K 39/395(2006.01)i; C12N 15/13(2006.01)i; C07K 19/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K,C12N,A61K,A61P

Documentation searched other than minimum documentation to the extent that such documents are included in the fields searched

Electronic data base consulted during the international search (name of data base and, where practicable, search terms used)

CNKI, CNABS, CNTXT, DWPI, SIPOABS, EPTXT, WOTXT, USTXT, JPTXT, ELSEVIER, ISI Web of Knowledge, PubMed, 中国专利生物序列检索系统, Chinese Patent Biological Sequence Retrieval System, NCBI, EMBL, STN: 抗体, 抗原, 抗原结合片段, 单克隆抗体, 单抗, 嵌合抗体, 人源化抗体, antibody, McAb, monoclonal antibody, chimeric antibody, humanized antibody, CDR, CD73, 胞外-5'-核苷酸酶, Ecto-5'-Nucleotidase, antigen binding fragment, Fab, SEQ ID NOs: 1-84.

**C. DOCUMENTS CONSIDERED TO BE RELEVANT**

| Category* | Citation of document, with indication, where appropriate, of the relevant passages | Relevant to claim No. |
|---|---|---|
| X | WO 2018137598 A1 (I-MAB) 02 August 2018 (2018-08-02) the abstract, claims 1-22 and 36, and paragraphs [0013] and [0014], specific embodiment, tables A and 1-11, and sequence list | 17-26 |
| X | WO 2017064043 A1 (INNATE PHARMA et al.) 20 April 2017 (2017-04-20) claims 16-50, and embidiments 1-8, and sequence list | 17-26 |
| X | CN 107001474 A (BRISTOL-MYERS SQUIBB COMPANY) 01 August 2017 (2017-08-01) claims 1-62 | 17-26 |
| X | WO 2017100670 A1 (CORVUS PHARMACEUTICALS INC. et al.) 15 June 2017 (2017-06-15) claims 1-66, and paragraphs [0004]-[0038], embodiments 1 and 2, and sequence list | 17-26 |
| A | US 2014271625 A1 (BAYER HEALTHCARE LLC) 18 September 2014 (2014-09-18) entire document | 1-26 |
| A | ZHANG, B. "CD73: A Novel Target for Cancer Immunotherapy" *Cancer Research*, Vol. 70, No. 16, 15 August 2010 (2010-08-15), pp. 6407-6411 | 1-26 |

☑ Further documents are listed in the continuation of Box C. ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **03 January 2020** | **31 January 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2019/117114** |

**C.    DOCUMENTS CONSIDERED TO BE RELEVANT**

| Category* | Citation of document, with indication, where appropriate, of the relevant passages | Relevant to claim No. |
|---|---|---|
| A | Antonioli, L. et al. "Anti-CD73 Immunotherapy: A Viable Way to Reprogram the Tumor Microenvironment" *Oncoimmunology*, Vol. 5, No. 9, 10 September 2016 (2016-09-10), p. e1216292 | 1-26 |

Form PCT/ISA/210 (second sheet) (January 2015)

**INTERNATIONAL SEARCH REPORT**

International application No.

**PCT/CN2019/117114**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

## INTERNATIONAL SEARCH REPORT
### Information on patent family members

International application No.

**PCT/CN2019/117114**

| Patent document cited in search report | | | Publication date (day/month/year) | Patent family member(s) | | | Publication date (day/month/year) |
|---|---|---|---|---|---|---|---|
| WO | 2018137598 | A1 | 02 August 2018 | KR | 20180093088 | A | 20 August 2018 |
| | | | | US | 2019256598 | A1 | 22 August 2019 |
| | | | | EP | 3383916 | A4 | 11 September 2019 |
| | | | | AU | 2018213549 | A1 | 06 June 2019 |
| | | | | CN | 109476755 | A | 15 March 2019 |
| | | | | CO | 2019006657 | A2 | 28 June 2019 |
| | | | | EP | 3383916 | A1 | 10 October 2018 |
| | | | | CL | 2019001756 | A1 | 27 September 2019 |
| | | | | CA | 3045376 | A1 | 02 August 2018 |
| | | | | KR | 20190105122 | A | 11 September 2019 |
| | | | | IL | 267942 | D0 | 26 September 2019 |
| | | | | CL | 2018001512 | A1 | 08 March 2019 |
| WO | 2017064043 | A1 | 20 April 2017 | EP | 3362475 | A1 | 22 August 2018 |
| | | | | US | 2019225703 | A1 | 25 July 2019 |
| CN | 107001474 | A | 01 August 2017 | SG | 11201703192S | A | 30 May 2017 |
| | | | | AU | 2015349878 | A1 | 25 May 2017 |
| | | | | MA | 40309 | A1 | 31 January 2018 |
| | | | | MX | 2017006624 | A | 21 August 2017 |
| | | | | US | 10100129 | B2 | 16 October 2018 |
| | | | | CL | 2017001296 | A1 | 16 February 2018 |
| | | | | WO | 2016081748 | A2 | 26 May 2016 |
| | | | | KR | 20170080699 | A | 10 July 2017 |
| | | | | BR | 112017010110 | A2 | 30 January 2018 |
| | | | | WO | 2016081748 | A3 | 15 September 2016 |
| | | | | EP | 3221363 | A2 | 27 September 2017 |
| | | | | JP | 2017537620 | A | 21 December 2017 |
| | | | | EA | 201790986 | A1 | 31 October 2017 |
| | | | | PH | 12017500918 | A1 | 20 November 2017 |
| | | | | UY | 36404 | A | 01 June 2016 |
| | | | | IL | 252353 | D0 | 31 July 2017 |
| | | | | US | 2019055320 | A1 | 21 February 2019 |
| | | | | PE | 07822017 | A1 | 04 July 2017 |
| | | | | PE | 20170782 | A1 | 04 July 2017 |
| | | | | TW | 201625693 | A | 16 July 2016 |
| | | | | CA | 2968357 | A1 | 26 May 2016 |
| | | | | CL | 2018001414 | A1 | 24 August 2018 |
| | | | | US | 2018127513 | A1 | 10 May 2018 |
| | | | | TN | 2017000203 | A1 | 19 October 2018 |
| | | | | AR | 102698 | A1 | 15 March 2017 |
| WO | 2017100670 | A1 | 15 June 2017 | EP | 3387442 | A1 | 17 October 2018 |
| | | | | JP | 2019503709 | A | 14 February 2019 |
| | | | | CA | 3007646 | A1 | 15 June 2017 |
| | | | | KR | 20180134837 | A | 19 December 2018 |
| | | | | AU | 2016366548 | A1 | 28 June 2018 |
| | | | | CN | 109154611 | A | 04 January 2019 |
| | | | | US | 2019077873 | A1 | 14 March 2019 |
| | | | | EP | 3387442 | A4 | 08 May 2019 |
| | | | | MX | 2018006973 | A | 16 May 2019 |
| | | | | EA | 201891366 | A1 | 28 December 2018 |
| | | | | CL | 2018001512 | A1 | 08 March 2019 |

Form PCT/ISA/210 (patent family annex) (January 2015)

INTERNATIONAL SEARCH REPORT
Information on patent family members

International application No.

**PCT/CN2019/117114**

| Patent document cited in search report | Publication date (day/month/year) | Patent family member(s) | | Publication date (day/month/year) |
|---|---|---|---|---|
| | | IL 259927 D0 | | 31 July 2018 |
| US 2014271625 A1 | 18 September 2014 | US 9090697 B2 | | 28 July 2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9938356 B
- US 9605080 B
- WO 2016075099 A
- WO 2017152085 A
- WO 2017064043 A
- WO 2018013611 A
- US 4683195 A

### Non-patent literature cited in the description

- **ZHI et al.** *Cancer Sci,* 2010, vol. 101 (12), 2561-2569
- **STAGG J et al.** *PNAS,* 2010, vol. 107 (4), 1547-1552
- **RYZHOV et al.** *J Immunol,* 2011, vol. 187 (11), 6120-6129
- **XIONG et al.** *Cell Tissue Res,* 2014, vol. 355 (2), 365-374
- **STAGG ; WANG et al.** *Cancer Res,* 2011, vol. 71 (8), 2892-2900
- *J Cancer Res Clin Oncol,* 2008, vol. 134 (3), 365-372
- *J. biol. chem,* 1968, vol. 243, 3558
- **KABAT et al.** Sequences of Proteins of Immunological Interest. Public Health Service, National Institutes of Health, 1991
- **AL-LAZIKANI et al.** *JMB,* 1997, vol. 273, 927-948
- **LEFRANC MP.** *Immunologist,* 1999, vol. 7, 132-136
- **LEFRANC, MP.** *Dev. Comp. Immunol.,* 2003, vol. 27, 55-77
- **KABAT, EA et al.** Sequences of Proteins of Immunological Interest. 1991
- **WARD et al.** *Nature,* 1989, vol. 341, 544-546
- **BIRD et al.** *Science,* 1988, vol. 242, 423-426
- **HUSTON et al.** *Proc. Natl. Acad. Sci USA,* 1988, vol. 85, 5879-5883
- **HOLLIGER et al.** *Proc. Natl. Acad. Sci. USA,* 1993, vol. 90, 6444-6448
- **ALFTHAN et al.** *Protein Eng.,* 1995, vol. 8, 725-731
- **CHOI et al.** *Eur. J. Immunol.,* 2001, vol. 31, 94-106
- **HU et al.** *Cancer Res.,* 1996, vol. 56, 3055-3061
- **KIPRIYANOV et al.** *J. Mol. Biol.,* 1999, vol. 293, 41-56
- **ROOVERS et al.** *Cancer Immunol.,* 2001
- *Protein Engineering,* 1994, vol. 7, 697
- Epitope Mapping Protocols in Methods in Molecular Biology. 1996, vol. 66
- **STAHLI et al.** *Methods in Enzymology,* 1983, vol. 9, 242-253
- **KIRKLAND et al.** *J. Immunol.,* 1986, vol. 137, 3614-3619
- **CHEUNG et al.** *Virology,* 1990, vol. 176, 546-552
- **HARLOW ; LANE.** Antibodies, A Laboratory Manual. Cold Spring Harbor Press, 1988
- **MOREL et al.** *Molec. Immunol.,* 1988, vol. 25, 7-15
- **MOLDENHAUER et al.** *Scand. J. Immunol.,* 1990, vol. 32, 77-82
- *The Immunoglobulin Facts,* 2001, ISBN 012441351
- **WATSON et al.** Molecular Biology of the Gene. The Benjamin/Cummings Pub. Co, 1987, 224
- **MULLIS et al.** *Cold Spring Harbor Symp. Ouant. Biol,* 1987, vol. 51, 263
- PCR TECHNOLOGY. Stockton Press, 1989